(19) Europäisches Patentamt
European Patent Office
Office européen des brevets

(11) **EP 4 413 055 B1**

(12) **EUROPEAN PATENT SPECIFICATION**

(45) Date of publication and mention of the grant of the patent:
**03.12.2025 Bulletin 2025/49**

(21) Application number: **22818509.6**

(22) Date of filing: **06.10.2022**

(51) International Patent Classification (IPC):
***C08F 220/18*** $^{(2006.01)}$ ***A61K 8/00*** $^{(2006.01)}$
***A61K 8/36*** $^{(2006.01)}$ ***A61K 8/81*** $^{(2006.01)}$
***A61Q 17/00*** $^{(2006.01)}$ ***A61Q 19/10*** $^{(2006.01)}$
***A61K 8/73*** $^{(2006.01)}$

(52) Cooperative Patent Classification (CPC):
(C-Sets available)
**C08F 220/1802; A61K 8/361; A61K 8/732; A61K 8/8129; A61K 8/8152; A61Q 17/005; A61Q 19/10;** A61K 2800/30; A61K 2800/548
(Cont.)

(86) International application number:
**PCT/US2022/045873**

(87) International publication number:
**WO 2023/059784 (13.04.2023 Gazette 2023/15)**

(54) **STABILIZED RHEOLOGY MODIFIER EMULSIONS**

STABILISIERTE EMULSIONEN ZUR RHEOLOGIEVERÄNDERUNG

ÉMULSIONS DE MODIFICATEUR DE RHÉOLOGIE STABILISÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **06.10.2021 US 202163252802 P**

(43) Date of publication of application:
**14.08.2024 Bulletin 2024/33**

(73) Proprietor: **Lubrizol Advanced Materials, Inc.**
**Cleveland, OH 44141-3247 (US)**

(72) Inventors:
- **PRATA, Joseph E.**
**Cleveland, Ohio 44141-3247 (US)**
- **TAMARESELVY, Krishnan**
**Piscataway, New Jersey 08854 (US)**
- **SMITH, Steven J.**
**Cleveland, Ohio 44141-3247 (US)**
- **MARCU, Ioan**
**Cleveland, Ohio 44141-3247 (US)**
- **LI, Sinan**
**Cleveland, Ohio 44141-3247 (US)**
- **CARVER, Toni E.**
**Cleveland, Ohio 44141-3247 (US)**
- **LIPP, Lindsay L.**
**Cleveland, Ohio 44141-3247 (US)**
- **MOHAMMED, Sami**
**Avon Lake, Ohio 44012 (US)**

(74) Representative: **Santas, Jonatan**
**Lubrizol**
**Isaac Peral 17**
**(Pol. Industrial Camí Ral)**
**08850 Gavà (Barcelona) (ES)**

(56) References cited:
**WO-A1-2014/139904 WO-A1-2017/112586**
**WO-A1-2019/126162**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08F 220/1802, C08F 220/06, C08F 222/103**

## Description

### TECHNICAL FIELD

The present technology relates to an alkali-swellable emulsion prepared in the presence of a hydrocolloid stabilizer composition. More particularly, it relates to alkali swellable emulsion (ASE) and hydrophobically modified alkali-swellable emulsion (HASE) rheology modifiers prepared by the free radical emulsion polymerization of an acrylic monomer mixture in the presence of a stabilizer composition comprising polyvinyl alcohol (PVOH) and at least one saturated $C_2$ to $C_{50}$ mono- or di-acid and/or unsaturated $C_{10}$ to $C_{50}$ mono- or diacid to produce a starch-based stabilizer composition supported ASE or HASE thickener composition having improved stability, rheology and clarity properties, especially when such thickeners are employed in sulfate and sulfate-free surfactant systems.

It is known in the art to provide rheology modifiers that are emulsion polymers prepared from ethylenically unsaturated carboxylic acid monomer(s) (e.g., methacrylic acid), ethylenically unsaturated ester monomer(s) of a carboxylic acid (e.g., ethyl acrylate), and optional hydrophobically modified associative monomer(s) (e.g., behenyl ethoxylated methacrylate). When provided in acidic form, such polymers are referred to as alkali swellable emulsions (ASE) or hydrophobically modified alkali-swellable emulsions (HASE). When these polymers are neutralized with a base, they expand in water to act as a thickening agent. Such polymer emulsions are typically stabilized by surfactants, generally have a milky appearance, and when neutralized in aqueous surfactant containing media change from milky to clear. Commercially available ASE copolymer emulsions include, for example, Alcogum® L 11 marketed by Akzo Nobel Surface Chemistry LLC and Carbopol™ Aqua SF-1 marketed by Lubrizol Advanced Materials, Inc. Commercially available HASE copolymer emulsions include, for example, Aculyn™ 28 marketed by Dow and Novethix™ L-10 marketed by Lubrizol Advanced Materials, Inc.

The use of these emulsion polymers is advantageous in that the final product can be used "as is" and does not generally need to be altered or processed as compared to powdered polymers prepared by precipitation polymerization which have to be stripped of volatile organic solvent(s) and dried after recovery from the reactor. Moreover, the liquid nature of polymer emulsions allows for easier end product formulation versus the powdered polymers which require "wetting-out" before they can be homogeneously dispersed throughout a liquid medium.

Emulsion polymerization is a process that occurs in a three-phase reaction system including a continuous aqueous phase containing a dissolved initiator, droplets of monomer, and colloidal particles of monomer-swollen polymer. The initiator radical is formed in the aqueous phase. Essentially all of the polymerization reaction occurs in the monomer-polymer particles, with the monomer in the droplets being gradually transferred to the colloidal particles by dissolving in and diffusing through the aqueous phase. The monomer droplets and the swollen monomer-polymer particles are stabilized in the aqueous medium by absorbed surface-active agents, which are incorporated into the reaction mixture before the start of the polymerization. Chain transfer agents or modifiers can be added to the reaction mixture to limit the molecular weight. Emulsion polymerization is described in the Kirk-Othmer Encyclopedia of Chemical Technology, copyright 2020, John Wiley & Sons, DOI: 10.1002/0471238961.koe00054.

However, conventional emulsion polymerization techniques have some disadvantages. To produce typical latex polymer emulsions used in paint, adhesive and thickener applications, an emulsion stabilizer, or surfactant must be used in order to prevent or minimize the formation of agglomerates during the polymerization process and to ensure that the emulsion polymerization is stably carried out and the desired end-product has shelf-life stability against coagulation. Traditionally, anionic surfactants have been used to stabilize latex particle size during polymerization and nonionic surfactants have been employed to convey shear and freeze-thaw stability to the latex end-product. However, when the polymer emulsion is recovered from the reactor, the surfactant remains in the polymer emulsion as an "impurity" which may have a harmful effect on products formulated with the emulsion. Accordingly, it is desired that the stabilizer be used in a minimum amount so as not to deleteriously influence the performance of the end product in which the emulsion polymer is an added component.

International Publication No. WO2014/139904 discloses a rheology modifier in which carboxylic acid and ester monomers are copolymerized in the presence of a polysaccharide to prepare alkali swellable emulsions. When neutralized these polymer emulsions are said to change from milky to clear. In one embodiment, the polysaccharide is a water-soluble starch. Advantageously, these starch-modified copolymer emulsions do not require the use of a surfactant because the starch allows the emulsion to self-stabilize. The disclosed starch supported rheology modifiers are said to be able to suspend active ingredients in a formulation and are also partly biodegradable. However, they have been shown to have the same problems with short shelf-life as the non-starch-based copolymer emulsions. Moreover, when such starch supported latex emulsions are formulated into a sulfate-free anionic surfactant chassis, the viscosities and clarity properties of these systems suffer.

Sulfate-based surfactants (e.g., sodium lauryl sulfate and sodium lauryl ether sulfate) are particularly popular because of their effectiveness in cleansing, foam production, and stability. Personal care cleansers containing sulfate-based surfactants are also generally easy to thicken with typical thickeners, such as salt, cellulose-based and synthetic

materials. Nonetheless, these surfactants can be harsh and irritating to skin. For instance, continued use of sulfate-based surfactants may cause drying and/or irritation to the skin and scalp and contribute to color fading and drying of hair. Eliminating sulfate surfactants from cleansing compositions has been challenging because sulfate-free compositions typically have poor foaming properties, are difficult to thicken, and may not provide the desired degree of clarity or transparency.

Consumers expect that personal care compositions such as body wash, shampoo, and hand cleanser formulations will exhibit a suitable viscosity. This ideal viscosity serves at least two purposes. First, it improves handling and spreading of the composition. Second, it acts as a sensory cue that consumers tend to associate with product efficacy. Achieving an acceptable composition viscosity is an important factor in providing a mild personal cleansing composition that can be applied in a controlled manner and readily spread in use. Composition viscosity, together with attributes such as product clarity can also impact consumer perception of such products. When sulfate surfactants are eliminated, developing a microstructure that results in desirable rheological properties can be challenging, particularly in the case of mild compositions with low surfactant concentrations. Building the viscosity of such compositions by the addition of simple salts, natural gums, synthetic polymers, and natural/synthetic polymer hybrids can be problematic.

WO2017/112586 A1 discloses emulsion polymers prepared from a polymerizable monomer composition comprising: (A) at least one acidic vinyl monomer; (B) at least one nonionic vinyl monomer; (C) at least one alkoxylated associative monomer; (D) at least one polyunsaturated amphiphilic macromonomer; and optionally one or more of, (E) at least one semihydrophobic monomer; and (F) at least one crosslinking monomer.

WO2019/126162 A1 discloses detersive composition comprising a. water, b. a crosslinked nonionic polymer prepared from a monomer mixture comprising i) at least one $C_1$ to $C_5$ hydroxyalkyl ester of (meth)acrylic,at least one $C_1$ to $C_5$ alkyl ester of (meth)acrylic acid, an associative monomer, and at least one polyunsaturated crosslinker monomer; and c) an oil phase.

Accordingly, there is an increasing demand for sustainable, less irritating and/or environmentally friendly personal care products that are sulfate-free, but which have improved properties over sulfate-free products formulated with conventionally prepared rheology modifiers.

## SUMMARY OF THE DISCLOSED TECHNOLOGY

This section describes several aspects of the present technology of relevance to the claimed invention. The invention as claimed is, however, as defined in the appended claim set.

In accordance with one aspect of the present technology there is provided an alkali-swellable emulsion (ASE) polymer or a hydrophobically modified alkali-swellable emulsion (HASE) polymer prepared by the free radical polymerization of a monomer mixture comprising: a) at least one ethylenically unsaturated acid group containing monomer; b) at least one alkyl ester of (meth)acrylic acid; c) an optional monomer selected from an ethylenically unsaturated associative monomer, an ethylenically unsaturated semi-hydrophobic monomer, and mixtures thereof; and d) an optional crosslinking monomer in the presence of a stabilizer composition comprising digested starch, polyvinyl alcohol and at least one saturated $C_2$ to $C_{50}$ mono- or di-acid and/or unsaturated $C_{10}$ to $C_{50}$ mono- or diacid to yield a stable a copolymer emulsion.

The disclosed ASE and HASE polymers of the present technology can be prepared with or without a surfactant in the polymerization medium.

In accordance with one aspect of the present technology, there is provided an alkali-swellable emulsion (ASE) polymer or a hydrophobically modified alkali-swellable emulsion (HASE) polymer prepared by the free radical polymerization of a monomer composition comprising:

a) from 20 to 60 parts by wt., or from 30 to 50 parts by wt., or from 35 to 40 parts by wt. of at least one ethylenically unsaturated $C_3$-$C_6$ acid group containing monomer;
b) from 40 to 75 parts by wt., or from 50 to 70 parts by wt., or from 60 to 68 parts by wt. of at least one $C_1$ to $C_{30}$ alkyl ester of (meth)acrylic acid;
c) from 0 to 15 parts by wt., or from 0.5 to 10 parts by wt., or from 1 to 5 parts by wt. of at least one monomer selected from an associative monomer, a semi-hydrophobic monomer, and mixtures thereof;
d) from 0 to 5 parts by wt., or from 0.05 to 3 parts by wt., or from 0.1 to 2 parts by wt., or from 0.3 to 1 part by wt. of at least one polyunsaturated crosslinking monomer; wherein the sum of a), b), c) and d) in said monomer mixture is 100 parts by wt., and wherein said monomer mixture is polymerized in the presence of a stabilizer composition comprising:

i) from 0.1 to 75 parts by wt., or from 0.5 to 60 parts by wt., or from 1 to 55 parts by wt., or from 5 to 35 parts by wt., or from 10 to 30 parts by wt. of an enzymatically digested starch per 100 parts by wt. of said monomer composition;
ii) from 0.001 to 2 parts by wt., or from 0.005 to 1.5 parts by wt., or from 0.05 to 1 parts by wt., or from 0.1 to 0.75 parts by wt., or from 0.15 to 0.5 parts by wt. of PVOH per 100 parts by wt. of said polymerizable monomer composition; and

iii) from 0.001 to 0.5 parts by wt., or from 0.005 to 0.4 parts by wt., or from 0.01 to 0.3 parts by wt., or from 0.03 to 0.2 parts by wt., or from 0.05 to 0.1 parts by wt. of at least one acid selected from at least one saturated $C_2$ to $C_{50}$ mono- or di-acid and/or unsaturated $C_{10}$ to $C_{50}$ mono- or diacid, and mixtures thereof per 100 parts by wt. of said polymerizable monomer composition.

In accordance with one aspect of the present technology, there is provided an alkali-swellable emulsion (ASE) polymer or a hydrophobically alkali-swellable emulsion (HASE) polymer prepared by the free radical polymerization of a polymerizable monomer composition comprising:

a) from 30 to 40 parts by wt. of an ethylenically unsaturated carboxylic acid monomer selected from acrylic acid, methacrylic acid, and mixtures thereof;
b) from 60 to 70 parts by wt. of an alkyl ester of (meth)acrylic acid selected from ethyl acrylate, ethyl methacrylate, butyl acrylate, butyl methacrylate, and mixtures thereof;
c) from 0 to 15 parts by wt. of an optional associative monomer selected from lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), wherein the polyethoxylated moiety contains from about 5 to about 60 ethylene oxide units; and
d) from 0 or from 0.1 to 2 parts by wt. of at least one polyunsaturated crosslinking monomer; wherein the sum of a), b), c) and d) in said monomer composition is 100 parts by wt.; and wherein said monomer mixture is polymerized in the presence of a stabilizer composition comprising:

i) from 10 to 30 parts by wt. of a digested starch per 100 parts by wt. of said polymerizable monomer composition;
ii) from 0.1 to 0.5 parts by wt. of PVOH per 100 parts by wt. of said polymerizable monomer composition; and
iii) from 0.01 to 0.3 parts by wt. of at least one acid selected from a saturated $C_2$ to $C_{50}$ mono- or di-acid and/or unsaturated $C_{10}$ to $C_{50}$ mono- or diacid, and mixtures thereof per 100 parts by wt. of said polymerizable monomer composition.

In accordance with one aspect of the present technology, there is provided an alkali-swellable emulsion (ASE) polymer prepared by the free radical polymerization of a polymerizable monomer composition comprising:

a) from 30 to 40 parts by wt. of an ethylenically unsaturated carboxylic acid monomer selected from acrylic acid, methacrylic acid, and mixtures thereof;
b) from 60 to 70 parts by wt.an alkyl ester of acrylic acid selected from ethyl acrylate, butyl acrylate, and mixtures thereof;
d) from 0.1 to 2 parts by wt. of at least one polyunsaturated crosslinking monomer; wherein the sum of a), b), and d) in said polymerizable monomer composition totals 100 parts by wt.; and wherein said monomer mixture is polymerized in the presence of a stabilizer composition comprising:

i) from 10 to 30 parts by wt. of a digested starch per 100 parts by wt. of said polymerizable monomer composition;
ii) from 0.1 to 0.5 parts by wt. of PVOH per 100 parts by wt. of said polymerizable monomer composition; and
iii) from 0.01 to 0.3 parts by wt. of at least one acid comprising lauric acid per 100 parts by wt. of said polymerizable monomer composition.

In accordance with one aspect of the present technology, there is provided a hydrophobically modified alkali-swellable emulsion (HASE) polymer prepared by the free radical polymerization of a polymerizable monomer composition comprising:

a) from 30 to 50 parts by wt. of at least one ethylenically unsaturated acid group containing monomer selected from acrylic acid, methacrylic acid, and mixtures thereof;
b) from 40 to 60 parts by wt. of at least one alkyl ester of (meth)acrylic acid selected from ethyl acrylate, butyl acrylate, and mixtures thereof;
c) from 0.1 to 15 parts by wt. of at least one associative monomer selected from lauryl polyethoxylated methacrylate (LEM), cetyl polyethoxylated methacrylate (CEM), cetearyl polyethoxylated methacrylate (CSEM), stearyl polyethoxylated (meth)acrylate, behenyl polyethoxylated methacrylate (BEM), and mixtures thereof, wherein the polyethoxylated moiety of said associative monomer contains from 2 to 30 ethylene oxide units; wherein the sum of a), b), and c) in said monomer mixture totals 100 parts by wt., and wherein said monomer mixture is polymerized in the presence of a stabilizer composition comprising:

i) from 10 to 30 parts by wt. of a digested starch per 100 parts by wt. of said polymerizable monomer composition;
ii) from 0.1 to 0.5 parts by wt. of PVOH per 100 parts by wt. of said polymerizable monomer composition; and
iii) from 0.01 to 0.3 parts by wt. of at least one acid comprising lauric acid per 100 parts by wt. of said polymerizable monomer composition.

In one aspect, the present technology relates to dosing the starch-stabilized emulsion polymer latex prepared in accordance with the disclosed technology with an acid and/or reducing agent to further stabilize the latex polymer particles against coalescence and/or flocculation upon aging.

One aspect of the present technology relates to emulsion polymers prepared in the absence of emulsifying surfactant(s).

Also disclosed herein are uses for the disclosed copolymer emulsions as a thickener in personal care formulations.

Also disclosed herein are uses for the disclosed copolymer emulsions as a thickener in surfactant containing personal care formulations.

Also disclosed herein are uses for the disclosed copolymer emulsions as a thickener in sulfate surfactant containing personal care formulations.

Also disclosed herein are uses for the disclosed copolymer emulsions as a thickener in sulfate-free surfactant containing personal care formulations.

Also disclosed herein are uses for the disclosed copolymer emulsions as a film former in personal care formulations.

Also disclosed herein are uses for the disclosed copolymer emulsions as a hair fixative in personal care formulations.

## DETAILED DESCRIPTION

The polymers and compositions of the present invention may suitably comprise, consist of, or consist essentially of the components, elements, and process delineations described herein. The technology illustratively disclosed herein suitably may be practiced in the absence of any element which is not specifically disclosed herein.

In all aspects of the disclosed technology all percentages are calculated by the weight of the total composition. All ratios are expressed as weight ratios. All numerical ranges of amounts are inclusive and combinable unless otherwise specified.

While overlapping weight ranges for the various components and ingredients that can be contained in the disclosed compositions have been expressed for selected embodiments and aspects of the disclosed technology, the amount of each component in the disclosed compositions is selected from its disclosed range such that the sum of all components or ingredients in the composition will total 100 weight percent. The amounts employed will vary with the purpose and character of the desired product and can be readily determined by one skilled in the art.

The prefix "(meth)acrylic" includes "acrylic" as well as "methacrylic". For example, the term "(meth)acrylic acid" includes both acrylic acid and methacrylic acid.

Monomer composition means a composition of a mixture of comonomers which are polymerizable.

As defined herein, "stable" and "stability" means that no visible phase separation is observed for a period of at least about one week of storage, or at least about 1 month of storage, or at least about 6 months of storage at ambient room temperature (20 to about 25 °C). In another aspect, the products of the disclosed technology show no visible phase separation after about at least four weeks, or at least about 6 weeks, or at least about 8 weeks of storage, or at least about 12 weeks of storage at 45 °C.

As used herein the term "optically clear" or "transparent" refers to compositions of the present technology having turbidity value that is equal to or less than 100 Nephelometric Turbidity Units (NTU), or equal to or less than 60 NTU, or equal to or less than 50 NTU, or equal to or less than 40 NTU, or equal to or less than 30 NTU, or equal to or less than 20 NTU, or equal to or less than 15 NTU, as measured by the Turbidity Test described in the test protocol hereinbelow (a lower NTU value relates to a composition that is clearer or more transparent than a composition having a higher NTU value).

Advantageously, the ASE and HASE polymers of the disclosed technology can be employed, without limitation, in personal care products, health care products, home care products, institutional and industrial care products, and the like and in industrial chemical processes and applications as, for example, rheology modifiers, film formers, thickeners, emulsifiers, stabilizers, solubilizers, suspending agents, and the like. The disclosed ASE and HASE polymers are particularly useful as thickeners in personal care compositions, textile treatment compositions for finishing, coating, and printing applications, and in industrial paints and coatings.

In particular, the HASE polymers of the disclosed technology are able to provide surfactant containing compositions over a wide concentration range with an ideal viscosity, long term suspension stability and clarity.

The term "personal care" as used herein includes, without being limited thereto, cosmetics, toiletries, cosmeceuticals, beauty aids, insect repellents, sunscreens, UV absorbers, hand sanitizers, personal hygiene and cleansing products (e.g., shampoos, conditioning shampoos, anti-dandruff shampoos, body washes, hand soaps, facial scrubs, and the like) applied to the body, including the skin, hair, scalp, and nails of humans and mammals.

The term "home care products" as used herein includes, without being limited thereto, products employed in a domestic household for surface cleaning or maintaining sanitary conditions, such as in the kitchen and bathroom (e.g., hard surface cleaners, hand and automatic dish care, toilet bowl cleaners and disinfectants), and laundry products for fabric care and cleaning (e.g., detergents, fabric conditioners, pre-treatment stain removers), and the like.

The term "health care products" as used herein includes, without being limited thereto, pharmaceuticals (controlled release pharmaceuticals), pharmacosmetics, oral care (mouth and teeth) products, such as oral suspensions, mouthwashes, toothpastes, dentifrices, and the like, and over-the-counter products and appliances (topical and transdermal), such as patches, plasters and the like, externally applied to the body, including the skin, scalp, nails and mucous membranes of humans and animals, for ameliorating a health-related or medical condition, for generally maintaining hygiene or well-being, and the like.

The term "institutional and industrial care" ("|&|") as used herein includes, without being limited thereto, products employed for surface cleaning or maintaining sanitary conditions in institutional and industrial environments, textile treatments (e.g., textile conditioners, carpet and upholstery cleaners), automobile care (e.g., hand and automatic car wash detergents, tire shines, leather conditioners, liquid car polishes, plastic polishes and conditioners), paints and coatings, and the like.

As used herein, the term "rheological properties" and grammatical variations thereof, includes, without limitation such properties as Brookfield viscosity, increase or decrease in viscosity in response to shear stress, flow characteristics, gel properties such as stiffness, resilience, flowability, and the like, foam properties such as foam stability, foam density, ability to hold a peak, and the like, suspension properties such as yield value, and aerosol properties such as ability to form aerosol droplets when dispensed from propellant based or mechanical pump type aerosol dispensers.

The term "aesthetic property" and grammatical variations thereof as applied to compositions refers to visual and tactile psychosensory product properties, such as color, clarity, smoothness, tack, lubricity, texture, conditioning and feel, and the like.

Here, as well as elsewhere in the specification and claims, individual numerical values (including carbon atom numerical values), or limits, can be combined to form additional non-disclosed and/or non-stated ranges.

The headings provided herein serve to illustrate, but not to limit the invention in any way or manner.

Emulsion Stabilizer Package

Starch Component

The ASE and HASE polymers of the present technology are prepared by the free radical emulsion polymerization of a monomer composition in the presence of a stabilizer package comprising a digested starch, polyvinyl alcohol and an acid selected from at least one saturated $C_2$ to $C_{50}$ mono- or di-acid, at least one unsaturated $C_{10}$ to $C_{50}$ mono- or diacid, and mixtures thereof. Examples of suitable starches for use in the stabilizer composition are digested starches obtained from natural sources such as, for example, potato starch, corn starch, wheat starch, rice starch, tapioca starch, pea starch, sorghum starch, and mixtures thereof.

The degradation of the starches can be conducted 1) before the polymerization of the monomers, 2) during the polymerization of the monomers, 3) after the polymerization of the monomers, and 4) combinations of all three. The degradation reaction can be carried out oxidatively, thermally, acid hydrolytically, enzymatically, and combinations thereof. In one aspect, the molecular weight of the degraded starch should be in a range the permits the starch to be water soluble. As used herein by "water soluble" is meant that the starch has a solubility in water of at least from 1 to 100 mg/ml, or at least from 25 to 85 mg/ml, or at least from 50 to 75 mg/ml in water at 25 °C.

In one aspect, the weight average molecular weight ($M_w$) ranges from 10 to 200 kDa, or from 20 to 100 kDa, or from 30 to 80 kDa, or from 40 to 65 kDa, or from 45 to 56 kDa. The molecular weight ($M_w$) of the degraded starches can easily be determined by methods known in the art, for example, by means of gel permeation chromatography with the use of a multiangle light scattering detector.

In one aspect, the starches are digested with the aid of enzymes. The enzymatic degradation of the starch is carried out using techniques known in the art. The amount of enzyme used is dependent upon the enzyme type, source and activity, the starch material used, as well as the amount of degradation desired. Typically, the enzyme is used in an amount of from 0.001 to

1.0 wt. %, or from 0.01 to 0.8 wt. %, or from 0.1 to 0.6 wt.%, based on the weight of the starch.

In one aspect the starch is digestion or degradation reaction is conducted in aqueous media at a temperature of about 60 °C and a pH of 5.

Any of the known starch digestion enzymes can be used to digest the starch component of the stabilizer package.

Suitable enzymes include, but are not limited to, α-amylase, glucoamylase, pullulanase, isoamylase, maltogenic amylase, β-amylase, debranching enzymes, branching enzymes, maltogenic amylase, cyclodextrin glucotransferases, α-glucanotranferases, glucanosyltransferases, and mixtures thereof.

In one aspect, a suitable starch digestion enzyme is pullulanase which typically is isolated from plants, bacteria, or fungi. Pullulanase enzyme is commercially available from Bio-Cat, Inc. marketed under Bacterial Pullulanase L. In another aspect, a suitable digestion enzyme is alpha/beta-amylase and combinations of amylase and Pullulanase.

The starch can be pre-digested, post-digested, or digested *in situ* in the polymerization reactor.

In one aspect, the starch(es) can be pre-digested in an aqueous medium and combined with PVOH and an acid selected from at least one saturated $C_2$ to $C_{50}$ mono- or di-acid, at least one unsaturated $C_{10}$ to $C_{50}$ mono- or diacid, and combinations thereof to form a pre-formed stabilizer package which can be added to the reactor containing the monomer to be polymerized by batch or by metering, or combinations of batch addition and metering. Alternatively, the pre-formed stabilizer package can be pre-mixed with the monomer components and added to the reactor by batch or by metering.

In another aspect, the pre-formed stabilizer package, and the monomers to be polymerized can be metered into the reactor in separate streams.

In another aspect, the neat, pre-digested starch component, the PVOH component and the acid component of the stabilizer package can each be separately added to the reactor by batch addition, by metering, or by combinations of batch addition and metering.

In one exemplary aspect, the starch component can be digested *in situ* in the polymerization reactor. In this aspect, undigested starch, PVOH, the at least one acid selected from at least one saturated $C_2$ to $C_{50}$ mono- or di-acid, at least one unsaturated $C_{10}$ to $C_{50}$ mono- or diacid, and combinations thereof, water, and surfactant is charged to the reactor where the pH of the reactor contents is adjusted to about 5. The reactor contents are heated to a temperature of about 60 °C which is held for about 120-minutes. Following the hold time, enzyme is fed into the mixture and allowed to digest the starch for 30-minutes. After the starch digestion, the reactor is heated to a reaction temperature of 85 °C after which, monomer and initiator is added to the reactor by any combination of the batch or metering addition methods described above. In one aspect, the monomer component is continuously metered into the reactor over the course of 150-minutes. Following the feed, the reactor contents are held at 85 °C for 60-minutes and then cooled to room temperature and recovered.

Polyvinyl Alcohol (PVOH) Component

The PVOH component of the stabilizer package is selected from fully hydrolyzed PVOH, partially hydrolyzed PVOH, and mixtures thereof. Poly(vinyl alcohol) compositions can be obtained by known and conventional methods. Poly(vinyl alcohol) is typically obtained through polymerization of vinyl acetate monomer, followed by conversion of the poly(vinyl acetate) to PVOH through hydrolysis. These PVOH products are typically referred to as "hydrolyzed" or "partially hydrolyzed". The term "partially hydrolyzed" means that not all of the acetate groups are completely converted to alcohol groups. When poly(vinyl acetate) homopolymer is only partially hydrolyzed, the resulting PVOH is actually a vinyl alcohol/vinyl acetate copolymer. However, as noted, such polymers are generally referred to as partially hydrolyzed PVOH homopolymers. In one aspect, the partially hydrolyzed PVOH has a degree of hydrolysis ranging from 80 to 98 percent, or from 83 to 95 percent, or from 85 to 93 percent. In one aspect, the molecular weight of the PVOH ranges from 1 kDa to 500 kDa, or from 2 kDa to 400 kDa, or from 5 kDa to 200 kDa, or from 8 kDa to 100 kDa, or from 10 to 50 kDa, or from 11 kDa to 25 kDa.

Commercially available PVOH, of varying degrees of hydrolysis, is marketed by Sekisui Specialty Chemicals (trade name Selvol™), Kuraray America, Inc. (trade names Polval™ and Exceval™), and Sigma-Aldrich.

Acid Component

The acid component of the stabilizer package of the present technology is selected from at least one saturated $C_2$ to $C_{50}$ mono- or di-acid, at least one unsaturated $C_{10}$ to $C_{50}$ mono- or diacid, and combinations thereof. Representative saturated $C_2$ to $C_{50}$ mono- and diacids and unsaturated $C_{10}$ to $C_{50}$ mono- and diacids include, but are not limited to acetic acid, caproic acid, caprylic acid, pelargonic acid, capric acid, undecanoic acid, lauric acid, myristic acid, palmitic acid, palmitoleic acid, stearic acid, isostearic acid, hydroxy stearic acid, oleic acid, ricinoleic acid, vaccenic acid, linolenic acid, α-linolenic acid, γ-linolenic acid, arachidic acid, gadoleic acid, arachidonic acid, eicosapentaenoic acid, behenic acid, docosahexaenoic acid, lignoceric acid, malonic acid, succinic acid, glutaric acid, adipic acid, pimelic acid, suberic acid, azelaic acid, undecanedioic acid, dodecandioic acid, brassylic acid, thapsic acid japanic acid, phellogenic acid, equisetolic acid, citric acid, isocitric acid, aconitic acid, propane-1,2,3-tricarboxylic acid, agaric acid, trimesic acid, tartronic acid, mesoxalic acid, malic acid, tartaric acid, oxaloacetic acid aspartic acid, dioxosuccinic acid, and mixtures thereof.

Dimer acids (also known as dimerized fatty acids or dimer fatty acids) containing 24 to 44 carbon atoms can used as a diacid component in the stabilizer package. Dimer acids are described by T. E. Breuer, "Dimer Acids", Kirk-Othmer Encyclopedia of Chemical Technology, 4th Ed., John Wiley & Sons, New York, 1993, Vol. 8, pp. 223-237. They are

prepared, for example, by catalytic dimerization of unsaturated plant fatty acids at elevated temperature and pressure on a clay catalyst and then removing most of the unreacted fatty acid starting materials by distillation. The starting materials are unsaturated $C_{12}$ to $C_{22}$ fatty acids obtained from natural sources such as tall oil and through dimerization yield $C_{24}$ to $C_{44}$ dimer acids.

Examples of commercially available dimer acids include but are not limited to Unidyme™ 14 and Unidyme™ 22, marketed by Arizona Chemical Company and Pripol™ 1006, Pripol™ 1013 and Pripol™ 1017 marketed by Croda International Plc.

Hydroxy acids (alpha and beta) as well as amino acids (natural and synthetic) are suitable acid components of the stabilizer package. Hydroxy acids include but are not limited to. glycolic acid, oxalic acid, lactic acid, malic acid, tartaric acid, salicylic acid, citric acid, carnitine, mandelic acid, fumaric acid, tartaric acid, and mixtures thereof. Suitable amino acids include, but are not limited to alanine, cysteine, aspartic acid, glutamic acid, phenylalanine, glycine, histidine, isoleucine, lysine, leucine, methionine, asparagine, proline, glutamine, arginine, serine, threonine, valine, tryptophan, tyrosine, ornithine, 2,3 diamino propionic acid, 2,4 diamino butyric acid and/or derivatives thereof.

The stabilizer package comprises: i) from 0.1 to 75 parts by wt., or from 0.5 to 60 parts by wt., or from 1 to 55 parts by wt., or from 5 to 35 parts by wt., or from 10 to 30 parts by wt. of the enzymatically digested starch component per 100 parts by wt. of the monomer composition; ii) from 0.001 to 2 parts by wt., or from 0.005 to 1.5 parts by wt., or from 0.05 to 1 parts by wt., or from 0.1 to 0.75 parts by wt., or from 0.15 to 0.5 parts by wt. of the PVOH per 100 parts by wt. of the monomer composition; and iii) from 0.001 to 0.5 parts by wt., or from 0.005 to 0.4 parts by wt., or from 0.01 to 0.3 parts by wt., or from 0.03 to 0.2 parts by wt., or from 0.05 to 0.1 parts by wt. of at least one acid selected from a saturated $C_2$ to $C_{50}$ mono- or diacid, an unsaturated $C_{10}$ to $C_{50}$ mono- or diacid, and combinations thereof per 100 parts by wt. of the monomer composition.

Monomers

The polymer composition of the present technology is prepared by polymerizing a monomer composition comprising:

a) from 25 to 60 parts by wt., or from 30 to 50 parts by wt., or from 35 to 40 parts by wt. of at least one ethylenically unsaturated $C_3$-$C_6$ acid group containing monomer;
b) from 40 to 75 parts by wt., or from 50 to 70 parts by wt., or from 60 to 68 parts by wt. of at least one $C_1$ to $C_{30}$ alkyl ester of (meth)acrylic acid;
c) from 0 to 15 parts by wt., or from 0.1 to 8 parts by wt., or from 1 to 5 parts by wt. of at least one monomer selected from an associative monomer, a semi-hydrophobic monomer, and mixtures thereof; and
d) from 0 to 5 parts by wt., or from 0.05 to 3 parts by wt., or from 0.1 to 2 parts by wt., or from 0.3 to 1 part by wt. of at least one polyunsaturated crosslinking monomer; wherein the sum of a), b), c) and d) in said monomer composition totals 100 parts by wt.

Exemplary component (a) monomers include but are not limited to acrylic acid, methacrylic acid, itaconic acid, citraconic acid, maleic acid, maleic anhydride, fumaric acid, crotonic acid, aconitic acid, 2-acrylamido-2-methyl-1-propanesulfonic acid (AMPS™ monomer) and the inorganic and organic salts thereof (e.g., ammonium, organic amine and alkali metal salts), and mixtures thereof.

In one aspect, the amount of the at least one ethylenically unsaturated $C_3$-$C_6$ acid group containing monomer set forth under monomer component (a) in the polymerizable monomer composition ranges from about 25 to about 60 parts by wt., or from 30 to 50 parts by wt., or from 35 to 40 parts by wt.

Exemplary component (b) monomers include but are not limited to methyl acrylate, methyl methacrylate, ethyl acrylate, ethyl methacrylate, hydroxyethyl acrylate, hydroxyethyl methacrylate, n-propyl acrylate, n-propyl methacrylate, isopropyl acrylate, isopropyl methacrylate, hydroxypropyl acrylate, hydroxypropyl methacrylate, n-butyl acrylate, n-butyl methacrylate, isobutyl acrylate, isobutyl methacrylate, tert-butyl acrylate, tert-butyl methacrylate, sec-butyl acrylate, sec-butyl methacrylate, hydroxybutyl acrylate, hydroxybutyl methacrylate, n-pentyl acrylate, n-pentyl methacrylate, neopentyl acrylate, neopentyl methacrylate, cyclohexyl acrylate, cyclohexyl methacrylate, 2-hexyl acrylate, 2-hexyl methacrylate, 2-ethylhexyl acrylate, 2-ethylhexyl methacrylate, n-octyl acrylate, n-octyl methacrylate, isooctyl acrylate, isooctyl methacrylate, decyl acrylate, decyl methacrylate, isodecyl acrylate, isodecyl methacrylate, dodecyl acrylate, dodecyl methacrylate, 2-propylheptyl acrylate and 2-propylheptyl methacrylate, lauryl acrylate, lauryl methacrylate, cetyl acrylate, cetyl methacrylate, stearyl acrylate, stearyl methacrylate, behenyl acrylate, behenyl methacrylate, melissyl acrylate, melissyl methacrylate, and mixtures thereof.

In one aspect, the amount of the at least one $C_1$ to $C_{30}$ alkyl ester of (meth)acrylic acid set forth under monomer component (b) in the polymerizable monomer composition ranges from 40 to 75 parts by wt., or from 50 to 70 parts by wt., or from 60 to 68 parts by wt.

The component (c) associative monomers are represented by formula I and formula II below.

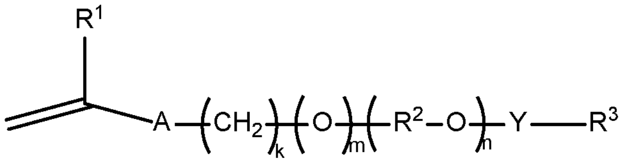

I

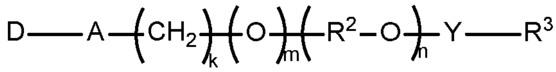

II

wherein $R^1$ is hydrogen or methyl; A is $-CH_2C(O)O-$, $-C(O)O-$, $-O-$, $-CH_2O-$, $-NHC(O)NH-$, $-C(O)NH-$, $-Ar-(CE_2)_2-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; $(R^2-O)_n$ is a polyoxyalkylene moiety, which can be a homopolymer, a random copolymer, or a block copolymer of $C_2$-$C_4$ oxyalkylene units; $R^2$ is a divalent alkylene moiety selected from $C_2H_4$, $C_3H_6$, or $C_4H_8$, and combinations thereof; and n is an integer in the range of 2 to 150 , or from 10 to 120, or from 15 to 60 ; Y is $-R^2O-$, $-R^2NH-$, $-C(O)-$, $-C(O)NH-$, $-R^2NHC(O)NH-$, or $-C(O)NHC(O)-$; $R^3$ is a substituted or unsubstituted alkyl selected from a $C_8$-$C_{50}$ linear alkyl, a $C_8$-$C_{50}$ branched alkyl, a $C_8$-$C_{30}$ carbocyclic alkyl, a $C_2$-$C_{30}$ alkyl-substituted phenyl, an aralkyl substituted phenyl, and an aryl-substituted $C_2$-$C_{30}$ alkyl; wherein the $R^3$ alkyl group, aryl group, phenyl group optionally comprises one or more substituents selected from the group consisting of a hydroxyl group, an alkoxyl group, benzyl group styryl group, and a halogen group.

In one aspect, the component (c) associative monomers are represented by formula III below.

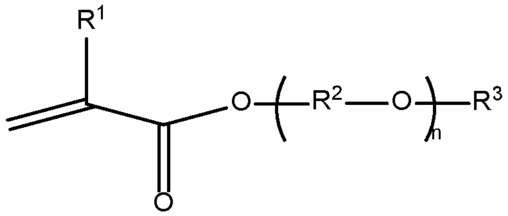

III

wherein $R^1$ is hydrogen or methyl; $R^2$ is a divalent alkylene moiety independently selected from $C_2H_4$, $C_3H_6$, and $C_4H_8$, and n represents an integer ranging from 5 to 60, $(R^2-O)$ can be arranged in a random or a block configuration; $R^3$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an aralkyl substituted phenyl, and an aryl-substituted $C_2$-$C_{30}$ alkyl, wherein the $R^3$ alkyl group, aryl group, phenyl group optionally comprises one or more substituents selected from the group consisting of a hydroxyl group, an alkoxyl group, benzyl group styryl group, and a halogen group.

Exemplary component (c) monomers include but are not limited to lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, wherein the polyethoxylated moiety of the monomer contains from 2 to 150, or from 5 to 120, or from 10 to 60, or from 15 to 30 ethylene oxide units.

The component (c) semi-hydrophobic monomers are represented by formula IV and formula V below.

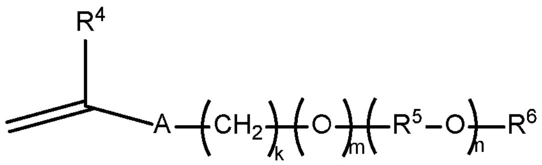

IV

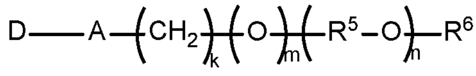

V

wherein $R^4$ is hydrogen or methyl; A is $-CH_2C(O)O-$, -C(O)O-, -O-, $-CH_2O-$, -NHC(O)NH-,-C(O)NH-, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; $(R^5-O)_n$ is a polyoxyalkylene moiety, which can be a homopolymer, a random copolymer, or a block copolymer of $C_2$-$C_4$ oxyalkylene units, $R^5$ is a divalent alkylene moiety selected from $C_2H_4$, $C_3H_6$, or $C_4H_8$, and combinations thereof; and n is an integer in the ranging from 2 to 150, or from 5 to 120, or from 10 to 60; $R^6$ is selected from hydrogen and a linear or branched $C_1$-$C_4$ alkyl group; and D represents a vinyl or an allyl moiety.

In one aspect, the component (c) semi-hydrophobic monomers are represented by formula VI and formula VII below.

$$CH_2=C(R^4)C(O)O-(C_2H_4O)_a(C_3HeO)_b-H \quad \text{VI}$$

$$CH_2=C(R^4)C(O)O-(C_2H_4O)_a(C_3H_6O)_b-CH_3 \quad \text{VII}$$

wherein $R^4$ is hydrogen or methyl, and "a" is an integer ranging from 0 or 2 to 120, or from 5 to 45, or from 10 to 25 in a further aspect; and "b" is an integer ranging from 0 to 120, or from 2 to 45, or from 10 to 25, subject to the proviso that "a" and "b" cannot be 0 at the same time.

Examples of semi-hydrophobic monomers under formula VI include polyethyleneglycol methacrylate available under the product names Blemmer® PE-90 ($R^4$ = methyl, a = 2, b = 0), PE-200 ($R^4$ = methyl, a = 4.5, b = 0), and PE-350 ($R^4$ = methyl a = 8, b = 0,); polypropylene glycol methacrylate available under the product names Blemmer® PP-1000 ($R^4$ = methyl, b = 4-6, a = 0), PP-500 ($R^4$ = methyl, a = 0, b = 9), PP-800 ($R^4$ = methyl, a = 0, b = 13); polyethyleneglycol polypropylene glycol methacrylate available under the product names Blemmer® 50PEP-300 ($R^4$ = methyl, a = 3.5, b = 2.5), 70PEP-350B ($R^{14}$ = methyl, a = 5, b = 2); polyethyleneglycol acrylate available under the product names Blemmer® AE-90 ($R^4$ = hydrogen, a = 2, b = 0), AE-200 ($R^4$ = hydrogen, a = 2, b = 4.5), AE-400 ($R^4$ = hydrogen, a = 10, b = 0); polypropyleneglycol acrylate available under the product names Blemmer® AP-150 ($R^4$ = hydrogen, a = 0, b = 3), AP-400($R^4$ = hydrogen, a = 0, b = 6), AP-550 ($R^4$ = hydrogen, a = 0, b = 9). Blemmer® is a trademark of NOF Corporation, Tokyo, Japan.

Examples of semi-hydrophobic monomers under formula VII include methoxypolyethyleneglycol methacrylate available under the product names Visiomer® MPEG 750 MA W ($R^4$ = methyl, a = 17, b = 0), MPEG 1005 MA W ($R^4$ = methyl, a = 22, b = 0), MPEG 2005 MA W ($R^4$ = methyl, a = 45, b = 0), and MPEG 5005 MA W ($R^4$ = methyl, a = 113, b = 0) from Evonik Röhm GmbH, Darmstadt, Germany); Bisomer® MPEG 350 MA ($R^4$ = methyl, a = 8, b = 0), and MPEG 550 MA ($R^4$ = methyl, a = 12, b = 0) from GEO Specialty Chemicals, Ambler PA; Blemmer® PME-100 ($R^4$ = methyl, a = 2, b = 0), PME-200 ($R^4$ = methyl, a = 4, b = 0), PME400 ($R^4$ = methyl, a = 9, b = 0), PME-1000 ($R^4$ = methyl, a = 23, b = 0), PME-4000 ($R^4$ = methyl, a = 90, b = 0).

In one aspect, the amount of the at least one associative monomer and/or semi-hydrophobic monomer set forth under monomer component (c) in the polymerizable monomer composition ranges from 0 to 15 parts by wt., or from 0.5 to 10 parts by wt., or from 1 to 5 parts by wt.

Exemplary component (d) polyunsaturated crosslinking monomers include but are not limited to ethylene glycol di(meth)acrylate, triethylene glycol di(meth)acrylate, 1,3-butylene glycol di(meth)acrylate, 1,4-butylene glycol di(meth) acrylate, 1,6-hexanediol di(meth)acrylate, neopentyl glycol di(meth)acrylate, 1,9-nonanediol di(meth)acrylate, trimethylolpropane tri(meth)acrylate, trimethylolethane tri(meth)acrylate, tetramethylolmethane tri(meth)acrylate, ditrimethylolpropane tetra(meth)acrylate, tetramethylolmethane tetra(meth)acrylate, pentaerythritol tetra(meth)acrylate; dipentaerythritol hexa(meth)acrylate, 1,4-cyclohexanediol dimethacrylate, allyl (meth)acrylate, diallylphthalate, diallyl itaconate, diallyl fumarate, and diallyl maleate; polyallyl ethers of sucrose having from 2 to 8 allyl groups per molecule, polyallyl ethers of pentaerythritol, polyallyl ethers of trimethylolpropane, amphiphilic polyunsaturated macromonomers, and mixtures thereof.

The amphiphilic polyunsaturated macromonomer utilized in the present technology contains a hydrophobic moiety and a hydrophilic moiety. Exemplary amphiphilic polyunsaturated macromonomers suitable for use with the present technology include, but not be limited to, compounds such as those disclosed in US Patent No. 9,051,341, represented by the following formulas:

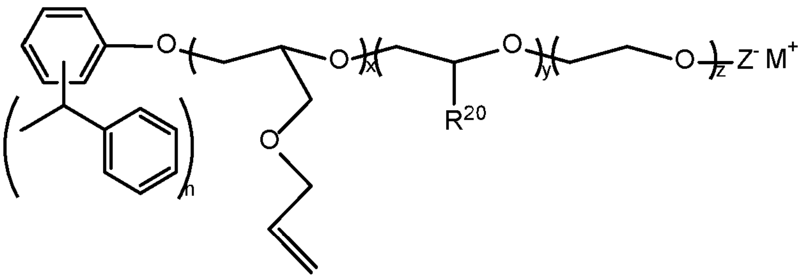

(VIII)

wherein $R^{20}$ is $CH_3$, $CH_2CH_3$, $C_6H_5$, or $C_{14}H_{29}$; n is 1, 2, or 3; x is 2-10, y is 0-200, z is 4-200, or from 5 to 60, or from 5 to 40; Z can be either $SO_3^-$ or $PO_3^{2-}$, and $M^+$ is $Na^+$, $K^+$, $NH_4^+$, or an alkanolamine such as, for example, monoethanolamine, diethanolamine, and triethanolamine;

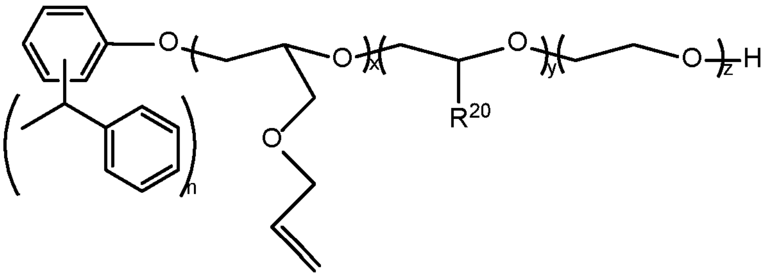

(IX)

wherein $R^{20}$ is $CH_3$, $CH_2CH_3$, $C_6H_5$, or $C_{14}H_{29}$; n is 1, 2, 3; x is 2-10, y is 0-200, z is 4-200, or from 5 to 60, or from 5 to 40;

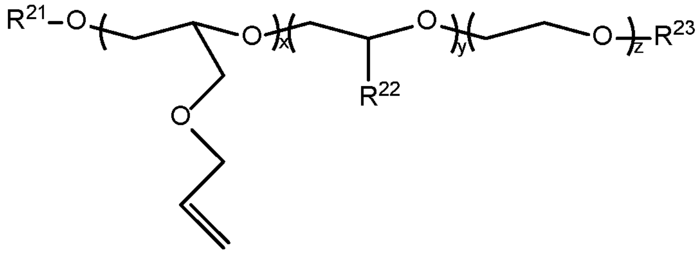

(X)

where $R^{21}$ is a $C_8$-$C_{30}$ alkyl, alkaryl, alkenyl, or cycloalkyl group in one aspect, and a $C_{10}$-$C_{24}$ alkyl, aryl, alkylaryl, and aralkylaryl group in another aspect; $R^{22}$ is $CH_3$, $CH_2CH_3$, $C_6H_5$, or $C_{14}H_{29}$; x is 2-100, or 2-10, y is 0-200, or from 0 or 1-50, z is 4-200, or from 5 to 60, or from 5-40; and $R^{23}$ is H or $Z^-$ $M^+$, where Z can be either $SO_3^-$ or $PO_3^{2-}$, and $M^+$ is $Na^+$, $K^+$, $NH_4^+$, or an alkanolamine such as, for example, monoethanolamine, diethanolamine, and triethanolamine.

In one aspect, the polyunsaturated macromonomer is selected from the compounds represented by formulas (XI) or (XII) as follows:

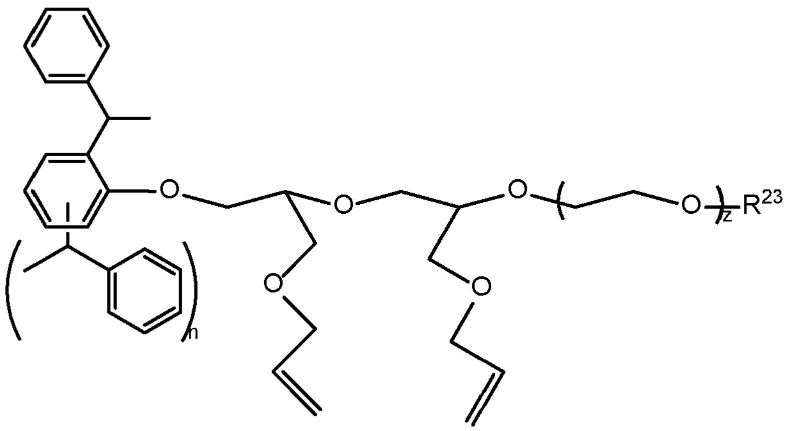

(XI)

wherein n is 1 or 2; z is 4-40, or 5-38, or 10-20; and $R^{23}$ is H, $SO_3^-M^+$ or $PO_3^{2-}$ $M^+$, and M is selected from $Na^+$, $K^+$, and $NH_4^+$,

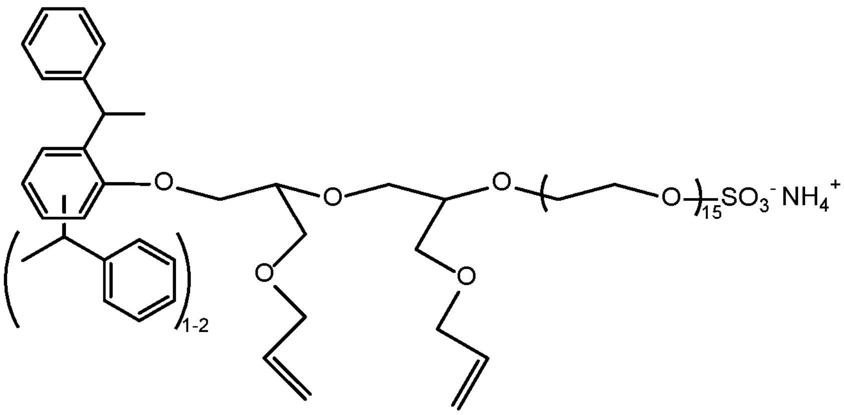

(XII)

In one aspect, the amount of the at least one polyunsaturated crosslinking monomer set forth under monomer component (d) in the polymerizable monomer composition ranges from 0 to 10 parts by wt., or from 0.05 to 8 parts by wt., or from 0.1 to 5 parts by wt., or from 0.15 to 3 parts by wt., or from 0.2 to 2 parts by wt., based on 100 parts by wt. of the polymerizable monomer composition.

In one aspect, the amphiphilic polyunsaturated macromonomer contains an average of 1.5 to 2 unsaturated moieties in the molecule.

The sum of monomers selected from component (a), component (b), component (c) and component (d) in said polymerizable monomer composition totals 100 parts by wt.

Emulsion Process

The ASE and HASE polymers of the technology can be synthesized via emulsion polymerization techniques well known in the art such as set forth in U.S. Patent No. 4,138,380, or U.S. Patent No. 4,110,291

In one aspect, the polymers of the present technology can be synthesized in a staged, semi-batch, emulsion polymerization process to produce stable latex polymer particles. The emulsion polymerization of monomers (a) to (d) is conducted in an aqueous medium in the presence of the stabilizer package comprising the degraded starch, PVOH and the at least one acid selected from a saturated $C_2$ to $C_{50}$ mono- or diacid and/or an unsaturated $C_{10}$ to $C_{50}$ mono- or diacid, and mixtures thereof described hereinabove. The addition of the stabilizer package components to the reactor can be carried out as previously described, e.g., by batch addition and metering, or a combination of both. Additionally, the undigested starch component can be added to the reactor to be enzymatically digested *in situ.*

The monomers can be added to the reactor either separately or as a mixture by batch addition or by metering. If the monomer addition is by metering, the addition can be conducted uniformly or nonuniformly, i.e., by changing the metering rate over the feed interval.

In one exemplary aspect, the mixture of monomers (a), (b), and (c) is charged to an agitator equipped first reactor under inert atmosphere, e.g., nitrogen, and homogeneously mixed. Optionally, the monomer mixture can be charged to the first reactor containing deionized water and the desired emulsifying surfactant(s).

To a second reactor equipped with an agitator, an inert gas inlet, and metering feed pumps are added under inert an atmosphere a desired amount of deionized water and optional additional surfactant(s) and optional processing aids typically used in emulsion polymerization. The contents of the second reactor are heated to a temperature ranging from 45 to 70 °C with mixing agitation under a nitrogen atmosphere, after which the components of the stabilizer package (undigested starch, PVOH and the at least one acid selected from a saturated $C_2$ to $C_{50}$ mono- or diacid and/or an unsaturated $C_{10}$ to $C_{50}$ mono- or diacid) are charged to the reactor. Deionized water can be used to wash any reactants remaining on the reactor wall into the reaction medium. After the contents of the reactor are homogeneously mixed the pH of the mixture is adjusted to a suitable pH value for the selected starch digestion enzyme using an appropriate pH adjusting agent. After about a 1 to 4 hour hold time at a suitable temperature for the selected enzyme(s), a suitable amount of digestion enzyme(s) dissolved in deionized water is added to the reaction medium with continued mixing. The enzyme containing reaction mixture is held for 5 to 120-minutes at the optimal temperature for the selected enzyme(s) to digest the starch component, after which the reactor contents are heated to 65 to 99 °C. Alternatively, a similar enzyme treatment can be performed as post-polymerization procedure either alone or in combination with a pre-enzyme treatment. Upon reaching at temperature of 65 to 99 °C, an aqueous solution of a free radical polymerization initiator (e.g., ammonium persulfate) is metered into the reactor at a suitable feed rate. Simultaneously with the initiator feed, the monomer mixture from the first reactor is metered into the second reactor over a period of from 60 to 300-minutes.

In the second reactor, first stage latex particles are allowed to form for 10 to 60-minutes. In a second stage polymerization, a suitable amount of crosslinking monomer (d) mixed with a suitable amount monomer (b) is added to the

remaining monomers in the first reactor. The temperature of the second reactor contents is raised to 70 to 99 °C, whereupon the remaining first reactor monomers (containing the crosslinking monomer) are metered into the second reactor over the remainder of the 60 to 300-minute feed cycle and polymerized in the presence of the first stage latex particles to obtain a polymer latex product.

In an optional step, residual monomer can be removed from the emulsion product by the introduction of a redox couple (oxidant and reductant) to the reactor until the desired amount of residual monomer is reduced.

During the polymerization, thorough mixing of the reaction components should be performed. Thus, the reaction mixture is stirred during the entire duration of the polymerization and of any subsequent post-polymerization. Typically, the polymerization process can be carried out at a reaction temperature ranging from 20 to 150 °C, or from 50 to 125 °C, or from 60 to 90 °C.

The polymerization can be carried out the presence of chain transfer agents. Suitable chain transfer agents include, but are not limited to, thio- and disulfide containing compounds, such as $C_1$-$C_{18}$ alkyl mercaptans, such as tert-butyl mercaptan, n-octyl mercaptan, n-dodecyl mercaptan, tert-dodecyl mercaptan hexadecyl mercaptan, octadecyl mercaptan; mercaptoalcohols, such as 2-mercaptoethanol, 2-mercaptopropanol; mercaptocarboxylic acids, such as mercaptoacetic acid and 3-mercaptopropionic acid; mercaptocarboxylic acid esters, such as butyl thioglycolate, isooctyl thioglycolate, dodecyl thioglycolate, isooctyl 3-mercaptopropionate, and butyl 3-mercaptopropionate; thioesters; $C_1$-$C_{18}$ alkyl disulfides; aryldisulfides; polyfunctional thiols such as trimethylolpropane-tris-(3-mercaptopropionate), pentaerythritol-tetra-(3-mercaptopropionate), pentaerythritol-tetra-(thioglycolate), pentaerythritol-tetra-(thiolactate), dipentaerythritol-hexa-(thioglycolate), and the like; phosphites and hypophosphites; $C_1$-$C_4$ aldehydes, such as formaldehyde, acetaldehyde, propionaldehyde; haloalkyl compounds, such as carbon tetrachloride, bromotrichloromethane, and the like; hydroxylammonium salts such as hydroxylammonium sulfate; formic acid; sodium bisulfite; isopropanol; and catalytic chain transfer agents such as, for example, cobalt complexes (e.g., cobalt (II) chelates).

The chain transfer agents are generally used in amounts ranging from 0.001 to 10 wt.%, based on the total weight of the monomers present in the polymerization medium.

As mentioned previously, the polymerization can be conducted with or without an emulsifying surfactant. To facilitate the emulsification of the monomer mixture, the emulsion polymerization can be carried out in the presence of at least one surfactant. Surfactants for facilitating emulsion polymerizations include anionic, nonionic, amphoteric, and cationic, as well as mixtures thereof. Most commonly, anionic, and nonionic surfactants are utilized as well as mixtures thereof. Typical emulsifiers used in the art are described in detail in the literature, see for example Handbook of Industrial Surfactants, Ash, Michael, Ash, Irene, fifth edition, 2010, Synapse Information Resources, Inc.

Suitable anionic surfactants for facilitating emulsion polymerizations are well-known in the art and include, but are not limited to, sodium lauryl sulfate, sodium dodecyl benzene sulfonate, sodium ($C_6$-$C_{16}$) alkyl phenoxy benzene sulfonate, disodium ($C_6$-$C_{16}$) alkyl phenoxy benzene sulfonate, disodium ($C_6$-$C_{16}$) di-alkyl phenoxy benzene sulfonate, disodium laureth-3 sulfosuccinate, sodium dioctyl sulfosuccinate, sodium di-sec-butyl naphthalene sulfonate, disodium dodecyl diphenyl ether sulfonate, disodium n-octadecyl sulfosuccinate, phosphate esters of branched alcohol ethoxylates, and the like.

Nonionic surfactants suitable for facilitating emulsion polymerizations are well-known in the polymer art, and include, without limitation, linear or branched $C_8$-$C_{30}$ fatty alcohol ethoxylates, such as capryl alcohol ethoxylate, lauryl alcohol ethoxylate, myristyl alcohol ethoxylate, cetyl alcohol ethoxylate, stearyl alcohol ethoxylate, cetearyl alcohol ethoxylate, sterol ethoxylate, oleyl alcohol ethoxylate, and, behenyl alcohol ethoxylate; alkylphenol alkoxylates, such as octylphenol ethoxylates; and polyoxyethylene polyoxypropylene block copolymers, and the like Additional fatty alcohol ethoxylates suitable as non-ionic surfactants are described below. Other useful nonionic surfactants include alpha-olefin sulfonates, e.g., $C_{14}$-$C_{16}$ alpha olefin sulfonates, $C_8$-$C_{22}$ fatty acid esters of polyoxyethylene glycol, ethoxylated mono- and diglycerides, sorbitan esters and ethoxylated sorbitan esters, $C_8$-$C_{22}$ fatty acid glycol esters, block copolymers of ethylene oxide and propylene oxide, and combinations thereof. The number of ethylene oxide units in each of the foregoing ethoxylates can range from 2 and above in one aspect, and from 2 to about 150 in another aspect.

The amount of emulsifying surfactant when employed ranges in the amount from about 0 or about 0.0001 to about 10 wt.% by weight, or from about 0.003 to about 8 wt., or from about 0.01 to about 7 wt.%, or from about 1 to about 3.5 wt.%, based on a total weight of monomers (a), (b), (c) and (d) employed.

Advantageously, when the combination of monomers (a), (b), (c), and (d) are polymerized in the presence of the stabilizer package comprising digested starch, PVOH, and least one acid selected from a saturated $C_2$ to $C_{50}$ mono- or diacid and/or an unsaturated $C_{10}$ to $C_{50}$ mono- or diacid, and mixtures thereof of the present technology, emulsifying surfactants are not necessary in the reaction medium. Accordingly, a polymer emulsion product that is devoid of a surfactant(s), particularly sulfate containing surfactants, is obtained. Thus, the personal care product formulator desiring to formulate a sulfate-free cosmetic product with the rheology modifier of the present technology, no extrinsic sulfates (other than very low levels of the initiator component if a persulfate initiator(s) is employed) are introduced into the end-product by the use thereof. In one aspect, the persulfate initiator component is present in an amount of < 1 part by wt. per 100 parts by wt. of total monomer.

The polymerization can be carried out in a suitable solvent system such as water. Minor amounts of a hydrocarbon solvent, organic solvent, hydroalcoholic solvent as well as mixtures thereof can be employed. The polymerization reactions are initiated by any means which results in the generation of a suitable free-radical. Thermally derived radicals, in which the radical species is generated from thermal, homolytic dissociation of peroxides, hydroperoxides, persulfates, percarbonates, peroxyesters, hydrogen peroxide and azo compounds can be utilized. The initiators can be water soluble or water insoluble depending on the solvent system employed for the polymerization reaction.

Exemplary free radical water soluble initiators include, but are not limited to, inorganic persulfate compounds, such as ammonium persulfate, potassium persulfate, and sodium persulfate; peroxides such as hydrogen peroxide, benzoyl peroxide, acetyl peroxide, and lauryl peroxide; organic hydroperoxides, such as cumene hydroperoxide and t-butyl hydroperoxide; organic peracids, such as peracetic acid, and water soluble azo compounds, such as 2,2'-azobis(tert-alkyl) compounds having a water solubilizing substituent on the alkyl group. Exemplary free radical oil soluble compounds include, but are not limited to 2,2'-azobisisobutyronitrile, and the like. The peroxides and peracids can optionally be activated with reducing agents, such as sodium bisulfite, sodium formaldehyde sulfate (SFS), or ascorbic acid, transition metals, hydrazine, and the like.

In one aspect, azo polymerization initiators include the Vazo™ free-radical polymerization initiators, available from DuPont, such as Vazo™ 44 (2,2'-azobis(2-(4,5-dihydroimidazolyl)propane), Vazo™ 56 (2,2'-azobis(2-methylpropionamidine) dihydrochloride), Vazo™ 67 (2,2'-azobis(2-methylbutyronitrile)), Vazo™ 68 (4,4'-azobis(4-cyanovaleric acid)), and VA-086 2,2'-Azobis([2-methyl-N-(2-hydroxyethyl)propionamide) from Fujifilm Wako Pure Chemical Corporation.

The initiator compounds can be utilized in an amount of up to 30 wt.%, or from 0.01 to 10 wt. %, or from 0.2 to 3 wt.%, based on the total weight of the dry polymer.

Optionally, the use of known redox initiator systems as polymerization initiators can be employed. Such redox initiator systems include an oxidant (initiator) and a reductant. Suitable oxidants include, for example, hydrogen peroxide, sodium peroxide, potassium peroxide, t-butyl hydroperoxide, t-amyl hydroperoxide, cumene hydroperoxide, sodium perborate, perphosphoric acid and salts thereof, potassium permanganate, and ammonium or alkali metal salts of peroxydisulfuric acid, typically at a level of from 0.01 to 3.0 wt. %, based on dry polymer weight, are used. Suitable reductants include, for example, alkali metal and ammonium salts of sulfur-containing acids, such as sodium sulfite, bisulfite, thiosulfate, hydrosulfite, sulfide, hydrosulfide or dithionite, formadinesulfinic acid, hydroxymethanesulfonic acid, acetone bisulfite, amines such as ethanolamine, glycolic acid, glyoxylic acid hydrate, ascorbic acid, isoascorbic acid, lactic acid, glyceric acid, malic acid, 2-hydroxy-2-sulfinatoacetic acid, tartaric acid and salts of the preceding acids typically at a level of 0.01 to 3.0 wt.%, based on dry polymer weight, is used. In one aspect, combinations of peroxodisulfates with alkali metal or ammonium bisulfites can be used, for example, ammonium peroxodisulfate and ammonium bisulfite. In another aspect, combinations of hydrogen peroxide containing compounds (t-butyl hydroperoxide) as the oxidant with ascorbic or erythorbic acid as the reductant can be utilized. The weight ratio of peroxide-containing compound to reductant ranges from 30:1 to 0.05:1.

The polymer emulsion product can be prepared to contain from
5 percent to 60 percent total polymer solids (TS), or from about 10 percent to 50 percent total polymer solids, or from 15 percent to 45 percent total polymer solids, or from 17 to 35 percent total polymer solids, or from 18 to 30 percent total polymer solids, based on the weight of the emulsion.

In one aspect, the polymer emulsions can be diluted with water and/or solvent(s) (e.g., a hydroalcoholic solvent system) or concentrated by evaporating a portion of the water. Alternatively, the obtained polymer emulsion can be substantially dried to a powder or crystalline form by utilizing equipment well known in the art, such as, for example, a spray drier, a drum drier, a freeze drier, and the like.

In one aspect, the polymer product is a random copolymer and has number average molecular weight as measured by gel permeation chromatography (GPC) calibrated with a poly(methyl methacrylate) (PMMA) ranging from above 500,000 to at least a billion Da or more in one aspect, from 600,000 to 4.5 billion Da in another aspect, and from 1,000,000 to 3,000,000 Da in a further aspect, and from 1,500,000 to 2,000,000 Das in a still further aspect (see TDS-222, October 15, 2007. Lubrizol Advanced Materials, Inc. ).

In one aspect, it has been discovered that the post-reaction addition of an acid and/or reducing agent to the emulsion polymer latex product further stabilizes the polymer latex particles against coalescence and/or flocculation, thus mitigating increases in the particle size of the latex. Due to coalescence and/or flocculation, larger latex particle sizes lead to increases in viscosity and deterioration of latex stability resulting in product that is difficult to handle and process. The acid and/or reducing agent can be dosed into the emulsion polymer latex in the reactor following the completion of the polymerization reaction. Alternatively, the acid and/or reducing agent can be dosed into the emulsion polymer latex after the latex is removed from the reactor. The acid and/or reducing agent is dosed into the emulsion polymer latex with adequate stirring to ensure that the acid and/or reducing agent is homogeneously blended into the latex.

Suitable acids for mitigating the coalescence and/or flocculation of the emulsion polymer latex particles of the disclosed technology include organic and/or inorganic acids. Exemplary organic acids are selected from, but are not

limited to, acetic acid, alpha-hydroxy acids (e.g., citric acid, glycolic acid, lactic acid, mandelic acid, hydroxycaproic acid, hydroxycaprylic acid, malic acid, tartaric acid, α-hydroxypropionic acid), beta-hydroxy acids (e.g., salicylic acid, tropic acid, trethocanic acid, β-hydroxypropionic acid, β-hydroxybutyric acid, β-hydroxy β-methylbutyric acid, carnitine), natural fruit acids, caproic acid, caprylic acid, pelargonic acid, capric acid, undecanoic acid, lauric acid, myristic acid, palmitic acid, palmitoleic acid, stearic acid, isostearic acid, hydroxy stearic acid, oleic acid, ricinoleic acid, vaccenic acid, linolenic acid, α-linolenic acid, γ-linolenic acid, arachidic acid, gadoleic acid, arachidonic acid, eicosapentaenoic acid, behenic acid, docosahexaenoic acid, lignoceric acid, malonic acid, succinic acid, glutaric acid, adipic acid, pimelic acid, suberic acid, azelaic acid, undecanedioic acid, dodecandioic acid, brassylic acid, thapsic acid japanic acid, phellogenic acid, equisetolic acid, isocitric acid, aconitic acid, propane-1,2,3-tricarboxylic acid, agaric acid, trimesic acid, tartronic acid, mesoxalic acid, oxaloacetic acid aspartic acid, dioxosuccinic acid, sulfinic acid derivatives (e.g., formamidine sulfinic acid, sodium salt of an organic sulfinic acid derivative marketed as Bruggolite™ FF6 M); hydroxymethanesulfonic acid; erythorbic acid; ascorbic acid; isoascorbic acid; glyceric acid; 2-hydroxy-2-sulfinatoacetic acid; and combinations thereof.

Exemplary inorganic acids are selected from, but are not limited to, hydrochloric acid, nitric acid, sulfuric acid, sulfamic acid, phosphoric acid, and combinations thereof.

Exemplary reducing agents are selected from, but are not limited to, sodium formaldehyde sulfate; transition metals, hydrazine; alkali metal and ammonium salts of sulfur-containing compounds such as sodium sulfite, sodium bisulfite, sodium thiosulfate, sodium hydrosulfite, sodium sulfide, sodium hydrosulfide, and sodium dithionite; acetone bisulfite; ethanolamine; monosaccharides, disaccharides, oligosaccharides, and oxidized derivatives thereof; and mixtures thereof.

In one aspect, the post-added acids and/or reducing agents are dosed into the latex in an amount ranging from 1.25 to 10 wt.%, or from 2.5 to 7.5 wt.%, or from 3.5 to 5 wt.%, based on the total weight of the emulsion polymer latex. When employing a combination of acid and reducing agent, the weight ratio of acid to reducing agent ranges from 10:1 to 1:10.

The ASE and HASE polymers of the disclosed technology can be utilized, for example, without being limited thereto, as a rheology modifier, suspending agent, film former, thickener, stabilizer, emulsifier, solubilizer, and the like, in formulated compositions for personal care products, topical health care products, household care products, institutional and industrial (I&I) products and industrial processes. The foregoing products can typically contain various additives and conventional adjuvants as are well known in the art, including, without being limited thereto, acidifying or alkalizing pH adjusting agents and buffering agents; fixatives and auxiliary film formers, such as gums, resins, polymers of synthetic or natural origin, and the like; auxiliary rheology modifiers, such as viscosity increasing polymeric thickeners or gellants, additives, such as emulsifiers, emulsion stabilizers, waxes, dispersants, and the like, and viscosity control agents, such as solvents, electrolytes, and the like; hair and skin conditioning agents, such as antistatic agents, synthetic oils, vegetable or animal oils, silicone oils, monomeric or polymeric quaternized ammonium salts, emollients, humectants, lubricants, sunscreen agents, disinfectants, antimicrobials, and the like; chemical hair waving or straightening agents; hair colorants, such as pigments and dyes for temporary, semipermanent, or permanent hair dyeing; surfactants, such as anionic, cationic, nonionic, amphoteric and zwitterionic surfactants; polymer film modifying agents, such as plasticizers, humectants, tackifiers, detackifiers, wetting agents and the like, product finishing agents, such as chelating agents, opacifiers, pearlescing agents, preservatives, fragrances, solubilizers, colorants, such as pigments and dyes, UV absorbers, and the like; propellants (water-miscible or water-immiscible), such as fluorinated hydrocarbons, liquid volatile hydrocarbons, compressed gases, and the like; and mixtures thereof.

In one aspect, the ASE and HASE polymers of the disclosed technology can be utilized to suspend particulate materials and insoluble droplets within an aqueous composition. Such fluids are useful in the oil and gas, personal care, homecare, coatings and inks and adhesive/binder industries.

The stable compositions maintain a smooth, acceptable rheology with good shear thinning properties without significant increases or decreases in viscosity, with no phase separation, e.g., settling or creaming out (rising to the surface), or loss of clarity over extended periods of time, such as for at least one month at 45°C.

In the coatings, inks, and adhesive/binder industries the ASE and HASE polymers of the disclosed technology are useful to adjust the viscosity of a liquid composition to: a) control or minimize settling or creaming of solid particles, dispersed liquids, trapped gases, and particulates (aid in suspension) that are more dense or less dense than the continuous media (often water based); b) to control application viscosity of continuous or discontinuous layers of a coating, ink, or adhesive to a substrate; c) to minimize movement or flow of coatings, inks, or adhesives immediately prior to application or in the time after application until the coating, ink, or adhesive forms a continuous gelled polymer; e) reduce splatter and misting in some application processes; f) etc., to facilitate optimal storage, application ease, and final surface finish in those applications. The coatings, inks and adhesives may comprise particulate or fibrous fillers, pigments, dyes, other polymers, surfactants and/or dispersants, coalescing aids, plasticizers, biocides and other conventional additives employed in coatings, inks, and adhesives. The coatings can be used on metals, plastics, wood, masonry, textiles, papers, etc. The inks can be used on any ink substrates such as paper, polymers, wovens, nonwovens, films, etc. The HASE polymer can contribute to both viscosity control and optical clarity (helping color intensity of pigmented compositions) of the coating, ink, or adhesive.

In the personal care, topical health care, and homecare industries the ASE and HASE polymers of the disclosed technology can be utilized as rheology modifiers to thicken aqueous and surfactant containing compositions as well as to improve the yield stress (stable suspension of insoluble and particulate materials) properties of surfactant containing compositions, hair and skin care compositions, and cosmetics. The ASE and HASE polymers can be utilized to suspend insoluble silicones, opacifiers and pearlescent agents (e.g., mica, coated mica), pigments, exfoliants, anti-dandruff agents, clay, swellable clay, laponite, gas bubbles (aesthetic air bubbles), liposomes, microsponges, cosmetic beads, fragrance microcapsules, fragrance particles, benefit agent containing microcapsules and particles, cosmetic microcapsules, and flakes. The ASE and HASE polymers of the disclosed technology can stabilize these materials in suspension for at least one month at 45 °C in one aspect, at least 6 months in another aspect, and at least one year in a further aspect.

Compositions for personal care and topical health care can comprise any cosmetic, toiletry, and topical pharmaceutical formulation that requires rheology modification, thickening, and film formation known from the cosmetic and pharmaceutical literature. Typical personal care formulations that can include the ASE and HASE polymers as a rheology modifier include, without being limited thereto, shampoos, chemical and non-chemical hair curling and hair straightening products, hair style maintenance products (e.g., hair fixatives and hair color products), emulsion lotions and creams for the nails, hands, feet, face, scalp, and body, hair dyes, face and body makeup, nail care products, astringents, deodorants, antiperspirants, depilatories, skin-protective creams and lotions, such as sunscreens, skin and body cleansers, skin conditioners, skin toners, skin firming compositions, liquid soaps, soap bars, bath products, shaving products, and the like.

Formulated compositions for topical health care that are applied to the skin and mucous membranes for cleansing or soothing are compounded with many of the same physiologically tolerable cosmetic ingredients and chemically inert ingredients employed for personal care products in the same product forms, differing primarily in the purity grade of ingredients and by the presence of topically active medicaments. For example, topical health care products include oral hygiene products, such as toothpastes, oral suspensions, and mouth care products, which can be classified as pharmaceuticals or over-the-counter products and include pharmacosmetics which contain phytopharmaceutic or nutraceutical ingredients.

Compositions for personal care and topical health care can be in the form of, without being limited thereto, liquids, such as rinses, gels, hydroalcoholic gels (e.g., hand sanitizers), sprays, emulsions, such as lotions and creams, shampoos, pomades, foams, ointments, tablets, sticks, such as lip care products, makeup, and suppositories, and like products, which are applied to skin and hair and remain in contact therewith until removed as by rinsing with water or washing with shampoo or soap. Gels can be soft, stiff, or squeezable. Emulsions can be oil-in-water, water-in-oil, or multiphase. Sprays can be non-pressurized aerosols delivered from manually pumped finger-actuated sprayers or can be pressurized aerosols. The ASE and HASE polymers can be formulated in an aerosol composition, such as in a spray, mousse, or foam forming formulation, where a chemical or gaseous propellant is required. Physiologically and environmentally tolerable propellants, such as compressed gases, fluorinated hydrocarbons and liquid volatile hydrocarbons, and the amounts and suitable combinations to be used, are well known in the cosmetic and pharmaceutical art and literature.

An extensive listing of personal care and cosmetic ingredients and their functions, for example, appears in the INCI Dictionary, generally, and in Vol. 2, Section 4 of the Seventh Edition, in particular, incorporated herein by reference. Those skilled in the art of formulating personal care and health care products recognize that some ingredients are multifunctional and, hence, can serve more than one purpose in the formulation. Thus, the amount of ASE and HASE polymers employed as a personal care or health care product component is not limited, as long as the purpose and properties of the formulated composition performs its intended function.

Typical household care, and I&I care products that can contain the ASE and HASE polymers of the disclosed technology as a rheology modifier include, without being limited thereto, surface cleansers for kitchen and bathroom counter tops, tiled surfaces, and utilities, including appliances employed or located therein, toilet cleaners, including toilet bowl rim gels, floor cleansers, wall cleansers, polishes, air freshener gels, detergents, treatments and cleansers for dishes and laundry, such as fabric softener, spot reducer, fabric treatments, and the like.

The amount of the ASE and HASE polymer that can be employed in the foregoing compositions can be determined by person skilled in the formulation art. Thus, as long as the physicochemical and functional properties of a desired product are achieved, a useful amount of polymer on a total composition weight basis, typically can vary in the range of from 0.01 to 25 weight percent, from 0.1 to 15 weight percent, or from 0.5 to 10 weight percent, or from 1 to 5 weight percent, based on the weight of the total composition (all polymer weights based on 100 percent active polymer solids).

In one aspect, the disclosed technology concerns a personal care composition comprising water, one or more surfactants and an ASE or HASE polymer according to the disclosed technology. In one aspect of the present technology, the disclosed ASE or HASE polymers can be formulated with surfactants to provide thickened surfactant containing compositions. The surfactant can be selected from at least one anionic surfactant, at least one cationic surfactant, at least one amphoteric or zwitterionic surfactant, at least one nonionic surfactant, and mixtures thereof.

In one aspect, the disclosed technology relates to a personal care composition comprising water, one or more surfactants and at least one ASE or HASE polymer according to the disclosed technology, wherein the one or more surfactants are present over a wide concentration ranging from about 3 to 25 wt. %, or from 5 to 20 wt. %, or from 8 to 16 wt.%, based on the weight of the total composition, and wherein the at least one ASE or HASE polymer is present from 1 to 10 wt.%, or from 1.5 to 5 wt.%, or from 1.75 to 3 wt.%, based on the total weight of the composition, and wherein such compositions have an ideal viscosity ranging from 1,000 to 35,000 mPa·s, or from 3,000 to 25,000 mPa·s, or from 5,000 to 20,000 mPa·s, or from 8,000 to 15,000 mPa·s (as measured on a Brookfield rotating spindle viscometer, Model RVT, at about 20 rpm, at ambient room temperature ranging between 20 to 25 °C), and wherein such compositions have a turbidity value that is equal to or less than 60 Nephelometric Turbidity Units (NTU), or less than 50 NTU, or equal to or less than 40 NTU, or equal to or less than 30 NTU, or equal to or less than 20 NTU as measured by the Turbidity Test described in the test protocol hereinbelow. Lower NTU values (less than 20 NTUs) are attainable in sulfate-free surfactant systems.

In one aspect, the disclosed technology concerns a personal care composition comprising water, one or more sulfate-free anionic surfactants and an ASE or HASE polymer according to the disclosed technology. Generally speaking, it is difficult to obtain high clarity, yield stress (the ability to suspend insoluble and particulate materials) and build and maintain viscosity when thickening compositions containing sulfate-free surfactant systems. The polymers of the disclosed technology are able to thicken formulations containing sulfate-free surfactants while providing good clarity, yield stress, viscosity, and the stable suspension of insoluble and particulate materials within such formulations.

pH Adjusting Materials

The ASE and HASE polymers and polymer compositions containing same according to the present technology are pH-responsive. At the lower pH levels at which the emulsion polymerization takes place, i.e., pH levels of 5 or less, the composition is relatively thin or non-viscous. When the pH of the polymer dispersion or aqueous compositions containing the polymer are neutralized or adjusted by addition of an alkaline material (base) the composition thickens substantially. Viscosity increases as the polymer dissolves partially or completely in the aqueous phase of the composition. Neutralization can occur *in situ* when the emulsion polymer is blended with the base and added to the aqueous phase. Alternatively, if desired for a given application, neutralization can be carried out at any step in the formulation of the desired end-product. Compositions including the ASE and HASE polymers of the present technology have a desired pH range of 4 to 12, or from 6 to 7.5, or from 6.5 to 7.

Many types of alkaline materials can be used to neutralize the polymer, including inorganic and organic bases, and combinations thereof. Examples of inorganic bases include but are not limited to the alkali metal hydroxides (especially sodium, potassium, and ammonium), and alkali metal salts of inorganic acids, such as sodium borate (borax), sodium phosphate, sodium pyrophosphate, and the like, and mixtures thereof. Examples of organic bases include, but are not limited to, triethanolamine (TEA), diisopropanolamine, triisopropanolamine, aminomethyl propanol, dodecylamine, cocamine, oleamine, morpholine, triamylamine, triethylamine, tetrakis(hydroxypropyl)ethylenediamine, L-arginine, aminomethyl propanol, tromethamine (2-amino 2-hydroxymethyl-1,3-propanediol), and PEG-15 cocamine. Alternatively, other alkaline materials can be used alone or in combination with the above-mentioned inorganic and organic bases. Such materials include surfactants, surfactant mixtures, pre-neutralized surfactants, or materials that when combined in a composition containing a polymer of the technology is capable of neutralizing or partially neutralizing the acid groups on the polymer backbone. Any material capable of increasing the pH of the composition is suitable.

In one aspect, the base-neutralized compositions of the disclosed technology can be back-acid treated with an acidic material. The back-acid thickening technique can be used to further increase the viscosity and stability of compositions formulated in the alkaline pH range by actually reducing the pH of the composition. In one aspect of the present technology, the final target pH (subsequent to back-acid titration) should be at least about 0.5 to 8 pH units below that of the initial pH of the base-neutralized system, subject to the proviso that the final pH range is within the base-neutralized range of 4 to 12. In another aspect, the target pH of the back-acid treated system is at least 0.75 to 7 pH units below the initial base-neutralized pH, and in still another aspect, the target pH of the back-acid treated system is at least 1 to 6 pH units below the initial base-neutralized pH of the system.

In one aspect, the surfactant composition (sulfate containing or sulfate-free) comprising the polymers prepared in accordance with the present technology can be base neutralized to a pH value ranging from 6 to 7 followed by subsequent back-acid titration to a pH value ranging from 5 to 5.5.

Suitable acidifying materials include organic acids such as citric acid, acetic acid, alpha-hydroxy acid, beta-hydroxy acid, salicylic acid, lactic acid, glycolic acid, natural fruit acids, or combinations thereof. In addition, inorganic acids, for example hydrochloric acid, nitric acid, sulfuric acid, sulfamic acid, phosphoric acid, and combinations thereof can be utilized to reduce the pH value of the composition.

Buffer Agents

Buffering agents can be used in the exemplary compositions. Suitable buffering agents include alkali or alkali earth metal carbonates, phosphates, bicarbonates, citrates, borates, acetates, acid anhydrides, succinates, and the like, such as sodium phosphate, sodium citrate, sodium acetate, sodium bicarbonate, and sodium carbonate.

The amount of buffering agent utilized is dependent upon the desired pH range to be buffered or maintained. Such amounts can readily be determined by the skilled formulator.

Chelating Agents

Chelating agents can be employed to stabilize the composition against the deleterious effects of metal ions. When utilized, suitable chelating agents include EDTA (ethylene diamine tetraacetic acid) and salts thereof such as disodium and tetrasodium EDTA, citric acid and salts thereof, cyclodextrins, and the like, and mixtures thereof.

Such suitable chelating agents can comprise 0.001 wt.% to 3 wt. %, such as 0.01 wt.% to 2 wt.%, or 0.01 wt.% to 1 wt.% of the total weight of the hair straightening composition.

Surfactants

Suitable anionic surfactants include but are not limited to alkyl sulfates, alkyl ether sulfates, alkyl sulfonates, alkaryl sulfonates, $\alpha$-olefin-sulfonates, alkylamide sulfonates, alkarylpolyether sulphates, alkylamidoether sulfates, alkyl monoglyceryl ether sulfates, alkyl monoglyceride sulfates, alkyl monoglyceride sulfonates, alkyl succinates, alkyl sulfosuccinates, alkyl ether sulfosuccinates, alkyl sulfosuccinamates, alkyl amidosulfosuccinates; alkyl sulfoacetates, alkyl phosphates, alkyl ether phosphates, alkyl ether carboxylates, alkyl amidoethercarboxylates, acyl lactylates, alkyl isethionates, acyl isethionates, carboxylate salts, fatty acid soaps, and amino acid derived surfactants such as N-alkyl amino acids, N-acyl amino acids, as well as alkyl peptides.

In one aspect, the cation moiety of the forgoing surfactants is selected from sodium, potassium, magnesium, ammonium, and alkanolammonium ions such as monoethanolammonium, diethanolammonium triethanolammonium ions, as well as monoisopropylammonium, diisopropylammonium and triisopropylammonium ions. In one embodiment, the alkyl and acyl groups of the foregoing surfactants contain from 6 to 24 carbon atoms in one aspect, from 8 to 22 carbon atoms in another aspect and from 12 to 18 carbon atoms in a further aspect and may be unsaturated. The aryl groups in the surfactants are selected from phenyl or benzyl. The ether containing surfactants set forth above can contain from 1 to 10 ethylene oxide and/or propylene oxide units per surfactant molecule in one aspect, and from 1 to 3 ethylene oxide units per surfactant molecule in another aspect.

Examples of anionic surfactants include the sodium, potassium, lithium, magnesium, and ammonium salts of laureth sulfate, trideceth sulfate, myreth sulfate, $C_{12}$-$C_{13}$ pareth sulfate, $C_{12}$-$C_{14}$ pareth sulfate, and $C_{12}$-$C_{15}$ pareth sulfate, ethoxylated with 1, 2, and 3 moles of ethylene oxide; the sodium potassium, lithium, magnesium, ammonium, and triethanolammonium salts of lauryl sulfate, coco sulfate, tridecyl sulfate, myristyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium lauroyl isethionate, sodium lauroyl methyl isethionate, sodium $C_{12}$-$C_{14}$ olefin sulfonate, sodium laureth-6 carboxylate, sodium dodecylbenzene sulfonate, triethanolamine monolauryl phosphate, and fatty acid salts (soaps), including the sodium, potassium, ammonium, and triethanolamine salts of a saturated and unsaturated fatty acids containing from 8 to 22 carbon atoms.

In one aspect, the amino acid surfactants are selected from a N-acyl amino acid of the formula:

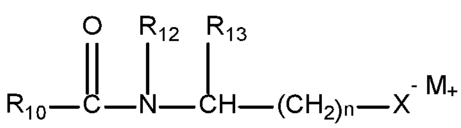

wherein $R_{10}$ is a saturated or unsaturated, straight, or branched alkyl chain containing 7 to 17 carbon atoms, $R_{12}$ is H or a methyl group, $R_{13}$ is H, $COO^-M^+$, $CH_2COO^-M^+$ or COOH, n is 0 to 2, X is $COO^-$ or $SO_3^-$ and M independently represents H, sodium, potassium, ammonium or triethanolammonium.

In one aspect, the N-acyl amino acid surfactants represented by the formula immediately above are derived from taurates, glutamates, alanine, alaninates, sacosinates, aspartates, glycinates, and mixtures thereof.

Representative taurate surfactants conform to the formula:

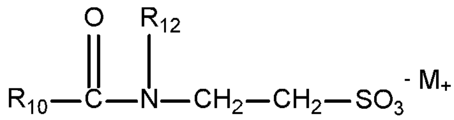

wherein $R_{10}$ is a saturated or unsaturated, straight or branched alkyl chain containing 7 to 17 carbon atoms in one aspect and 9 to 13 carbon atoms in another aspect, $R_{12}$ is H or methyl, and M is H, sodium, potassium, ammonium or triethanolammonium.

Non-limiting examples of taurate surfactants are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, sodium methyl stearoyl taurate, and mixtures thereof.

Representative glutamate surfactants conform to the formula:

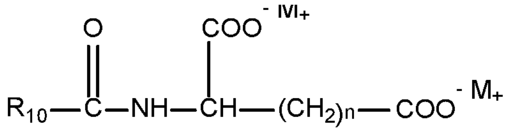

wherein $R_{10}$ is a saturated or unsaturated, straight or branched alkyl chain containing 7 to 17 carbon atoms in one aspect and 9 to 13 carbon atoms in another aspect, n is 0 to 2, and M independently is H, sodium, potassium, ammonium or triethanolammonium.

Non-limiting examples of glutamate surfactants are di-potassium capryloyl glutamate, di-potassium undecylenoyl glutamate, di-sodium capryloyl glutamate, di-sodium cocoyl glutamate, di-sodium lauroyl glutamate, di-sodium stearoyl glutamate, di-sodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, sodium undecylenoyl glutamate, and mixtures thereof.

Representative alanine and alaninate surfactants conform to the formula:

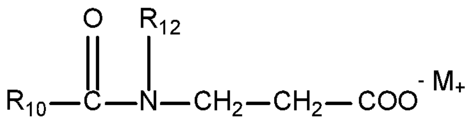

wherein $R_{10}$ is a saturated or unsaturated, straight or branched alkyl chain containing 7 to 17 carbon atoms in one aspect and 9 to 13 carbon atoms in another aspect, $R_{12}$ is H or methyl, and M is H, sodium, potassium, ammonium or triethanolammonium.

Non-limiting examples of alanine and alaninate surfactants are cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine, sodium myristoyl methyl β-alanine, and mixtures thereof.

Representative glycinate surfactants conform to the formula:

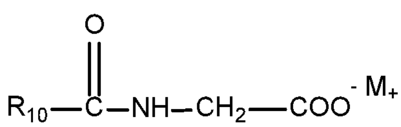

wherein $R_{10}$ is a saturated or unsaturated, straight or branched alkyl chain containing 7 to 17 carbon atoms in one aspect and 9 to 13 carbon atoms in another aspect, and M is H, sodium, potassium, ammonium or triethanolammonium.

Non-limiting examples of glycinate surfactants are sodium palmitoyl glycinate, sodium lauroyl glycinate, sodium cocoyl glycinate, sodium myristoyl glycinate, potassium lauroyl glycinate, potassium cocoyl glycinate, sodium stearoyl glycinate, and mixtures thereof.

Representative sarcosinate surfactants conform to the formula:

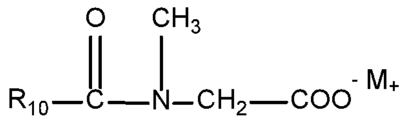

wherein $R_{10}$ is a saturated or unsaturated, straight, or branched alkyl chain containing 7 to 17 carbon atoms in one aspect and 9 to 13 carbon atoms in another aspect, and M is H, sodium, potassium, ammonium or triethanolamine.

Non-limiting examples of sarcosinate surfactants are potassium lauroyl sarcosinate, potassium cocoyl sarcosinate, sodium cocoyl sarcosinate, sodium lauroyl sarcosinate, sodium myristoyl sarcosinate, sodium palmitoyl sarcosinate, and mixtures thereof.

Representative aspartate surfactants conform to the formula:

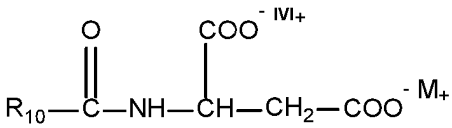

wherein $R_{10}$ is a saturated or unsaturated, straight or branched alkyl chain containing 7 to 17 carbon atoms in one aspect and 9 to 13 carbon atoms in another aspect, and M independently is H, sodium, potassium, ammonium or triethanolammonium.

Non-limiting examples of aspartate surfactants are sodium lauroyl aspartate, sodium myristoyl aspartate, sodium cocoyl aspartate, sodium caproyl aspartate, di-sodium lauroyl aspartate, di-sodium myristoyl aspartate, di-sodium cocoyl aspartate, di-sodium caproyl aspartate, potassium lauroyl aspartate, potassium myristoyl aspartate, potassium cocoyl aspartate, potassium caproyl aspartate, di-potassium lauroyl aspartate, di-potassium myristoyl aspartate, di-potassium cocoyl aspartate, di-potassium caproyl aspartate, and mixtures thereof.

In one aspect of the disclosed technology, the anionic surfactant can be selected from a fatty acid soap(s) containing from 8 to 22 carbon atoms, or from 10 to 20 carbon atoms, or from 12 to 18 carbon atoms, or from 12 to 16 carbon atoms, and mixtures thereof.

Exemplary saturated fatty acids include, but are not limited to, the sodium, potassium, and ammonium salts of lauric acid, myristic acid, palmitic acid, steric acid, and mixtures thereof.

In one aspect of the present technology, suitable cationic surfactants include but are not limited to alkylamines, amidoamines, alkyl imidazolines, ethoxylated amines, quaternary compounds, and quaternized esters. In addition, alkylamine oxides can function as a cationic surfactant at a lower pH value.

Non-limiting examples of alkylamines and salts thereof include dimethyl cocamine, dimethyl palmitamine, dioctylamine, dimethyl stearamine, dimethyl soyamine, soyamine, myristyl amine, tridecyl amine, ethyl stearylamine, N-tallowpropane diamine, ethoxylated stearylamine, dihydroxy ethyl stearylamine, arachidylbehenylamine, dimethyl lauramine, stearylamine hydrochloride, soyamine chloride, stearylamine formate, N-tallowpropane diamine dichloride, and amodimethicone (INCI name for a silicone polymer endblocked with amino functional groups, such as aminoethylamino propylsiloxane).

Non-limiting examples of alkyl imidazoline surfactants include alkyl hydroxyethyl imidazoline, such as stearyl hydroxyethyl imidazoline, coco hydroxyethyl imidazoline, ethyl hydroxymethyl oleyl oxazoline, and the like.

Non-limiting examples of ethyoxylated amines include PEG-cocopolyamine, PEG-15 tallow amine, quaternium-52, and the like.

Exemplary quaternary ammonium surfactants include, but are not limited to cetyl trimethylammonium chloride, cetylpyridinium chloride, dicetyl dimethyl ammonium chloride, dihexadecyl dimethyl ammonium chloride, stearyl dimethyl benzyl ammonium chloride, dioctadecyl dimethyl ammonium chloride, dieicosyl dimethyl ammonium chloride, didocosyl dimethyl ammonium chloride, dihexadecyl dimethyl ammonium chloride, dihexadecyl dimethyl ammonium acetate, behenyl trimethyl ammonium chloride, benzalkonium chloride, benzethonium chloride, and di(cocoalkyl) dimethyl ammonium chloride, ditallowdimethyl ammonium chloride, di(hydrogenated tallow) dimethyl ammonium chloride, di(hydrogenated tallow) dimethyl ammonium acetate, ditallowdimethyl ammonium methyl sulfate, ditallow dipropyl ammonium phosphate, and ditallow dimethyl ammonium nitrate.

In one aspect of the present technology, suitable zwitterionic or amphoteric surfactants include but are not limited to alkyl betaines, e.g., lauryl betaine; alkylamido betaines, e.g., cocamidopropyl betaine and cocohexadecyl dimethylbetaine; alkylamido sultaines, e.g., cocamidopropyl hydroxysultaine; (mono- and di-) amphocarboxylates, e.g., sodium cocoamphoacetate, sodium lauroamphoacetate, sodium capryloamphoacetate, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, and disodium capryloamphodipropionate; amine oxides, e.g., dimethyldodecylamine oxide, oleyldi(2-hydroxyethyl) amine oxide, dimethyltetradecylamine oxide, di(2- hydroxyethyl)-tetradecylamine oxide, dimethylhexadecylamine oxide, behenamine oxide, cocamine oxide, decyltetradecylamine oxide, dihydroxyethyl $C_{12-15}$ alkoxypropylamine oxide, dihydroxyethyl cocamine oxide, dihydroxyethyl lauramine oxide, dihydroxyethyl stearamine oxide, dihydroxyethyl tallowamine oxide, hydrogenated palm kernel amine oxide, hydrogenated tallowamine oxide, hydroxyethyl hydroxypropyl $C_{12}$-$C_{15}$ alkoxypropylamine oxide, lauramine oxide, myristamine oxide, cetylamine oxide, oleamidopropylamine oxide, oleamine oxide, palmitamine oxide, PEG-3 lauramine oxide, dimethyl lauramine oxide, potassium trisphosphonomethylamine oxide, soyamidopropylamine oxide, cocamidopropylamine oxide, stearamine oxide, tallowamine oxide, and mixtures thereof, and mixtures thereof.

The foregoing zwitterionic or amphoteric surfactants (i.e., the betaines and sultaines) are disclosed without a counter ion, as one of ordinary skill in the art will recognize that the under the pH conditions of the compositions containing the amphoteric surfactants, these surfactants are either electrically neutral by virtue of having balancing positive and negative charges, or they contain counter ions such as alkali metal, alkaline earth or ammonium ions as a charge balancing moiety.

The nonionic surfactant can be any of the nonionic surfactants known or previously used in the art of aqueous surfactant compositions. Suitable nonionic surfactants include, but are not limited to, aliphatic ($C_6$-$C_{18}$) primary or secondary linear or branched chain acids, alcohols or phenols; alkyl ethoxylates; alkyl phenol alkoxylates (especially ethoxylates and mixed ethoxy/propoxy moieties); block alkylene oxide condensates of alkyl phenols; alkylene oxide condensates of alkanols; and ethylene oxide/propylene oxide block copolymers. Other suitable nonionic surfactants include mono- or dialkyl alkanolamides; alkyl polyglucosides (APGs); sorbitan fatty acid esters; polyoxyethylene sorbitan fatty acid esters; polyoxyethylene sorbitol esters; polyoxyethylene acids, and polyoxyethylene alcohols. Other examples of suitable nonionic surfactants include coco mono- or diethanolamide, coco glucoside, decyl diglucoside, lauryl diglucoside, coco diglucoside, polysorbate 20, 40, 60, and 80, ethoxylated linear alcohols, cetearyl alcohol, lanolin alcohol, stearic acid, glyceryl stearate, PEG-100 stearate, laureth 7, and oleth 20.

In another embodiment, non-ionic surfactants include, but are not limited to, alkoxylated methyl glucosides such as, for example, methyl gluceth-10, methyl gluceth-20, PPG-10 methyl glucose ether, and PPG-20 methyl glucose ether, available from Lubrizol Advanced Materials, Inc., under the trade names, Glucam® E10, Glucam® E20, Glucam® P10, and Glucam® P20, respectively; and hydrophobically modified alkoxylated methyl glucosides, such as PEG 120 methyl glucose dioleate, PEG-120 methyl glucose trioleate, and PEG-20 methyl glucose sesquistearate, available from Lubrizol Advanced Materials, Inc., under the trade names, Glucamate® DOE-120, Glucamate™ LT, and Glucamate™ SSE-20, respectively, are also suitable. Other exemplary hydrophobically modified alkoxylated methyl glucosides are disclosed in United States Patent Nos. 6,573,375 and 6,727,357

Other surfactants which can be utilized in the present technology are set forth in more detail in WO 99/21530, U.S. Patent No. 3,929,678, U.S. Patent No. 4,565,647, U.S. Patent No. 5,720,964, and U.S. Patent No. 5,858,948. In addition, suitable surfactants are also described in McCutcheon's Emulsifiers and Detergents (North American and International Editions, by Schwartz, Perry and Berch) .

Other surfactants which can be utilized in the method of the present technology are set forth in more detail in WO 99/21530, U.S. Patent No. 3,929,678, U.S. Patent No. 4,565,647, U.S. Patent No. 5,456,849, U.S. Patent No. 5,720,964, U.S. Patent No. 5,858,948, and U.S. Patent No. 7,115,550 . Additionally, suitable surfactants are described in McCutcheon's Emulsifiers and Detergents (North American and International Editions, by Schwartz, Perry and Berch)

Cationic Polymers

Cationic polymers are components that can enhance the delivery and deposition of conditioning agents and/or provide auxiliary conditioning benefits to the hair, scalp, or skin to improve and enhance the conditioning benefits delivered by the compositions of the present technology. Cationic polymer refers to polymers containing at least one cationic moiety or at least one moiety that can be ionized to form a cationic moiety. Typically, these cationic moieties are nitrogen containing groups such as quaternary ammonium or protonated amino groups. The cationic protonated amines can be primary, secondary, or tertiary amines. The cationic polymer typically has a cationic charge density ranging from 0.2 to 7 meq/g at the pH of the intended use of the composition. The average molecular weight of the cationic polymer ranges from 5,000 daltons to 10,000,000 daltons. Non-limiting examples of such polymers are described in the CTFA *International Cosmetic Ingredient Dictionary/Handbook* via the CTFA website as well as the CTFA Cosmetic Ingredient Handbook, Ninth Ed., Cosmetic and Fragrance Assn., Inc., Washington D.C. (2002), can be used.

Suitable cationic polymers can be synthetically derived or natural polymers can be synthetically modified to contain cationic moieties. In one aspect, the cationic polymer contains at least one repeating unit containing a quaternary ammonium salt moiety. Such polymers can be prepared by the polymerization of a diallylamine such as dialkyldiallylammonium salt or copolymer thereof in which the alkyl group contains 1 to 22 carbon atoms in one aspect and methyl or ethyl in another aspect. Copolymers containing a quaternary moiety derived from a dialkyldiallylammonium salt and an anionic component derived from anionic monomers of acrylic acid and methacrylic acid are suitable conditioning agents. Also suitable are, polyampholyte terpolymers having a cationic component prepared from a derivative of diallylamine,

such as a dimethyldiallylammonium salt, an anionic component derived from anionic monomers of acrylic acid or 2-acrylamido-2-methylpropane sulfonic acid and a nonionic component derived from nonionic monomers of acrylamide. The preparation of such quaternary ammonium salt moiety containing polymers can be found, for example, in U.S. Patent. Nos. 3,288,770; 3,412,019; 4,772,462 and 5,275,809

In one aspect, suitable cationic polymers include the chloride salts of the foregoing quaternized homopolymers and copolymers in which the alkyl group is methyl or ethyl, and are commercially available under the Merquat® series of trademarks from Lubrizol Advanced Materials, Inc. A homopolymer prepared from diallyl dimethyl ammonium chloride (DADMAC) having the CTFA name, Polyquaternium-6, is available under the Merquat 100 and Merquat 106 trademark. A copolymer prepared from DADMAC and acrylamide having the CTFA name, Polyquaternium-7, is sold under the Merquat 550 trademark. Another copolymer prepared from DADMAC and acrylic acid having the CTFA name, Polyquaternium-22, is sold under the Merquat 280 trademark. The preparation of Polyquaternium-22 and its related polymers is described in U.S. Patent. No. 4,772,462

Also useful is an ampholytic terpolymer prepared from a nonionic component derived from acrylamide or methyl acrylate, a cationic component derived from DADMAC or methacrylamidopropyl trimethyl ammonium chloride (MAPTAC), and an anionic component derived from acrylic acid or 2-acrylamido-2-methylpropane sulfonic acid or combinations of acrylic acid and 2-acrylamido-2-methylpropane sulfonic acid. An ampholytic terpolymer prepared from acrylic acid, DADMAC and acrylamide having the CTFA name, Polyquarternium-39, is available under the Merquat Plus 3330 and Mequat 3330PR trademarks. Another ampholytic terpolymer prepared from acrylic acid, methacrylamidopropyl trimethyl ammonium chloride (MAPTAC) and methyl acrylate having the CTFA name, Polyquarternium-47, is available under the Merquat 2001 trademark. Still another ampholytic terpolymer prepared from acrylic acid, MAPTAC and acrylamide having the CTFA name, Polyquarternium-53, is available under the Merquat 2003PR trademark. The preparation of such terpolymers is described in U.S. Patent. No. 5,275,809

Exemplary cationically modified natural polymers suitable for use in the hair conditioning composition includes polysaccharide polymers, such as cationically modified cellulose and cationically modified starch derivatives modified with a quaternary ammonium halide moiety. Exemplary cationically modified cellulose polymers are salts of hydroxyethyl cellulose reacted with trimethyl ammonium substituted epoxide (CTFA, Polyquaternium-10). Other suitable types of cationically modified cellulose include the polymeric quaternary ammonium salts of hydroxyethyl cellulose reacted with lauryl dimethyl ammonium substituted epoxide (CTFA, Polyquaternium-24). Cationically modified potato starch having the CTFA name, Starch Hydroxypropyltrimonium Chloride, is available under the Sensomer™ CI-50 trademark, from Lubrizol Advanced Materials, Inc.

Other suitable cationically modified natural polymers include cationic polygalactomannan derivatives such as guar gum derivatives and cassia gum derivatives, e.g., CTFA: Guar Hydroxypropyltrimonium Chloride and Cassia Hydroxypropyltrimonium Chloride. Guar hydroxypropyltrimonium chloride is commercially available under the Jaguar™ trade name series from Rhodia Inc. and the N-Hance trade name series from Ashland Inc. Cassia Hydroxypropyltrimonium Chloride is commercially available under the Sensomer™ CT-250 and Sensomer™ CT-400 trademarks from Lubrizol Advanced Materials, Inc.

Representative cationic and ampholytic polymers and copolymers suitable as conditioners and/or deposition aids in the disclosed technology have the CTFA names Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquarternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquarternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, and mixtures thereof.

The cationic and ampholytic polymers can be present from 0.05 to 5 wt. % percent, or from 0.1 to 3 wt. %, or from 0.5 to 2.0 wt.%, based on the total weight of the composition.

Silicone Conditioning Agents

Silicone conditioning agents can be used in compositions employing the ASE and HASE polymers of the present

technology. The optional silicone conditioning agent may comprise a volatile silicone, a nonvolatile silicone, or combinations thereof. The silicone conditioning agent particles may comprise volatile silicone, non-volatile silicone, or combinations thereof. Preferred are non-volatile silicone conditioning agents. If volatile silicones are present, they will typically be incidental to their use as a solvent or carrier for commercially available forms of non-volatile silicone ingredients, such as silicone gums and resins. The silicone hair conditioning agents for use in the disclosed technology have a viscosity ranging from 20 to 2,000,000 centistokes (1 centistoke equals 1 x $10^{-6}$ $m^2/s$), or from 1,000 to 1,800,000 centistokes, or from 50,000 to 1,500,000, or from 100,000 to 1,500,000 centistokes, as measured at 25 °C.

In one embodiment, the dispersed silicone conditioning agent particles can have a volume average particle diameter ranging from 5 μm to 125 μm. For small particle application to hair, the volume average particle diameters range from 0.01 μm to 4 μm, or from 0.01 μm to 2 μm, or from 0.01 μm to 0.5 μm. For larger particle application to hair, the volume average particle diameters typically range from 5 μm to 25 μm, or from 10 μm to 90 μm, or from 15 μm to 70 μm, or from 20 μm to 50 μm.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989), incorporated herein by reference. Silicone fluids are generally described as alkylsiloxane polymers. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609

Silicone fluids include silicone oils, which are flowable silicone materials having a viscosity, as measured at 25 °C of less than 1,000,000 cSt, and typically range from 5 cSt to 1,000,000 cSt. Suitable silicone oils include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used.

Silicone oils include polyalkyl, polyaryl siloxanes, or polyalkylaryl siloxanes which conform to the following formula:

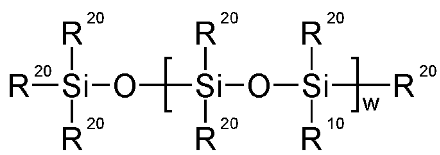

wherein $R^{20}$ is aliphatic, independently selected from alkyl, alkenyl, and aryl, $R^{20}$ can be substituted or unsubstituted, and w is an integer from 1 to about 8,000. Suitable unsubstituted $R^{20}$ groups for use in the personal cleansing compositions of the disclosed technology include, but are not limited to: alkoxy, aryloxy, alkaryl, arylalkyl, arylalkenyl, alkamino, and ether-substituted, hydroxyl-substituted, and halogen-substituted aliphatic and aryl groups. Suitable $R^{20}$ groups also include cationic amines and quaternary ammonium groups.

In one aspect of the disclosed technology, exemplary $R^{20}$ alkyl and alkenyl substituents range from $C_1$-$C_5$ alkyl and alkenyl, from $C_1$-$C_4$ in another aspect, from $C_1$-$C_2$ in a further aspect. The aliphatic portions of other alkyl-, alkenyl-, or alkynyl-containing groups (such as alkoxy, alkaryl, and alkamino) can be straight or branched chains, and range from $C_1$-$C_5$ in one aspect, from $C_1$-$C_4$ in another aspect, and from $C_1$-$C_2$ in a further aspect. As discussed above, the $R^{20}$ substituents can also contain amino functionalities (e.g., alkamino groups), which can be primary, secondary or tertiary amines or quaternary ammonium. These include mono-, di- and tri-alkylamino and alkoxyamino groups, wherein the aliphatic portion chain length is as described above.

Exemplary siloxanes are polydimethyl siloxane, polydiethylsiloxane, and polymethylphenylsiloxane. These siloxanes are available, for example, from the General Electric Company in their Viscasil R and SF 96 series, and from Dow Corning marketed under the Dow Corning 200 series. Exemplary polyalkylaryl siloxane fluids that may be used, include, for example, polymethylphenylsiloxanes. These siloxanes are available, for example, from the General Electric Company as SF 1075 methyl phenyl fluid or from Dow Corning as 556 Cosmetic Grade Fluid.

Cationic silicone fluids are also suitable for use in the disclosed technology. The cationic silicone fluids can be represented, but are not limited, to the general formula):

$$(R^{21})_eG_{3-f}\text{-Si-}(OSiG_2)_g\text{-}(OSiG_f(R_1)_{(2-f)h}\text{-O-}SiG_{3-e}(R^{21})_f$$

wherein G is hydrogen, phenyl, hydroxy, or $C_1$-$C_8$ alkyl, preferably methyl; e is 0 or an integer having of from 1 to 3; f is 0 or 1; g is a number from 0 to 1,999; h is an integer from 1 to 2,000, preferably from 1 to 10; the sum of g and h is a number from 1 to 2,000 in one aspect, and from 50 to 500 in another aspect of the disclosed technology; $R^{21}$ is a monovalent radical conforming to the general formula $C_qH_{2q}L$, wherein q is an integer having a value from 2 to 8 and L is selected from the following groups:

a) $-N(R^{22})CH_2CH_2N(R^{22})_2$
b) $-N(R^{22})$
c) $-N(R^{22})_3$ CA-
d) $-N(R^{22})CH_2CH_2N(R^{22})_2H_2$ $CA^-$

wherein $R^{22}$ is independently selected from hydrogen, $C_1$-$C_{20}$ alkyl, phenyl, benzyl; and $A^-$ is a halide ion selected from chloride, bromide, fluoride, and iodide.

An exemplary cationic silicone corresponding to the previous formula defined immediately above is the polymer known as "trimethylsilylamodimethicone" of formula:

$$(CH_3)_3\text{-Si-}[O\text{-Si}(CH_3)_2)]_g\text{-}[O\text{-}(CH_3)Si((CH_2)_3\text{-NH-}(CH_2)_2\text{-NH}_2)]_h\text{-O-Si}(CH_3)_3$$

Another cationic silicone useful in the disclosed technology can be represented by the formula:

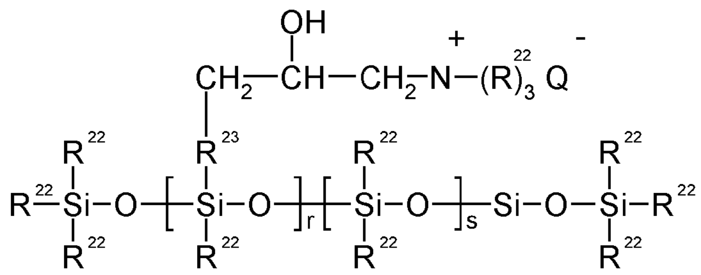

wherein where $R^{22}$ represents a radical selected from a $C_1$-$C_{18}$ alkyl and $C_1$-$C_{18}$ alkenyl radical; $R^{23}$ independently represents a radical selected from a $C_1$-$C_{18}$ alkylene radical or a $C_1$-$C_{18}$ alkyleneoxy radical; Q is a halide ion; r denotes an average statistical value from 2 to 20 in one aspect, and from 2 to 8 in another aspect; s denotes an average statistical value from 20 to 200 in one aspect, and from 20 to 50 in another aspect. In one aspect $R^{22}$ is methyl. In another aspect Q is chloride.

Other optional silicone fluids are the insoluble silicone gums. These gums are polysiloxane materials having a viscosity at 25 °C of greater than or equal to 1,000,000 centistokes. Silicone gums are described in U.S. Pat. No. 4,152,416; Noll and Walter, Chemistry and Technology of Silicones, New York: Academic Press 1968; and in General Electric Silicone Rubber Product Data Sheets SE 30, SE 33, SE 54 and SE 76

The silicone gums will typically have a mass molecule weight in excess of 200,000 Daltons, generally between 200,000 to 1,000,000 Daltons, specific examples of which include polydimethylsiloxane, polydimethylsiloxane/methylvinylsiloxane copolymer, polydimethylsiloxane/diphenyl siloxane/methylvinylsiloxane) copolymer, and mixtures thereof.

Another category of nonvolatile, insoluble silicone fluid conditioning agents are the high refractive index polysiloxanes, having a refractive index of at least 1.46, or at least 1.48, or at least 1.52, or at least 1.55. The refractive index of the polysiloxane fluid will generally be less than 1.70, typically less than 1.60. In this context, polysiloxane "fluid" includes oils as well as gums.

The high refractive index polysiloxane fluid includes those represented by the general formula set forth for the polyalkyl, polyaryl, and polyalkylaryl siloxanes described above, as well as cyclic polysiloxanes (cyclomethicones) represented by the formula:

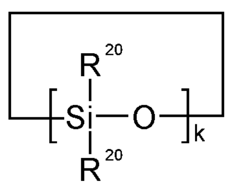

wherein the substituent $R^{20}$ is as defined above, and the number of repeat units, k, ranges from 3 to 7 in one aspect, and from 3 to 5 in another aspect. The high refractive index polysiloxane fluids can contain an amount of aryl containing $R^{20}$ substituents sufficient to increase the refractive index to the desired level, which is described above. Additionally, $R^{20}$ and k must be selected so that the material is non-volatile. Aryl containing substituents include those which contain alicyclic and heterocyclic five- and six-member aryl rings and those which contain fused five or six member rings. The aryl rings can be substituted or unsubstituted. Substituents include aliphatic substituents, and can also include alkoxy substituents, acyl substituents, ketones, halogens (e.g., Cl and Br), amines, etc. Exemplary aryl containing groups include substituted and

unsubstituted arenes, such as phenyl, and phenyl derivatives such as phenyls with $C_1$-$C_5$ alkyl or alkenyl substituents, e.g., allylphenyl, methyl phenyl and ethyl phenyl, vinyl phenyls such as styrenyl, and phenyl alkynes (e.g. phenyl $C_2$-$C_4$ alkynes). Heterocyclic aryl groups include substituents derived from furan, imidazole, pyrrole, pyridine, etc. Fused aryl ring substituents include, for example, naphthalene, coumarin, and purine.

The high refractive index polysiloxane fluids will have a degree of aryl containing substituents of at least 15% by wt., or at least 20% by wt., or at least 25% by wt., or at least 35% by wt., or at least 50% by wt., based on the wt. of the polysiloxane fluid. Typically, the degree of aryl substitution will be less than 90% by wt., more typically less than 85% by wt., and can generally ranges from 55% to 80% by wt. of the polysiloxane fluid.

In another aspect, the high refractive index polysiloxane fluids have a combination of phenyl or substituted phenyl derivatives. The substituents can be selected from $C_1$-$C_4$ alkyl (e.g., methyl), hydroxy, and $C_1$-$C_4$ alkylamino (e.g., $-R^{24}NHR^{25}NH_2$ wherein each $R^{24}$ and $R^{25}$ group independently is a $C_1$-$C_3$ alkyl, alkenyl, and/or alkoxy).

When high refractive index silicones are used in the compositions of the present technology, they optionally can be used in solution with a spreading agent, such as a silicone resin or a surfactant, to reduce the surface tension by a sufficient amount to enhance spreading and thereby enhance the glossiness (subsequent to drying) of hair treated with such compositions. Silicone fluids suitable for use in the compositions of the present technology are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984) . High refractive index polysiloxanes are available from Dow Corning Corporation (Midland, Mich.) Huls America (Piscataway, N.J.), and General Electric Silicones (Waterford, N.Y.).

Silicone resins can be included in the silicone conditioning agent suitable for use in the present composition. These resins are crosslinked polysiloxanes. The crosslinking is introduced through the incorporation of trifunctional and tetrafunctional silanes with monofunctional or difunctional (or both) silanes during manufacture of the silicone resin.

As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. In one aspect, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and terachlorosilane, with the methyl-substituted silanes being most commonly utilized. Silicone resins are offered by General Electric as GE SS4230 and SS4267.

Silicone materials and silicone resins are identified according to a shorthand nomenclature system known to those of ordinary skill in the art as "MDTQ" nomenclature. Under this system, the silicone is described according to the presence of various siloxane monomer units which make up the silicone. Briefly, the symbol M denotes the monofunctional unit $(CH_3)_3SiO_{0.5}$; D denotes the difunctional unit $(CH_3)_2SiO$; T denotes the trifunctional unit $(CH_3)SiO_{15}$; and Q denotes the quadra- or tetra-functional unit $SiO_2$. Primes of the unit symbols (e.g., M', D', T', and Q') denote substituents other than methyl, and must be specifically defined for each occurrence. Typical alternate substituents include groups such as vinyl, phenyls, amines, hydroxyls, etc. The molar ratios of the various units, either in terms of subscripts to the symbol indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or M' in a silicone resin is indicative of higher levels of crosslinking. As discussed before, however, the overall level of crosslinking can also be indicated by the oxygen to silicon ratio.

Exemplary silicone resins for use in the compositions of the present technology include, but are not limited to MQ, MT, MTQ, MDT and MDTQ resins. In one aspect, methyl is the silicone resin substituent. In another aspect, the silicone resin is selected from a MQ resins, wherein the M:Q ratio is from 0.5:1.0 to 1.5:1.0 and the average molecular weight of the silicone resin is from 1000 to 10,000 Daltons.

When employed with non-volatile silicone fluids having a refractive index below 1.46, the weight ratio of the non-volatile silicone fluid to the silicone resin component, ranges from 4:1 to 400:1 in one aspect, from 9:1 to 200:1 in another aspect, from 19:1 to 100:1 in a further aspect, particularly when the silicone fluid component is a polydimethylsiloxane fluid or a mixture of polydimethylsiloxane fluid and polydimethylsiloxane gum as described above. Insofar as the silicone resin forms a part of the same phase in the compositions hereof as the silicone fluid, i.e., the conditioning active, the sum of the fluid and resin should be included in determining the level of silicone conditioning agent in the composition.

The volatile silicones described above include cyclic and linear polydimethylsiloxanes, and the like. Cyclic volatile silicones (cyclomethicones) typically contain 3 to 7 silicon atoms, alternating with oxygen atoms, in a cyclic ring structure such as described above for the non-volatile cyclic silicones. However, each $R^{20}$ substituent and repeating unit, k, in the formula must be selected so that the material is non-volatile. Typically, $R^{20}$ is substituted with two alkyl groups (e.g., methyl groups). The linear volatile silicones are silicone fluids, as described above, having viscosities of not more than 25 mPa·s. "Volatile" means that the silicone has a measurable vapor pressure, or a vapor pressure of at least 2 mm of Hg at 20

°C. Non-volatile silicones have a vapor pressure of less than 2 mm Hg at 20 °C. A description of cyclic and linear volatile silicones is found in Todd and Byers, "Volatile Silicone Fluids for Cosmetics", Cosmetics and Toiletries, Vol. 91(1), pp. 27-32 (1976), and in Kasprzak, "Volatile Silicones", Soap/Cosmetics/Chemical Specialities, pp. 40-43 (December 1986), each incorporated herein by reference.

Exemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from G.E. Silicones as SF1173, SF1202, SF1256, and SF1258, Dow Corning Corporation as Dow Corning® 244, 245, 246, 345, and 1401 Fluids. Blends of volatile cyclomethicones and volatile linear dimethicones are also contemplated.

Exemplary volatile linear dimethicones include hexamethyldisiloxane, octamethyltrisiloxane, decamethyltetrasiloxane, dodecamethylpentasiloxane and blends thereof. Volatile linear dimethicones and dimethicone blends are commercially available from Dow Corning Corporation as Dow Corning 200® Fluid (e.g., product designations 0.65 CST, 1 CST, 1.5 CST, and 2 CST) and Dow Corning® 2-1184 Fluid.

Emulsified silicones are also suitable for combination in the disclosed technology. Typically, silicone emulsions have an average silicone particle size in the composition of less than 30 μm, or less than 20 μm, or less than 10 μm. In one aspect of the disclosed technology, the average silicone particle size of the emulsified silicone in the composition is less than 2 μm, and ideally it ranges from 0.01 to 1 μm. Silicone emulsions having an average silicone particle size of <0.15 micrometers are generally termed micro-emulsions. Particle size may be measured by means of a laser light scattering technique, using a 2600D Particle Sizer from Malvern Instruments. Suitable silicone emulsions for use in the disclosed technology are also commercially available in a pre-emulsified form. Examples of suitable pre-formed emulsions include emulsions DC2-1766, DC2-1784, and micro-emulsions DC2-1865 and DC2-1870, all available from Dow Corning. These are all emulsions/micro-emulsions of dimethiconol. Crosslinked silicone gums are also available in a pre-emulsified form, which is advantageous for ease of formulation. An exemplary material is available from Dow Corning as DC X2-1787, which is an emulsion of crosslinked dimethiconol gum. Another exemplary material is available from Dow Corning as DC X2-1391, which is a micro-emulsion of crosslinked dimethiconol gum. Preformed emulsions of amino functional silicone are also available from suppliers of silicone oils such as Dow Corning and General Electric. Particularly suitable are emulsions of amino functional silicone oils with nonionic and/or cationic surfactant. Specific examples include DC929 Cationic Emulsion, DC939 Cationic Emulsion, DC949 Cationic emulsion, and the non-ionic emulsions DC2-7224, DC2-8467, DC2-8177 and DC2-8154 (all available from Dow Corning). Mixtures of any of the above types of silicone may also be used. Specific examples of amino functional silicones suitable are the aminosilicone oils DC2-8220, DC2-8166, DC2-8466, and DC2-8950-114 (all available from Dow Corning), and GE 1149-75, (General Electric Silicones). An example of a quaternary silicone polymer useful in the disclosed technology is the material K3474, available from Goldschmidt, Germany.

Other suitable silicone oils include the dimethicone copolyols, which are linear or branched copolymers of dimethylsiloxane (dimethicone) modified with alkylene oxide units. The alkylene oxide units can be arranged as random or block copolymers. A generally useful class of dimethicone polyols are block copolymers having terminal and/or pendent blocks of polydimethylsiloxane and blocks of polyalkylene oxide, such as blocks of polyethylene oxide, polypropylene oxide, or both. Dimethicone copolyols can be water soluble or insoluble depending on the amount of polyalkylene oxide present in the dimethicone polymer and can be anionic, cationic, or nonionic in character.

The water soluble or water dispersible silicones can also be used in the disclosed technology. Such water-soluble silicones contain suitable anionic functionality, cationic functionality, and/or nonionic functionality to render the silicone water soluble or water dispersible. In one embodiment, the water-soluble silicones contain a polysiloxane main chain to which is grafted at least one anionic moiety. The anionic moiety can be grafted to a terminal end of the polysiloxane backbone, or be grafted as a pendant side group, or both. By anionic group is meant any hydrocarbon moiety that contains at least one anionic group or at least one group that can be ionized to an anionic group following neutralization by a base. As discussed previously, the quantity of the hydrocarbon groups of anionic character which are grafted onto the silicone chain are chosen so that the corresponding silicone derivative is water-soluble or water-dispersible after neutralization of the ionizable groups with a base. The anionic silicone derivatives can be selected from existing commercial products or can be synthesized by any means known in the art. The nonionic silicones contain alkylene oxide terminal and/or pendant side chain units (e.g., dimethicone copolyols).

Silicones with anionic groups can be synthesized by reaction between (i) a polysiloxane containing a silinic hydrogen and (ii) a compound containing olefinic unsaturation that also contains an anionic functional group. Exemplary of such a reaction is the hydrosilylation reaction between poly(dimethylsiloxanes) containing a Si-H group(s) and an olefin, $CH_2=CHR^{26}$, wherein $R^{26}$ represents a moiety containing an anionic group. The olefin can be monomeric, oligomeric, or polymeric. Polysiloxane compounds that contain a pendant reactive thio (-SH) group(s) are also suitable for grafting an unsaturated anionic group containing compound to the poly(siloxane) backbone.

According to one aspect of the disclosed technology, the anionic monomers containing ethylenic unsaturation are used alone or in combination and are selected from linear or branched, unsaturated carboxylic acids. Exemplary

unsaturated carboxylic acids are acrylic acid, methacrylic acid, maleic acid, maleic anhydride, itaconic acid, fumaric acid and crotonic acid. The monomers can optionally be partially or completely neutralized by base to form an alkali, alkaline earth metal, and ammonium salt. Suitable bases include but are not limited to the alkali, alkaline earth (e.g., sodium, potassium, lithium, calcium) and ammonium hydroxides. It will be noted that, similarly, the oligomeric and polymeric graft segments formed from the forgoing monomers can be post-neutralized with a base (sodium hydroxide, aqueous ammonia, etc.) to form a salt. Examples of silicone derivatives which are suitable for use in the disclosed technology are described in patent applications numbers EP-A-0 582,152 and WO 93/23009. An exemplary class of silicone polymers are the polysiloxanes containing repeat units represented by the following structure:

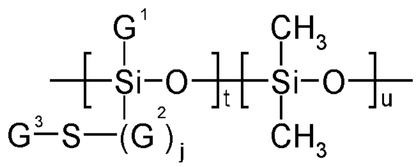

wherein $G^1$ represents hydrogen, $C_1$-$C_{10}$ alkyl and phenyl radical; $G^2$ represents $C_1$-$C_{10}$ alkylene; $G^2$ represents an anionic polymeric residue obtained from the polymerization of at least one anionic monomer containing ethylenic unsaturation; j is 0 or 1; t is an integer ranging from 1 to 50; and u is an integer from 10 to 350. In one embodiment of the disclosed technology, $G^1$ is methyl; j is 1; and $G_2$ is propylene radical; $G^3$ represents a polymeric radical obtained from the polymerization of at least one unsaturated monomer containing a carboxylic acid group (e.g., acrylic acid, methacrylic acid, itaconic acid, fumaric acid, crotonic acid, maleic acid, or aconitic acid, and the like).

The carboxylate group content in the final polymer preferably ranges from 1 mole of carboxylate per 200 g of polymer to 1 mole of carboxylate per 5000 g of polymer. The number molecular mass of the silicone polymer preferably ranges from 10,000 to 1,000,000 and still more preferably from 10,000 to 100,000. Exemplary unsaturated monomers containing carboxylic acid groups are acrylic acid and methacrylic acid. In addition, to the carboxylic acid group containing monomers, $C_1$-$C_{20}$ alkyl esters of acrylic acid and methacrylic acid can be copolymerized into the polymeric backbone. Exemplary esters include but are not limited to the ethyl and butyl esters of acrylic and methacrylic acid. A commercially available silicone-acrylate polymer is marketed by the 3M Company under the trademark Silicones "Plus" Polymer 9857C (VS80 Dry). These polymers contain a polydimethylsiloxanes (PDMS) backbone onto which is grafted (through a thiopropylene group) random repeating units of poly(meth)acrylic acid and the butyl ester of poly(meth)acrylate. These products can be obtained conventionally by radical copolymerization between thiopropyl functionalized polydimethylsiloxane and a mixture of monomers comprising (meth)acrylic acid and of butyl(meth)acrylate.

In another embodiment the water-soluble silicone copolyol useful in the practice of the disclosed technology can be represented silicone copolyol carboxylates represented by the formula:

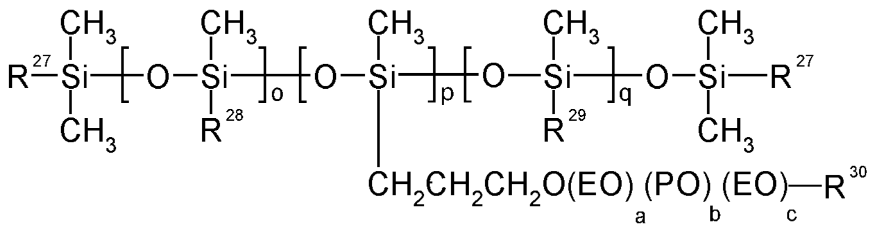

where $R^{27}$ and $R^{28}$ are independently selected from $C_1$-$C_{30}$ alkyl, $C_6$-$C_{14}$ aryl, $C_7$-$C_{15}$ aralkyl, $C_1$-$C_{15}$ alkaryl, or an alkenyl group of 1 to 40 carbons, hydroxyl, -$R^{31}$-G' or -$(CH_2)_3O(EO)_a(PO)_b(EO)_c$-G', with the proviso that both $R^{27}$ and $R^{28}$ are not methyl; $R^{29}$ is selected from $C_1$-$C_5$ alkyl or phenyl; in this formula a, b, and c are integers independently ranging from 0 to 100; EO is ethylene oxide, - $(CH_2CH_2O)$-; PO is propylene oxide, -$(CH_2CH(CH_3)O)$-; in this formula o is an integer ranging from 1 to 200, p is an integer ranging from 0 to 200, and q is an integer ranging from 0 to 1000; $R^{30}$ is hydrogen, $C_1$-$C_{30}$ alkyl, aryl, $C_7$-$C_{15}$ aralkyl, $C_7$-$C_{15}$ alkaryl, or alkenyl group of 1 to 40 carbons or -C(O)-X wherein X is $C_1$-$C_{30}$ alkyl, $C_6$-$C_{14}$ aryl, $C_7$-$C_{15}$ aralkyl, $C_1$-$C_{15}$alkaryl, or an alkenyl group of 1 to 40 carbons, or a mixture thereof; $R^{31}$ is a divalent group selected from alkylene radical of 1 to 40 carbon atoms which may be interrupted with arylene group of 6 to 18 carbons or an alkylene group containing unsaturation of 2 to 8 carbons; and G' is independently are selected from:

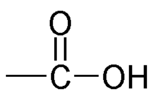 ; 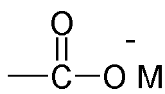 ; 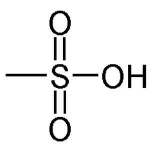 ;

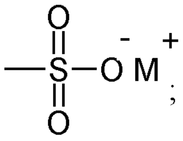 ; 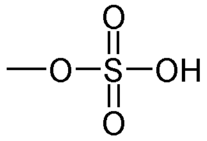 ; 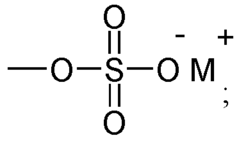 ;

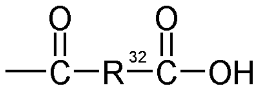 ; 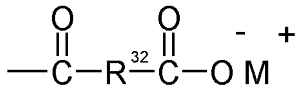

where $R^{32}$ is a divalent group selected from alkylene of 1 to 40 carbons, an unsaturated group containing 2 to 5 carbon atoms, or an arylene group of 6 to 12 carbon atoms; where M is a cation selected from Na, K, Li, $NH_4$, or an amine containing $C_1$-$C_{10}$ alkyl, $C_6$-$C_{14}$ aryl (e.g., phenyl, naphthyl), $C_2$-$C_{10}$ alkenyl, $C_1$-$C_{10}$ hydroxyalkyl, $C_7$-$C_{24}$ arylalkyl or $C_7$-$C_{24}$ alkaryl groups. Representative $R^{32}$ radicals are: $-CH_2CH_2-$, -CH=CH-, $-CH=CHCH_2-$, and phenylene.

In another embodiment the water-soluble silicones useful in the practice of the disclosed technology can be represented by an anionic silicone copolyol represented by the formula:

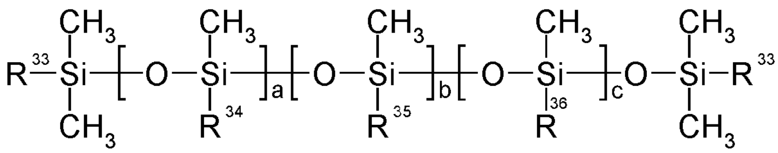

where is $R^{33}$ is methyl or hydroxyl; $R^{34}$ is selected from $C_1$-$C_8$ alkyl or phenyl; $R^{35}$ represents the radical $-(CH_2)_3O(EO)_x(PO)_y(EO)_z-SO_3^-M^+$; where M is a cation selected from Na, K, Li, or $NH_4$; in this formula x, y and z are integers independently ranging from 0 to 100; $R^{36}$ represents the radical - $(CH_2)_3O(EO)_x(PO)_y(EO)_z$-H; in this formula a and c are independently integers ranging from 0 to 50, and b is an integer ranging from 1 to 50; EO is ethylene oxide, e.g., $-(CH_2CH_2O)-$; PO is propylene oxide, e.g., $-(CH_2CH(CH_3)O)-$.

In still another embodiment the water-soluble silicones useful in the practice of the disclosed technology can be represented by an anionic silicone copolyol represented by the formula:

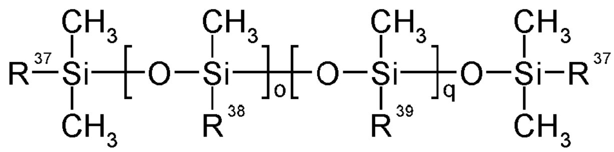

wherein $R^{37}$ and $R^{38}$ independently are $-CH_3$ or a radical represented by: $-(CH_2)_3O(EO)_a(PO)_b(EO)_c-C(O)-R^{40}-C(O)OH$, subject to the proviso that both $R^{37}$ and $R^{38}$ are not $-CH_3$ at the same time; $R^{40}$ is selected from the divalent radical $-CH_2CH_2$, -CH=CH-, and phenylene; $R^{39}$ is selected from $C_1$-$C_5$ alkyl or phenyl; in this formula a, b and c are integers independently ranging from 0 to 20; EO is an ethylene oxide residue, e.g., $-(CH_2CH_2O)-$; PO is a propylene oxide residue, e.g., $-(CH_2CH(CH_3)O)-$; in this formula o is an integer ranging from 1 to 200 and q is an integer ranging from 0 to 500.

Other water-soluble silicones useful in the disclosed technology are quaternized silicone copolyol polymers. These polymers have a pendant quaternary nitrogen functional group present and are represented by the formula:

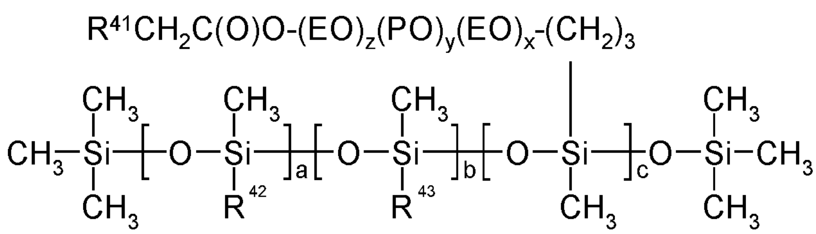

where $R^{41}$ represents a quaternary substituent $-N^+R^3R^4R^5\ X^-$, wherein $R^3$and $R^4$, and $R^5$, independently, are selected from hydrogen and linear and branched $C_1$-$C_{24}$ alkyl, and $X^-$ represents an anion suitable to balance the cationic charge on the nitrogen atom; $R^{42}$ is selected from $C_1$-$C_{10}$ alkyl and phenyl; $R^{43}$ is - $(CH_2)_{30}(EO)_x(PO)_y(EO)_z$-H, where EO is an ethylene oxide residue, e.g., - $(CH_2CH_2O)-$; PO is a propylene oxide residue, e.g., $-(CH_2CH(CH_3)O)-$; in this formula a is an integer from 0 to 200, b is an integer from 0 to 200, and c is an integer from 1 to 200; in this formula x, y and z are integers and

are independently selected from 0 to 20. In one aspect, the anion $X^-$ represents an anion selected from chloride, bromide, iodide, sulfate, methylsulfate, sulfonate, nitrate, phosphate, and acetate.

Other suitable water-soluble silicones are amine substituted silicone copolyols represented by the formula:

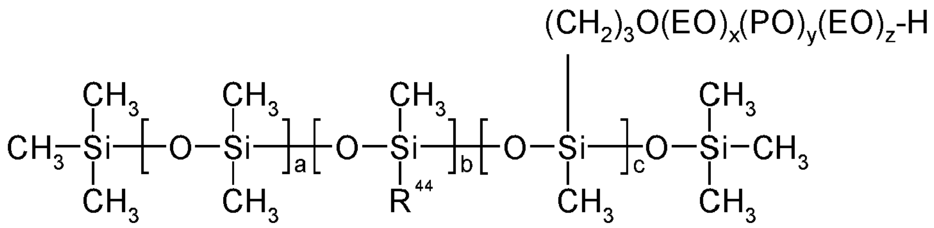

where $R^{44}$ is selected from $-NH(CH_2)_nNH_2$ or $-(CH_2)_nNH_2$, where in this formula n is an integer from 2 to 6; and x is an integer from 0 to 20; where EO is an ethylene oxide residue, e.g., $-(CH_2CH_2O)-$; PO is a propylene oxide residue, e.g., $-(CH_2CH(CH_3)O)-$; in this formula a is an integer from 0 to 200, b is an integer from 0 to 200, and c is an integer from 1 to 200; in this formula x, y and z are integers and are independently selected from 0 to 20.

Still other water-soluble silicones can be selected from nonionic silicone copolyols (dimethicone copolyols) represented by the formula:

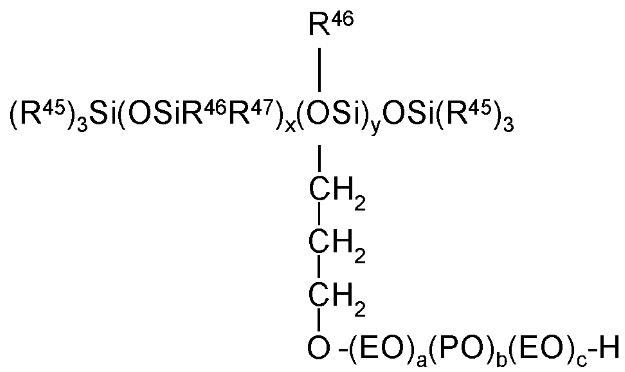

where $R^{45}$, independently, represents a radical selected from $C_1$-$C_{30}$ alkyl, $C_6$-$C_{14}$ aryl, and $C_2$-$C_{20}$ alkenyl; $R^{46}$ represents a radical selected from $C_1$-$C_{30}$ alkyl, $C_6$-$C_{14}$ aryl, and $C_2$-$C_{20}$ alkenyl; EO is an ethylene oxide residue, e.g., $-(CH_2CH_2O)-$; PO is a propylene oxide residue, e.g., $-(CH_2CH(CH_3)O)-$; in this formula (a), (b), and (c) are, independently, 0 to 100; in this formula x is 0 to 200; and y is 1 to 200.

In another embodiment water soluble silicones can be selected from nonionic silicone copolyols represented by the formula:

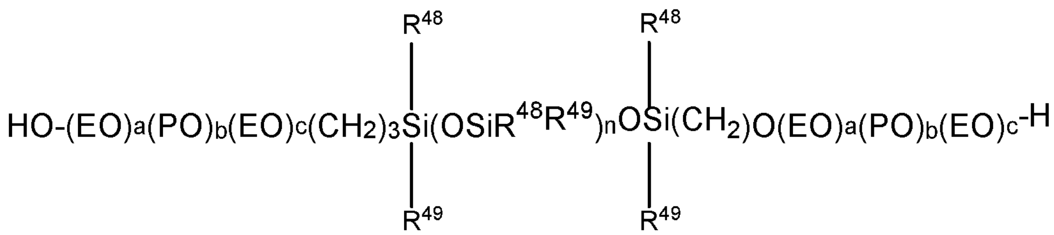

wherein $R^{48}$ and $R^{49}$, independently, represent a radical selected from $C_1$-$C_{30}$ alkyl, $C_6$-$C_{14}$ aryl, and $C_2$-$C_{20}$ alkenyl; EO is an ethylene oxide residue, e.g., $-(CH_2CH_2O)-$; PO is a propylene oxide residue, e.g., $-(CH_2CH(CH_3)O)-$; in this formula a, b, and c are independently 0 to 100; and in this formula n is 0 to 200.

In the copolyol embodiments set forth above, the EO and PO residues can be arranged in random, non-random, or blocky sequences.

Dimethicone copolyols are disclosed in U.S. Patent Nos. 5,136,063 and 5,180,843

In addition, dimethicone copolyols are commercially available under the Silsoft® and Silwet® brand names from the General Electric Company (GE-OSi). Specific product designations include but are not limited to Silsoft 305, 430, 475, 810, 895, Silwet L 7604 (GE-OSi); Dow Corning® 5103 and 5329 from Dow Corning Corporation; and Abil® dimethicone copolyols, such as, for example WE 09, WS 08, EM 90 and EM 97 from Evonik Goldschmidt Corporation; and Silsense™

dimethicone copolyols, such as Silsense Copolyol-1 and Silsense Copolyol-7, available from Lubrizol Advanced Materials, Inc.

The concentration of the silicone conditioning agent can range from 0.01% to 10%, by weight of the composition in which it is included. In another aspect the amount of silicone conditioning agent ranges from 0.1% to 8%, from 0.1% to 5% in still another aspect, and from 0.2% to 3% by wt. in a further aspect, all based on the total weight of the composition.

The silicone can be added from 0.05 to 20 wt.%, or from 0.08 to 5 wt.%, or from 0.1 to 3 wt.%, based on the weight of the total composition.

Conditioning Oils, Waxes and Esters

Suitable conditioning oils for use as conditioning agents (e.g., for the hair, scalp, skin, and nails) in the compositions of the disclosed technology include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils typically contain about 12 to 19 carbon atoms. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2,2,4,4,6,6,8,8-dimethyl-10-methylundecane and 2,2,4,4,6,6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from BP Chemical Company.

Natural oil conditioners are also useful in the practice of disclosed technology, and include but are not limited to peanut, sesame, avocado, coconut, cocoa butter, almond, safflower, corn, cotton seed, sesame seed, walnut oil, castor, olive, jojoba, palm, palm kernel, soybean, wheat germ, linseed, sunflower seed; eucalyptus, lavender, vetiver, litsea, cubeba, lemon, sandalwood, rosemary, chamomile, savory, nutmeg, cinnamon, hyssop, caraway, orange, geranium, cade, and bergamot oils, fish oils, glycerol tricaprocaprylate; and mixtures thereof. The natural oils can also be utilized as emollients.

Natural and synthetic wax conditioning agents can be employed in the compositions of the disclosed technology, including but are not limited to carnauba wax, carnauba acid wax, hydrolyzed carnauba wax, ethoxylated carnauba wax (e.g., PEG-12 carnauba wax), candelilla wax, hydrolyzed candelilla wax, clover wax, alfalfa wax, paraffin wax, ozokerite wax, olive wax, rice wax, hydrogenated castor wax, bayberry wax, hydrogenated jojoba wax, bees wax, modified bees wax, e.g., cera bellina wax, ethoxylated beeswax (e.g., PEG-6 beeswax, PEG-8 beeswax, PEG-12 beeswax, PEG-20 beeswax), dimethicone copolyol beeswax esters and dimethiconol beeswax ester (e.g. Bis-Hydroxyethoxypropyl Dimethicone Beeswax Esters, Dimethicone PEG-8 Beeswax, and Dimethiconol Beeswax available from Lubrizol Advanced Materials, Inc. under the Ultrabee® trademark), marine waxes, polyolefin waxes, e.g., polyethylene wax; and mixtures thereof.

Liquid polyolefin conditioning oils can be used in the compositions of the disclosed technology. The liquid polyolefin conditioning agents are typically poly-α-olefins that have been hydrogenated. Polyolefins for use herein can be prepared by the polymerization of $C_4$ to about $C_{14}$ olefinic monomers. Non-limiting examples of olefinic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. In one aspect of the disclosed technology hydrogenated α-olefin monomers include but are not limited to 1-hexene to 1-hexadecenes, 1-octene to 1-tetradecene, and mixtures thereof.

Fluorinated or perfluorinated oils are also contemplated within the scope of the disclosed technology. Fluorinated oils include perfluoropolyethers described in European Patent 0 486 135 and the fluorohydrocarbon compounds described in WO 93/11103. The fluoridated oils may also be fluorocarbons such as fluoramines, e.g., perfluorotributylamine, fluoridated hydrocarbons, such as perfluorodecahydronaphthalene, fluoroesters, and fluoroethers.

Other suitable ingredients include, but are not limited to, fatty esters having at least 10 carbon atoms. These fatty esters include esters derived from fatty acids or alcohols (e.g., mono-esters, polyhydric alcohol esters, and di- and tri-carboxylic acid esters). The fatty esters hereof may include or have covalently bonded thereto other compatible functionalities, such as amides and alkoxy moieties (e.g., ethoxy or ether linkages, etc.).

Exemplary fatty esters include, but are not limited to isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate,

lauryl acetate, cetyl propionate, and oleyl adipate.

Other fatty esters suitable for use in the compositions of the disclosed technology are mono-carboxylic acid esters of the general formula $R^{50}C(O)OR^{51}$, wherein $R^{50}$ and $R^{51}$ are alkyl or alkenyl radicals, and the sum of carbon atoms in $R^{50}$ and $R^{51}$ is at least 10 in one aspect, and at least 22 in another aspect of the disclosed technology.

Still other fatty esters suitable for use in the compositions of the disclosed technology are di- and tri-alkyl and alkenyl esters of carboxylic acids, such as esters of $C_4$-$C_8$ dicarboxylic acids (e.g., $C_1$-$C_{22}$ esters, preferably $C_{1\text{-}C6}$, of succinic acid, glutaric acid, adipic acid). Specific non-limiting examples of di- and tri-alkyl and alkenyl esters of carboxylic acids include isocetyl stearyol stearate, diisopropyl adipate, and tristearyl citrate.

Other fatty esters suitable for use in the compositions of the disclosed technology are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Specific non-limiting examples of suitable synthetic fatty esters for use in the personal cleansing compositions of the disclosed technology include P-43 ($C_8$-$C_{10}$ triester of trimethylolpropane), MCP-684 (tetraester of 3,3 diethanol-1,5 pentadiol), MCP 121 ($C_8$-$C_{10}$ diester of adipic acid), all of which are available from ExxonMobil Chemical Company.

Guerbet esters are also suitable in the compositions of the present technology. Guerbet esters can be formed from the esterification of a mono- or polyfunctional carboxylic acid by a Guerbet alcohol. Alternatively, the ester can be formed by reacting a Guerbet acid with a mono- or polyfunctional alcohol. For a review of Guerbet chemistry, see O'Lenick, A. J., Jr. 2001. Guerbet chemistry. Journal of Surfactants and Detergents 4: 311-315. Guerbet esters are commercially available from Lubrizol Advanced Materials, Inc. under product designations G-20, G-36, G-38, and G-66.

The amount of conditioning oils, waxes and esters can range from 0.05 to 10 wt.%, or from 0.5 to 5 wt.%. or from 1 to 3 wt.%, based on the total weight of the composition.

Fatty Acids and Fatty Alcohols

Fatty acids and fatty alcohols can be employed in the compositions of the present technology. Suitable fatty acids include saturated and unsaturated $C_8$ to $C_{30}$ fatty acids. Exemplary fatty acids include, but are not limited to, caprylic acid, capric acid, lauric acid, myristic acid, palmitic acid, palmitoleic acid, stearic acid, oleic acid, ricinoleic acid, vaccenic acid, linoleic acid, $\alpha$-linolenic acid, $\gamma$-linolenic acid, arachidic acid, gadoleic acid, arachidonic acid, EPA (5,8,11,14,17-eicosapentaenoic acid), behenic acid, erucic acid, DHA (4,7,10,13,16,19-docosahexaenoic acid), lignoceric acid, and mixtures thereof.

Alkoxylated fatty acids are also useful herein and can be formed by esterifying a fatty acid with an ethylene oxide and/or propylene oxide or with a pre-formed polymeric ether (e.g., polyethylene glycol or polypropylene glycol). The product is a polyethylene oxide ester, polypropylene oxide ester, or a polyethylene/polypropylene oxide ester of the respective fatty acid. In one aspect, an ethoxylated fatty acid can be represented by the formula: $R'\text{-}C(O)O(CH_2CH_2O)_{n'}\text{-}H$, wherein R' represents the aliphatic residue of a fatty acid and n' represents the number of ethylene oxide units. In another aspect, n' is an integer ranging from 2 to 50, from 3 to 25 in another aspect, and from 3 to 10 in a further aspect. In still another aspect of the present technology, R' is derived from a saturated or unsaturated fatty acid containing 8 to 30 carbon atoms. In another aspect, diesters can be formed by reacting two moles of the fatty acid with one mole of polyethylene or polypropylene glycol. The diesters can be represented by the formula: $R'\text{-}C(O)O(CH_2CH_2O)_{n'}(O)CR'$ where R' and n' are as defined immediately above.

Exemplary alkoxylated fatty acids include, but are not limited to, capric acid ethoxylate, lauric acid ethoxylate, myristic acid ethoxylate, stearic acid ethoxylate, oleic acid ethoxylate, coconut fatty acid ethoxylate, and the like, wherein the number of ethylene oxide units in each of the foregoing ethoxylates can range from 2 and above in one aspect, and from 2 to about 50 in another aspect. More specific examples of ethoxylated fatty acids are PEG-8 stearate (the 8 meaning the number of repeating ethylene oxide units), PEG-8 distearate, PEG-8 oleate, PEG-8 behenate, PEG-8 caprate, PEG-8 caprylate, PEG cocoates (PEG without a number designation meaning that the number of ethylene oxide units ranges from 2 to 50), PEG-15 dicocoate, PEG-2 diisononanoate, PEG-8 diisostearate, PEG-dilaurates, PEG-dioleates, PEG-distearates, PEG-ditallates, PEG-isostearates, PEG-jojoba acids, PEG-laurates, PEG-linolenates, PEG-myristates, PEG-oleates, PEG-palmitates, PEG-ricinoleates, PEG-stearates, PEG-tallates, and the like.

The fatty alcohols suitable for use in the compositions of the invention include, but are not limited to, the saturated and unsaturated $C_8$-$C_{30}$ fatty alcohols. Exemplary fatty alcohols include capryl alcohol, pelargonic alcohol, capric alcohol, decyl alcohol, undecyl alcohol, lauryl alcohol, myristyl alcohol, cetyl alcohol, isocetyl alcohol, stearyl alcohol, isostearyl alcohol, cetearyl alcohol, palmitoleyl alcohol, elaidyl alcohol, sterol, oleyl alcohol, linoleyl alcohol, elaidolinoleyl alcohol, linolenyl alcohol, ricinoleyl alcohol, arachidyl alcohol, icocenyl alcohol, behenyl alcohol, erucyl alcohol, lignoceryl alcohol,

ceryl alcohol, montanyl alcohol, myricyl alcohol, and mixtures thereof. Fatty alcohols are widely available and can be obtained through the hydrogenation of esterified vegetable and animal oils and fats.

Alkoxylated fatty alcohol compounds are ethers formed from the reaction of a fatty alcohol with an alkylene oxide, generally ethylene oxide or propylene oxide. Suitable ethoxylated fatty alcohols are adducts of fatty alcohols and polyethylene oxide. In one aspect of the present technology, the ethoxylated fatty alcohols can be represented by the formula $R'''-(OCH_2CH_2)_{n''}-OH$, wherein R‴ represents the aliphatic residue of the parent fatty alcohol and n" represents the number of ethylene oxide units. In another aspect of the present technology, R‴ is derived from a fatty alcohol containing 8 to 30 carbon atoms. In one aspect, n" is an integer ranging from 2 to 50, 3 to 25 in another aspect, and 3 to 10 in a further aspect. In a still further aspect, R‴ is derived from a fatty alcohol set forth immediately in the paragraph above. Exemplary ethoxylated fatty alcohols are but are not limited to capryl alcohol ethoxylate, lauryl alcohol ethoxylate, myristyl alcohol ethoxylate, cetyl alcohol ethoxylate, stearyl alcohol ethoxylate, cetearyl alcohol ethoxylate, sterol ethoxylate, oleyl alcohol ethoxylate, and, behenyl alcohol ethoxylate, wherein the number of ethylene oxide units in each of the foregoing ethoxylates can range from 2 and above in one aspect, and from 2 to about 150 in another aspect. It is to be recognized that the propoxylated adducts of the foregoing fatty alcohols and mixed ethoxylated/propoxylated adducts of the foregoing fatty alcohols are also contemplated within the scope of the present technology. The ethylene oxide and propylene oxide units of the ethoxylated/propoxylated fatty alcohols can be arranged in random or in blocky order.

Exemplary ethoxylated sterols include ethoxylated vegetable oil sterols such as, for example, soya sterols. The degree of ethoxylation is greater than 5 in one aspect, and at least 10 in another aspect. Suitable ethoxylated sterols are PEG-10 Soy Sterol, PEG-16 Soy Sterol and PEG-25 Soy Sterol.

Additional examples of ethoxylated alcohols are but are not limited to Beheneth 5-30 (the 5-30 meaning the range of repeating ethylene oxide units), Ceteareth 2-100, Ceteth 1-45, Cetoleth 24-25, Choleth 10-24, Coceth 3-10, C9-11 Pareth 3-8, C11-15 Pareth 5-40, C11-21 Pareth 3-10, C12-13 Pareth 3-15, Deceth 4-6, Dodoxynol 5-12, Glycereth 7-26, Isoceteth 10-30, Isodeceth 4-6, Isolaureth 3-6, isosteareth 3-50, Laneth 5-75, Laureth 1-40, Nonoxynol 1-120, Nonylnonoxynol 5-150, Octoxynol 3-70, Oleth 2-50, PEG 4-350, Steareth 2-100, and Trideceth 2-10.

Specific examples of propoxylated alcohols are but are not limited to PPG-10 Cetyl Ether, PPG-20 Cetyl Ether, PPG-28 Cetyl Ether, PPG-30 Cetyl Ether, PPG-50 Cetyl Ether, PPG-2 Lanolin Alcohol Ether, PPG-5 Lanolin Alcohol Ether, PPG-10 Lanolin Alcohol Ether, PPG-20 Lanolin Alcohol Ether, PPG-30 Lanolin Alcohol Ether, PPG-4 Lauryl Ether, PPG-7 Lauryl Ether, PPG-10 Oleyl Ether, PPG-20 Oleyl Ether, PPG-23 Oleyl Ether, PPG-30 Oleyl Ether, PPG-37 Oleyl Ether, PPG-50 Oleyl Ether, PPG-11 Stearyl Ether, PPG-15 Stearyl Ether, PPG-2 Lanolin Ether, PPG-5 Lanolin Ether, PPG-10 Lanolin Ether, PPG-20 Lanolin Ether, PPG-30 Lanolin Ether, and PPG-1 Myristyl Ether.

Specific examples of ethoxylated/propoxylated alcohols are but are not limited to PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-Isodeceth-4, PPG-2-Isodeceth-6, PPG-2-Isodeceth-8, PPG-2-Isodeceth-9, PPG-2-Isodeceth-10, PPG-2-Isodeceth-12, PPG-2-Isodeceth-18, PPG-2-Isodeceth-25, PPG-4-Isodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-Laureth-5, PPG-6-Laureth-3, PPG-25-Laureth-25, PPG-7 Lauryl Ether, PPG-3-Myreth-3, PPG-3-Myreth-11, PPG-20-PEG-20 Hydrogenated Lanolin, PPG-2-PEG-11 Hydrogenated Lauryl Alcohol Ether, PPG-12-PEG-50 Lanolin, PPG-12-PEG-65 Lanolin Oil, PPG-40-PEG-60 Lanolin Oil, PPG-1-PEG-9 Lauryl Glycol Ether, PPG-3-PEG-6 Oleyl Ether, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

The fatty acids and fatty alcohols be utilized in an amount ranging from 0.1 to 30 wt.%, or from 0.5 to 25 wt.%, or from 3 to 20 wt.%, or from 5 to 10 wt.%, based on the total weight of the composition.

## Humectants

Humectants are defined as materials that absorb or release water vapor, depending on the relative humidity of the environment, (Harry's Cosmeticology, Chemical Publishing Company Inc., 1982 p. 266). Suitable humectants that include, but are not limited to, allantoin; pyrrolidonecarboxylic acid and its salts; hyaluronic acid and salts thereof; sorbic acid and salts thereof; urea, lysine, cystine, and amino acids; polyhydroxy alcohols such as glycerin, propylene glycol,

hexylene glycol, hexanetriol, ethoxydiglycol, dimethicone copolyol, and sorbitol, and the esters thereof; polyethylene glycol; glycolic acid and glycolate salts (e.g. ammonium and quaternary alkyl ammonium); chitosan; aloe-vera extracts; algae extract; honey and derivatives thereof; inositol; lactic acid and lactate salts (e.g. ammonium and quaternary alkyl ammonium); sugars and starches (e.g., maltose, glucose, fructose); sugar and starch derivatives (e.g., glucose alkoxylated glucose, mannitol, xyliyol); DL-panthenol; magnesium ascorbyl phosphate, arbutin, kojic acid, lactamide monoethanolamine; acetamide monoethanolamine; and the like, and mixtures thereof. Humectants also include the $C_3$ to $C_6$ diols and triols, such as glycerin, propylene glycol, butane-1,2,3-triol, hexylene glycol, hexanetriol, and the like, and mixtures thereof. Ethoxylated methyl glucose ethers containing an average of 5 to 30 moles of ethoxylation, such as, for example, those available under the INCI names Lauryl Methyl Gluceth-10 Hydroxypropyldimonium chloride, Methyl Gluceth-10 and Methyl Gluceth-20, are suitable.

Such humectants may be present at from 0.01-20 wt. % of the composition, such as at least 0.1 wt. %, or at least 1 wt. %, e.g., up to 8 wt. %, or up to 5 wt. %.

Sensates

A skin sensate helps provide a sensory confirmation of the adequacy, activity, and evenness of the application thereof by a user. Some non-limiting examples of skin sensates are described in U.S. Pat. Nos. 4,230,688, 4,136,163, 6,183,766 and 7,001,594

Non-limiting examples of suitable sensates include butanedioic acid monomenthyl ester, camphor, carvone, cineole, clove oil, ethyl carboxamide, ethyl menthane carboxamide, eucalyptus oil, eucolytol, ginger oil, l-isopulegol, menthol, menthone glycerin acetal, menthoxy-1,2-propanediol, menthyl lactate, methyl diisopropylpropioniamide, methyl salicylate, peppermint oil, rosemary oil, trimethyl butanamide, vanillyl butyl ether or combinations thereof.

The sensate can be included in the composition in amounts ranging from 0.01 wt. % to 2 wt. % in one aspect, and from 0.05 wt.% to 1 wt.% in another aspect, based on the total weight of the composition.

Botanicals

The compositions of the disclosed technology can contain one or more botanical agents. Suitable botanical agents can include, for example, extracts from Echinacea (e.g., sp. *angustifolia, purpurea, pallida),* yucca glauca, willow herb, basil leaves, Turkish oregano, carrot root, grapefruit, fennel seed, rosemary, tumeric, thyme, blueberry, bell pepper, blackberry, spirulina, black currant fruit, tea leaves, such as for, example, Chinese tea, black tea (e.g., var. Flowery Orange Pekoe, Golden Flowery Orange Pekoe, Fine Tippy Golden Flowery Orange Pekoe), green tea (e.g., var. Japanese, Green Darjeeling), oolong tea, coffee seed, dandelion root, date palm fruit, gingko leaf, green tea, hawthorn berry, licorice, apricot kernel, sage, strawberry, sweet pea, tomato, sunflower seed extract, sandalwood extract, grape seed, aloe leaf, vanilla fruit, comfrey, arnica, *Centella asiatica,* cornflower, horse chestnut, ivy, *Macadamia ternifolia* seed, magnolia, oat, pansy, skullcap, seabuckthorn, white nettle, and witch hazel. Botanical extracts may also include, for example, chlorogenic acid, glutathione, glycrrhizin, neohesperidin, quercetin, rutin, morin, myricetin, absinthe, and chamomile.

In one aspect, the composition can contain from 0.01 to 10 wt. %, or from 0.05 to to 5 wt.%, or from 0.1 wt.% to 3 wt.%, or from 0.5 to 1 wt.% of one or more botanical extracts, based on the total weight of the composition.

Fragrances and Perfumes

Exemplary perfumes, fragrances and fragrance oils include but are not limited to allyl cyclohexane propionate, ambrettolide, Ambrox® DL (dodecahydro-3a,6,6,9a-tetramethylnaphtho[2,1-b]furan), amyl benzoate, amyl cinnamate, amyl cinnamic aldehyde, amyl salicylate, anethol, aurantiol, benzophenone, benzyl butyrate, benzyl iso-valerate, benzyl salicylate, cadinene, campylcyclohexal, cedrol, cedryl acetate, cinnamyl cinnamate, citronellyl acetate, citronellyl isobutyrate, citronellyl propionate, cuminic aldehyde, cyclohexylsalicylate, cyclamen aldehyde, cyclomyral, dihydro isojasmonate, diphenyl methane, diphenyl oxide, dodecanal, dodecalactone, ethylene brassylate, ethylmethyl phenylglycidate, ethyl undecylenate, exaltolide, Galoxilide® (1,3,4,6,7,8-hexhydro,4,6,6,7,8,8-hexamethyl-cyclopenta-γ-2-benzopyran), geranyl acetate, geranyl isobutyrate, hexadecanolide, hexenyl salicylate, hexyl cinnamic aldehyde, hexyl salicylate, α-ionone, β-ionone, γ-ionone, α-irone, isobutyl benzoate, isobutyl quinoline, Iso E Super® (7-acetyl,1,2,3,4,5,6,7,8-octahydro,1,1,6,7-tetramethyl napthalene), cis-jasmone, lilial, linalyl benzoate, 20 methoxy naphthaline, methyl cinnamate, methyl eugenol, γ-methylionone, methyl linolate, methyl linolenate, musk indanone, musk ketone, musk tibetine, myristicin, neryl acetate, δ-nonalactone, γ-nonalactone, patchouli alcohol, phantolide, phenylethyl benzoate, phenylethylphenylacetate, 2-phenylethanol, phenyl heptanol, phenyl hexanol, α-santalol, thibetolide, tonalid, δ-undecalactone, γ-undecalactone, vertenex, vetiveryl acetate, yara-yara, ylangene, allo-ocimene, allyl caproate, allyl heptoate, anisole, camphene, carvacrol, carvone, citral, citronellal, citronellol, citronellyl nitrile, coumarin, cyclohexyl ethylacetate, p-cymene, decanal, dihydromyrcenol, dihydromyrcenyl acetate, dimethyl octanol, ethyllinalool, ethylhexyl

ketone, eucalyptol, fenchyl acetate, geraniol, gernyl formate, hexenyl isobutyrate, hexyl acetate, hexyl neopentanoate, heptanal, isobornyl acetate, isoeugenol, isomenthone, isononyl acetate, isononyl alcohol, isomenthol, isopulegol, limonene, linalool, linalyl acetate, menthyl acetate, methyl chavicol, methyl octyl acetaldehyde, myrcene, napthalene, nerol, neral, nonanal, 2-nonanone, nonyl acetate, octanol, octanal, α-pinene, β-pinene, rose oxide, α-terpinene, γ-terpinene, α-terpinenol, terpinolene, terpinyl acetate, tetrahydrolinalool, tetrahydromyrcenol, undecenal, veratrol, verdox, acetanisol; amyl acetate; anisic aldehyde; anisylalcohol; benzaldehyde; benzyl acetate; benzyl acetone; benzyl alcohol; benzyl formate; hexenol; laevo-carveol; d-carvone; cinnamaldehyde; cinnamic alcohol; cinnamyl acetate; cinnamyl formate; cis-3-hexenyl acetate; Cyclal C (2,4-dimethyl-3-cyclohexen-1-carbaldehyde); dihydroxyindole; dimethyl benzyl carbinol; ethyl acetate; ethyl acetoacetate; ethyl butanoate; ethyl butyrate; ethyl vanillin; tricyclo decenyl propionate; furfural; hexanal; hexenol; hydratropic alcohol; hydroxycitronellal; indole; isoamyl alcohol; isopulegyl acetate; isoquinoline; ligustral; linalool oxide; methyl acetophenone; methyl amyl ketone; methyl anthranilate; methyl benzoate; methyl benzyl acetate; methyl heptenone; methyl heptyl ketone; methyl phenyl carbinyl acetate; methyl salicylate; octalactone; para-cresol; para-methoxy acetophenone; para-methyl acetophenone; phenethylalcohol; phenoxy ethanol; phenyl acetaldehyde; phenyl ethyl acetate; phenyl ethyl alcohol; prenyl acetate; propyl butyrate; safrole; vanillin and viridine.

The amounts of each of the fragrance or perfume components may range from 0.000001 to 2 wt. %, or from 0.00001 to 1.5 wt.%, or from 0.0001 to 1 wt.%, or from 0.001 to 0.8 wt.%, based on of the weight of the composition.

Amino Acids

The hair care composition provided herein can contain one or more non-guanidine moiety containing amino acids. Examples of amino acids that can be used include, without limitation, capryl keratin amino acids, capryl silk amino acids, jojoba amino acids, keratin amino acids, palmitoyl keratin amino acids, palmitoyl silk amino acids, sodium cocoyl amino acids, sodium cocoyl silk amino acids, and sweet almond amino acids.

The amount of amino acid ranges from 0.001 to 5 wt.%, or from 0.01 to 3 wt.%, or from 0.1 to 2 wt.%, or from 0.5 wt.% to 1 wt.%, based on the total weight of the composition.

Antidandruff Agents

The antidandruff agents of the present technology are any particulate compound capable of relieving the symptoms of dandruff and that are substantive to the hair, scalp and skin to afford residual antidandruff properties between shampoos. Among the many particulate compounds exhibiting antidandruff properties that are useful herein are salicylic acid, elemental sulfur, selenium sulfides, azole compounds, 2-pyridone derivatives based on 1-hydroxy-2-pyridone, and polyvalent metal salts of pyrithione.

Sulfur is a particulate antidandruff agent that is effective in the compositions of the disclosed technology. Sulfur can be utilized in an amount ranging from 1 to 5 wt.%, or from 2 to 4 wt.%, based on the weight of the total composition.

Selenium sulfide is a particulate anti-dandruff agent suitable for use in the antidandruff compositions of the present technology and is selected from compounds of the formula $Se_{8-x}S_x$ where x is a number ranging from 1 to 7. Effective concentrations of selenium sulfide can range from 0.1 to 4 wt.%, or from 0.3 to 2.5 wt. %, or from 0.5 to 1.5 wt.%, based on the weight of the composition.

The azole antidandruff agents include imidazoles such as benzimidazole, benzothiazole, bifonazole, butoconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole antidandruff agent can be included in an amount from 0.01 to 5 wt. %, or from 0.1 to 3 wt.%, or from 0.3 to 2 wt.%, based on the weight of the composition.

Exemplary antidandruff agents that are based on 1-hydroxy-2-pyridone are 1-hydroxy-4-methyl-2-pyridone, 1-hydroxy-6-methylpyridone, 1-hydroxy-4,6-dimethyl-2-pyridone, 1-hydroxy-4-methyl-6-(2,4,4-trimethylpentyl)-2-pyridone, 1-hydroxy-4-methyl-6-cyclohexyl-2-pyridone, 1-hydroxy-4-methyl-6-(methyl-cyclohexyl)2-pyridone, 1-hydroxy-4-methyl-6-(2-bicyclo[2,2,1]heptyl)-2-pyridone, 1-hydroxy-4-methyl-6 (4-methylphenyl)-2-pyridone, 1-hydroxy-4-methyl-6 [1-[4-nitrophenoxy]-butyl]-2-pyridone, 1-hydroxy-4-methyl-6-(4-cyanophenoxymethyl-2-pyridone), 1-hydroxy-4-methyl-6-(phenylsulfonylmethyl)-2-pyridone, 1-hydroxy-4-methyl-6-(4-bromobenzyl)-2-pyridone and salts thereof. In one embodiment, the monoethanolamine salt of 1-hydroxy-4-methyl-6-(2,4,4-trimethylpenthyl)-2-pyridone, monoethanolamine salt available from Clariant under the trade name Octopirox® is a suitable antidandruff agent.

The polyvalent metal salts of pyrithione include those formed from the polyvalent metals, e.g., magnesium, barium, bismuth, strontium, copper, zinc, cadmium, zirconium, and mixtures thereof. The polyvalent metal salts of pyrithione can be represented by Formula X as follows:

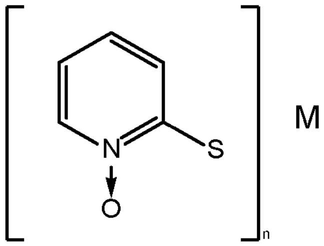

in which M is a polyvalent metal ion selected from magnesium, barium, bismuth, strontium, copper, zinc, cadmium and zirconium, and n corresponds to the valency of M. Any physical form of polyvalent metal pyrithione salts can be used, including platelet and needle configurations.

In one aspect, the polyvalent metal salt of pyrithione is selected from the zinc salt of 1-hydroxy-2-pyridinethione, i.e., the zinc complex of 2-pyridinethiol-1-oxide (known as "zinc pyrithione" or "ZPT") represented by as follows:

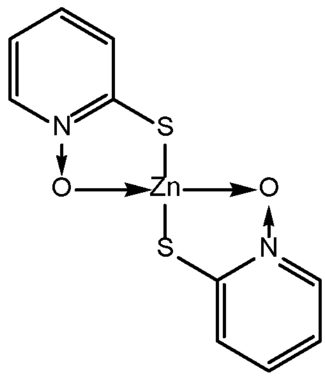

In one aspect the ZPT antidandruff agent has an average particle size of up to 20 μm, or up to 5 μm, or up to 2.5 μm, or up to 1 μm. In an additional aspect, the average particle size can range from 0.1 μm to 1 μm, or from 0.25 μm to 0.75 μm. The average particle size can be measured by light scattering techniques well-known in the art for determining average particle size for particulate materials. One such method involves measuring particle size by means of a laser light scattering technique using a Horiba model LA 910 laser scattering particle size distribution analyzer (Horiba Instruments, Inc., Irvine, California).

Pyridinethione antimicrobial and antidandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982. Zinc pyrithione can be made by reacting 1-hydroxy-2-pyridinethione (i.e., pyrithione acid) or a soluble salt thereof with a zinc salt (e.g., zinc sulfate) to form a zinc pyrithione precipitate, as illustrated in U.S. Pat. No. 2,809,971. Zinc pyrithione is commercially available from Arch Chemicals, Inc. (Lonza Group Ltd.), under the trade name Zinc Ormadine™.

In one aspect, the amount of polyvalent metal salt of pyrithione (e.g., ZPT) suitable for use in the compositions of the present technology range from 0.01 to 5 wt.%, or from 0.1 to 2 wt.%, based on the weight of the composition.

In one aspect of the present technology, the polyvalent metal salt of pyrithione can be used in combination with a secondary particulate zinc salt as disclosed in U.S. Pat. Application Pub. No. 2004/0213751 and U.S. Pat. No. 8,491,877

. It is disclosed that zinc containing layered materials (ZLM) are useful secondary salts that augment the antimicrobial efficacy of polyvalent metal salts of pyrithione, particularly ZPT.

Exemplary ZLM's include, but are not limited to, hydrozincite (zinc carbonate hydroxide), basic zinc carbonate, aurichalcite (zinc copper carbonate hydroxide), rosasite (copper zinc carbonate hydroxide) and many related minerals that are zinc-containing. Natural ZLM's can also occur wherein anionic layer species such as clay-type minerals (e.g., phyllosilicates) contain ion-exchanged zinc gallery ions.

In one aspect, basic zinc carbonate is used in combination with ZPT. Basic zinc carbonate, which also is referred to commercially as "zinc carbonate" or "zinc carbonate basic" or "zinc hydroxy carbonate", is a synthetic version consisting of materials similar to naturally occurring hydrozincite. The idealized stoichiometry is represented as $Zn_5(OH)_6(CO_3)_2$, but the actual stoichiometric ratios can vary slightly, and other impurities may be incorporated in the crystal lattice. Commercially available sources of basic Zinc Carbonate include Zinc Carbonate Basic (Cater Chemicals: Bensenville, IL., USA), Zinc Carbonate Basic (Sigma-Aldrich: St. Louis, MO, USA), Zinc Carbonate (Shepherd Chemicals: Norwood, OH, USA), Zinc Carbonate (CPS Union Corp.: New York, N.Y., USA), Zinc Carbonate (Elementis Pigments: Durham, UK), and Zinc Carbonate AC (Bruggemann Chemical: Newtown Square, PA, USA).

In aspect of the present technology, the ZLM (e.g., basic zinc carbonate) can have a particle size distribution wherein 90% of the particles are less than 50 μm. In another aspect, the ZLM can have a particle size distribution wherein 90% of the particles are less than 30 μm. In yet a further aspect, the ZLM can have a particle size distribution wherein 90% of the particles are less than 20 μm.

In another aspect of the present technology, the ZLM (e.g., basic zinc carbonate) can have a surface area of greater than 10 $m^2$/gm. In a further aspect, the ZLM can have a surface area of greater than 20 $m^2$/gm. In yet a further aspect of the ZLM can have a surface area of greater than 30 $m^2$/gm.

In aspects utilizing a ZLM and a polyvalent metal salt of pyrithione (e.g., ZPT), the ratio of ZLM to polyvalent metal salt of pyrithione is from 5:100 to 10:1 in one aspect, from 2:10 to 5:1 in another aspect, and from 1:2 to 3:1 in still another aspect (all ratios based on a wt./wt. basis).

In one aspect of the present technology, the polyvalent metal salt of pyrithione can be used in combination with a metal ion source such as copper and zinc salts, as disclosed in International Pat. Application Pub. No. WO 01/00151 .

It is disclosed that antidandruff efficacy can be dramatically increased in topical compositions using polyvalent metal salts of pyrithione, such as ZPT, in combination with a metal ion source such as copper and zinc salts. The metal ion source may be selected from zinc, copper, silver, nickel, cadmium, mercury, and bismuth. In one aspect, the metal ion is selected from zinc salts, copper salts, silver salts, and mixtures thereof.

In one aspect, the metal ion is selected from zinc salts, copper salts, and mixtures thereof. Exemplary metal ion salts of zinc and copper include, but are not limited to, zinc acetate, zinc oxide, zinc carbonate, zinc hydroxide, zinc chloride, zinc sulfate, zinc citrate, zinc fluoride, zinc iodide, zinc lactate, zinc oleate, zinc oxalate, zinc phosphate, zinc propionate, zinc salicylate, zinc selenate, zinc silicate, zinc stearate, zinc sulfide, zinc tannate, zinc tartrate, zinc valerate, zinc gluconate, zinc undecylate, and the like. Combinations of zinc salts may also be used in the composition of the disclosed technology. Exemplary metal ion salts of copper include, but are not limited to, copper disodium citrate, copper triethanolamine, copper carbonate, cuprous ammonium carbonate, cupric hydroxide, copper chloride, cupric chloride, copper ethylenediamine complex, copper oxychloride, copper oxychloride sulfate, cuprous oxide, copper thiocyanate, and the like. Combinations of these copper salts may also be used in the composition of the disclosed technology.

The metal ion source is present in the composition in a ratio (wt./wt.) to polyvalent metal salt of pyrithione of from 5: 100 to 5: 1 in one aspect, from 2: 10 to 3: 1 in another aspect, and from 1: 2 to 2: 1 in still another aspect.

Pharmaceutical and Cosmeceutical Actives

The compositions of the present technology can be formulated with a pharmaceutical and/or a cosmeceutical active to deliver a desired effect. Examples of such active ingredients include, but are not limited to, caffeine, vitamin C, vitamin D, vitamin E, anti-stretch mark compounds, astringents (e.g., alum, oatmeal, yarrow, witch hazel, bayberry, and isopropyl alcohol), draining compounds, depilatories (e.g., calcium and sodium hydroxide, calcium or sodium thioglycolate, or mixtures thereof), hair growth promoting compounds (e.g., monoxidil), skin and hair nourishing compounds, skin and hair protecting compounds, self-tanning compounds (e.g., mono- or polycarbonyl compounds such as, for example, isatin, alloxan, ninhydrin, glyceraldehyde, mesotartaric aldehyde, glutaraldehyde, erythrulose, tyrosine, tyrosine esters, and dihydroxyacetone), UV absorbers (e.g., ethylhexyl methoxy cinnamate, octinoxate, octisalate, oxybenzone), skin lighteners (e.g., kojic acid, hydroquinone, arbutin, fruital, vegetal or plant extracts, such as lemon peel extract, chamomile, green tea, paper mulberry extract, and the like, ascorbyl acid derivatives, such as ascorbyl palmitate, ascorbyl stearate, magnesium ascorbyl phosphate, and the like), lip plumping compounds, anti-aging, anti-cellulite, and anti-acne compounds (e.g., acidic agents such as alpha-hydroxy acids (AHAs), beta-hydroxy acids (BHAs), alpha amino-acids, alpha-keto acids (AKAs), acetic acid, azelaic acid, and mixtures thereof). In one aspect, AHAs can include, but are not limited to, lactic acid, glycolic acid, fruit acids, such as malic acid, citric acid, tartaric acid, extracts of natural compounds containing AHA, such as apple extract, apricot extract, and the like, honey extract, 2-hydroxyoctanoic acid, glyceric acid (dihydroxypropionic acid), tartronic acid (hydroxypropanedioic acid), gluconic acid, mandelic acid, benzilic acid, azelaic acid, alpha-lipoic acid, salicylic acid, AHA salts and derivatives, such as arginine glycolate, ammonium glycolate, sodium glycolate, arginine lactate, ammonium lactate, sodium lactate, alpha-hydroxybutyric acid, alpha-hydroxyisobutyric acid, alpha-hydroxyisocaproic acid, alpha-hydroxyisovaleric acid, atrolactic acid, and the like. BHAs can include, but are not limited to, 3-hydroxy propanoic acid, beta-hydroxybutyric acid, beta-phenyl lactic acid, beta-phenylpyruvic acid, and the like. Alpha-amino acids include, without being limited thereto, alpha-amino dicarboxylic acids, such as aspartic acid, glutamic acid, and mixtures thereof, sometimes employed in combination with fruit acid. AKAs include pyruvic acid. In some antiaging compositions, the acidic active agent may be retinoic acid, a halocarboxylic acid, such as trichloroacetic acid, an acidic antioxidant, such as ascorbic acid (vitamin C), a mineral acid, phytic acid, lysophosphatidic acid, and the like. Some acidic anti-acne actives, for example, can include salicylic acid, derivatives of salicylic acid, such as 5-octanoylsalicylic acid, retinoic acid, and its derivatives, and benzoic acid, anti-inflammatory compounds (e.g., aspirin, ibuprofen, and naproxen), analgesics (e.g., acetaminophen), antioxidant compounds, antiperspirant compounds (e.g., aluminum halides, aluminum hydroxyhalides, aluminum sulfate, zirconium (zirconyl) oxyhalides, zirconium (zirconyl) hydroxyhalides, and mixtures or

complexes thereof), deodorant compounds (e.g., 2-amino-2-methyl-1-propanol (AMP), ammonium phenolsulfonate; benzalkonium chloride; benzethonium chloride, bromochlorophene, cetyltrimethylammonium bromide, cetyl pyridinium chloride, chlorophyllin-copper complex, chlorothymol, chloroxylenol, cloflucarban, dequalinium chloride, dichlorophene, dichloro-m-xylenol, disodium dihydroxyethyl sulfosuccinylundecylenate, domiphen bromide, hexachlorophene, lauryl pyridinium chloride, methylbenzethonium chloride, phenol, sodium bicarbonate, sodium phenolsulfonate, triclocarban, triclosan, zinc phenolsulfonate, zinc ricinoleate, and mixtures thereof); and suitable mixtures of any of the above.

A discussion of the use and formulation of active skin treatment compositions is in COSMETICS & TOILETRIES, C&T Ingredient Resource Series, "AHAs & Cellulite Products How They Work", published 1995, and "Cosmeceuticals", published 1998, both available from Allured Publishing Corporation, incorporated herein by reference.

In one aspect, the amount of pharmaceutical and cosmeceutical active can range from 0.0001 to 5 wt. %, or from 0.001 to 3 wt.%, or from 0.01 to 1.5 wt.%, or from 0. 1 to 0.1 wt.%, based on the weight of the total composition.

Electrolytes

Optionally, the cleansing and conditioning compositions of the disclosed technology can contain an electrolyte. Suitable electrolytes are known compounds and include salts of multivalent anions, such as potassium pyrophosphate, potassium tripolyphosphate, and sodium or potassium citrate, salts of multivalent cations, including alkaline earth metal salts such as calcium chloride and calcium bromide, as well as zinc halides, barium chloride, magnesium sulfate and calcium nitrate, salts of monovalent cations with monovalent anions, including alkali metal or ammonium halides, such as potassium chloride, sodium chloride, potassium iodide, sodium bromide, and ammonium bromide, alkali metal or ammonium nitrates, and blends thereof.

The amount of the electrolyte used will generally depend on the amount of the emulsion polymer incorporated but may be used at concentration levels of from 0.1 to 4 wt.%, or from 0.2 to 2 wt., based on the weight of the total composition.

Hair Coloring Dyes and Pigments

The compositions of the present technology may also contain oxidative dye precursors, dye couplers, direct dyes, dyes, pigments, and mixtures thereof, which encompass permanent, semi-permanent and temporary dyes commonly known in the art. Hair coloring agents are disclosed in the CTFA International Color Handbook, 2nd Edition, Micelle Press, England (1992) and Cosmetic Handbook, US Food and Drug Administration, FDA/IAS Booklet (1992), the contents of which are hereby incorporated by reference.

Exemplary oxidative dye precursors include but are not limited to 4-amino-m-cresol, 1-hydroxyethyl-4-5-diaminophyrazole sulfate, N,N, bis [2-hydroxyethyl]-p-phenylene-di-amine sulfate, hydroxyethyl-p-phenylene-diamine sulfate, p-aminophenol, m-aminophenol, 3-methyl-4-aminophenol, p-methyl amino phenol sulphate, p-phenylenediamine sulfate, tetraamino pyrimidine sulfate, toluene-2-5-diamine sulfate, and mixtures thereof.

Exemplary dye couplers include but are not limited to resorcinol, 2-amino-3-hydroxypyridine, 4-amino-2-hydroxytoluene, 2-methyl-5-hydroxyethylaminophenol, 2-methyl resorcinol, 5-amino-6-chloro-o-cresol, 2-amino-4-hydroxyethylamino anisole sulfate, 1-napthol, 2,4-diaminophenoxy ethanol sulfate, m-amino phenol, phenyl methyl pyrazolone, 4-chloro resorcinol, and mixtures thereof.

As is well-known in the hair art, oxidative dye precursors and dye couplers are formulated in as two component system. Each component is packaged separately and mixed together immediately prior to use. A first component comprising the oxidative dye precursor, the dye coupling agent, and an alkalizing agent (e.g., ammonia) is packaged separately from a second component comprising an oxidizing agent (e.g., hydrogen peroxide). The first and second components are combined just prior to use and applied to the hair.

Exemplary direct dyes include 4-[4'-aminophenyl)-(4"-imino-2",5"-cyclohexadien-1"-yliden) methyl]-2-methylaminobenzene monohydrochloride (C.I. 42 510), 4-[4'-amino-3'-methylphenyl)-(4"-imino-3"-methyl-2",5"-cyclohexadien-1"-yliden)methyl]-2-methylaminobenzene monohydrochloride (C.I. 42 520), 4-hydroxypropylamino-3-nitrophenol, 4-amino-3-nitrophenol, 2-amino-6-chloro-4-nitrophenol, HC Blue 2, HC Yellow 4, HC Red 3, Disperse Violet 4, Disperse Black 9, HC Blue 7, HC Blue 12, HC Yellow 2, HC Yellow 12, Disperse Blue 3, Disperse violet 1, and mixtures thereof.

Suitable semi-permanent dyes can be selected from basic dyes, HC dyes, acid dyes, direct dyes, disperse dyes, and mixtures thereof.

Suitable semi-permanent dyes can be selected from basic dyes, HC dyes, acid dyes, direct dyes, disperse dyes, and mixtures thereof.

Suitable disperse dyes include Disperse Black 9, Disperse Blue 1, 3, and 7; Disperse Brown 1, Disperse Orange 3, Disperse Red 11, 15, and 17; and Disperse Violet 1, 4, and 15.

The temporary hair coloring agent may be any dye commonly used in temporary dyeing products. In one aspect, the temporary coloring agent is selected from at least one pigment. As used herein, the term "pigment" means any pigment that imparts a color to the hair.

The at least one pigment that may be used can be chosen from the organic and/or mineral pigments known in the art, such as those described in Kirk-Othmer's Encyclopedia of Chemical Technology and in Ullmann's Encyclopedia of Industrial Chemistry.

The at least one pigment may be in the form of powder or of pigmentary paste. The pigment may be coated or uncoated.

The at least one pigment may be chosen, for example, from mineral pigments, organic pigments, lakes, pigments with special effects such as nacres or glitter flakes, and mixtures thereof.

The at least one pigment may be a mineral pigment. As used herein, the term "mineral pigment" means any pigment that satisfies the definition in Ullmann's encyclopedia in the chapter on inorganic pigments. The mineral pigments that may be useful in the present disclosure include iron oxides, chromium oxides, manganese violet, ultramarine blue, chromium hydrate, and ferric blue.

Examples of colored mineral pigments include red pigments of red oxide, iron oxide, iron hydroxide, and iron titanate, inorganic brown pigments such as γ-iron oxide, inorganic yellow pigments such as yellow iron oxide and yellow soil, inorganic black pigments such as black iron oxide and carbon black, inorganic violet pigments such as manganese violet and cobalt violet, inorganic green pigments such as chromium hydroxide, chromium oxide, cobalt oxide, and cobalt titanate, inorganic blue pigments such as iron blue and ultramarine blue, pigments obtained by flaking tar-based dyes such as red No. 3, red No. 104, red No. 106, red No. 201, red No. 202, red No. 204, red No. 205, red No. 220, red No. 226, red No. 227, red No. 228, red No. 230, red No. 401, red No. 505, yellow No. 4, yellow No. 5, yellow No. 202, yellow No. 203, yellow No. 204, yellow No. 401, blue No. 1, blue No. 2, blue No. 201, blue No. 404, green No. 3, green No. 201, green No. 204, green No. 205, orange No. 201, orange No. 203, orange No. 204, orange No. 206, and orange No. 207, ones obtained by flaking natural pigments such as carminic acid, laccaic acid, carthamin, and brazilin, and the like, titanium oxide-coated mica, titanated mica, iron oxide-treated titanated mica, titanium oxide-coated mica, bismuth oxychloride, titanium oxide-coated bismuth oxychloride, titanium oxide-coated talc, titanium oxide-coated colored mica, and metal powder pigments such as aluminum, gold, silver, copper, platinum, and stainless steel; and mixtures thereof.

The at least one pigment may be an organic pigment. As used herein, the term "organic pigment" means any pigment that satisfies the definition in Ullmann's encyclopedia in the chapter on organic pigments. For instance, the at least one organic pigment may be chosen from nitroso, nitro, azo, xanthene, quinoline, anthraquinone, phthalocyanin, metal-complex, isoindolinone, isoindoline, quinacridone, perinone, perylene, diketopyrrolopyrrole, thioindigo, dioxazine, triphenylmethane, and quinophthalone compounds.

White or colored organic pigments may be chosen from carmine, carbon black, aniline black, melanin, azo yellow, quinacridone, phthalocyanin blue, sorghum red, the blue pigments codified in the Color Index under the references CI 42090, 69800, 69825, 73000, 74100, and 74160, the yellow pigments codified in the Color Index under the references CI 11680, 11710, 15985, 19140, 20040, 21100, 21108, 47000, and 47005, the green pigments codified in the Color Index under the references CI 61565, 61570, and 74260, the orange pigments codified in the Color Index under the references CI 11725, 15510, 45370, and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915, and 75470, and the pigments obtained by oxidative polymerization of indole or phenolic derivatives as described in French Patent Publication No. FR 2 679 771.

Exemplary pigmentary pastes of organic pigments include Pigment Yellow 3 (CI 11710), Pigment Yellow 1 (CI 11680), Pigment Orange 43 (CI 71105), Pigment Red 4 (CI 12085), Pigment Red 5 (CI 12490), Pigment Violet 23 (CI 51319), Pigment Blue 15.1 (CI 74160), Pigment Green 7 (CI 74260), Pigment Black 7 (CI 77266), and mixtures thereof.

The at least one pigment in accordance with the present technology may also be in the form of at least one composite pigment as described in European Patent Publication No. EP 1 184 426. These composite pigments may be, for example, compounds of particles comprising a mineral core, at least one binder for ensuring the binding of the organic pigments to the core, and at least one organic pigment at least partially covering the core.

The organic pigment may also be a lake. As used herein, the term "lake" means dyes adsorbed onto insoluble particles, the assembly thus obtained remaining insoluble during use. The insoluble particles onto which the dyes are adsorbed can be, for example, alumina, silica, calcium sodium borosilicate, calcium aluminum borosilicate, and aluminum. A non-limiting example of a lake is D&C Red 7 (CI 15 850:1).

Exemplary dyes include cochineal carmine, D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), and D&C Blue 1 (CI 42 090).

The at least one pigment may also be a pigment with special effects. As used herein, the term "pigments with special effects" means pigments that generally create a non-uniform colored appearance (characterized by a certain shade, a certain vivacity, and a certain lightness) that changes as a function of the conditions of observation (light, temperature, observation angles, etc.). They thus contrast with white or colored pigments that afford a standard uniform opaque, semi-transparent, or transparent shade. Several types of pigments with special effects exist those with a low

refractive index, such as fluorescent, photochromic, or thermochromic pigments, and those with a high refractive index, such as nacres or glitter flakes.

Examples of pigments that impart special effects include nacreous pigments such as mica/red iron oxide, mica coated with titanium or with bismuth oxychloride, colored nacreous pigments such as titanium mica with iron oxides, titanium mica with ferric blue or with chromium oxide, titanium mica with an organic pigment as set forth above, and nacreous pigments based on bismuth oxychloride. Exemplary nacreous pigments include mica-$TiO_2$-lake, mica-$TiO_2$, mica-$Fe_2O_3$, and mica-$TiO_2$-$Fe_2O_3$.

In addition to nacres on a mica support, multilayer pigments based on synthetic substrates such as alumina, silica, sodium calcium borosilicate, calcium aluminum borosilicate, and aluminum, may be utilized.

Pigments with an interference effect that are not fixed onto a substrate, for instance, liquid crystals (Helicones™ HC from Material District) and holographic interference flakes. Pigments with special effects also include fluorescent pigments, phosphorescent pigments, photochromic pigments, thermochromic pigments, and quantum dots.

The hair coloring dyes and pigments can be present in an amount ranging from 0.0005 to 20 wt.%, or from 0.005 to 10 wt.%, or from 0.05 to 6 wt.%, based on the total weight of the composition.

Auxiliary Fixatives

While the ASE and HASE polymers of the present technology possess film forming properties suitable for use in hair fixative compositions, auxiliary hair fixative polymers may be used therewith. Exemplary auxiliary hair fixative polymers include natural and synthetic polymers such as, for example, polyacrylates, polyvinyls, polyesters, polyurethanes, polyamides, modified cellulose, starches, and mixtures thereof. These polymers can be nonionic, anionic, cationic and amphoteric in nature and include without limitation one or more of polyoxyethylenated vinyl acetate/crotonic acid copolymers, vinyl acetate crotonic acid copolymers, vinyl methacrylate copolymers, monoalkyl esters of poly(methyl vinyl ether (PVM)/maleic anhydride (MA)), such as, for example, ethyl, butyl and isopropyl esters of PVM/MA copolymer acrylic acid/ethyl acrylate/N-tert-butyl-acrylamide terpolymers, and poly (methacrylic acid/acrylamidomethyl propane sulfonic acid), acrylates copolymer, octylacrylamide/acrylates/butylaminoethyl methacrylate copolymer, acrylates/octylacrylamide copolymer, vinyl acetate (VA)/crotonates/vinyl neodeanoate copolymer, poly(N-vinyl acetamide), poly(N-vinyl formamide), corn starch modified, sodium polystyrene sulfonate, polyquaterniums such as, for example, Polyquaternium-4, Polyquaternium-5, Polyquaternium-6, Polyquaternium-7, Polyquaternium-10, Polyquaternium-11, Polyquaternium-22, Polyquaternium-24, Polyquaternium-28, Polyquaternium-29, Polyquaternium-32, Polyquaternium-34, Polyquaternium-37, Polyquaternium-39, Polyquaternium-44, Polyquaternium-46, Polyquaternium-47, Polyquaternium-52, Polyquaternium-53, Polyquarternium-55, Polyquaternium-68, Polyquaternium-69, Polyquaternium-87, Laureth-16, polyether-1, VA/acrylates/lauryl methacrylate copolymer, adipic acid/dimethylaminohydroxypropyl diethylene AMP/acrylates copolymer, methacrylol ethyl betaine/acrylates copolymer, acrylamide/sodium acryloyldimethyltaurate/acrylic acid, polyvinylpyrrolidone (PVP), vinyl pyrrolidone (VP)/dimethylaminoethylmethacrylate copolymer, acrylic acid/VP crosspolymer, VP/methacrylamide/vinyl imidazole copolymer, VP/dimethylaminopropylamine (DMAPA) acrylates copolymer, VP/vinylcaprolactam/DMAPA acrylates copolymer, vinyl caprolactam/VP/dimethylaminoethyl methacrylate copolymer, VA/butyl maleate/isobornyl acrylate copolymer, VA/crotonates copolymer, acrylate/acrylamide copolymer, VA/crotonates/vinyl propionate copolymer, VP/vinyl acetate/vinyl propionate terpolymers, VP/vinyl acetate copolymer, VP/acrylates copolymer, VA/crotonic acid/vinyl proprionate, acrylates/acrylamide, acrylates/octylacrylamide, acrylates/hydroxyacrylates copolymer, acrylates/hydroxyesteracrylates copolymer, acrylates/stereth-20 methacrylate copolymer, tert-butyl acrylate/acrylic acid copolymer, diglycol/cyclohexanedimethanol/isophthalates/sulfoisophthalates copolymer, VA/alkylmaleate half ester/N-substituted acrylamide terpolymers, vinyl caprolactam/VP/ methacryloamidopropyl trimethylammonium chloride terpolymer, methacrylates/acrylates copolymer/amine salt, polyvinylcaprolactam, hydroxypropyl guar, poly (methacrylic acid/acrylamidomethyl propane sulfonic acid (AMPSA), ethylenecarboxamide (EC)/AMPSA/methacrylic acid (MAA), poylurethane/acrylate copolymers and hydroxypropyl trimmonium chloride guar, acrylates crosspolymer, acrylates crosspolymer-3, AMP-acrylates/allyl methacrylate copolymer, polyacrylate-14, polyacrylate-2 crosspolymer, acrylates/lauryl acrylate/stearyl acrylate/ethylamine oxide methacrylate copolymer, methacryloyl ethyl betaines/methacrylates copolymer, polyurethane/acrylates copolymer, pyrrolidone carboxylic acid salt of chitosan, chitosan glycolate, cationic polygalactomannans, such as, for example, quaternized derivatives of guar, such as, for example, guar hydroxypropyl trimmonium chloride, cassia hydroxypropyl trimmonium chloride and hydroxypropyl guar hydroxypropyl trimmonium chloride. Many of the foregoing polymers are referred to by their INCI nomenclature set forth in the *International Cosmetic Ingredient Dictionary* published by the Cosmetic, Toiletry, and Fragrance Association, Washington D.C. Other suitable auxiliary fixative polymers are disclosed in U.S. Patent No. 7,205,271

The auxiliary fixative polymer typically comprises from 0.01 to 8 wt.%, or from 0.1 to 5 wt.%, or from 0.2 to 3 wt.%, based on the total weight of the composition.

Opacifying/Pearlescent Materials

Some formulations are often opacified by deliberately incorporating pearlescent materials therein to achieve a cosmetically attractive pearl-like appearance, known as pearlescence. An opacifier often is included in a composition to mask an undesirable aesthetic property, such as to improve the color of a composition that is darkened due to the presence of a particular ingredient, or to mask the presence of particulates in the composition. Opacifiers also are included in aqueous compositions to improve the aesthetics and consumer acceptance of an otherwise esthetically unpleasing composition. For example, an opacifier can impart a pearlescent appearance to a clear composition, thereby communicating an appearance of creaminess, mildness, and body to the consumer. Persons skilled in the art are aware of problems faced by formulators in consistently preparing a stable pearlescent formulation. A detailed discussion is found in the article "Opacifiers and pearling agents in shampoos" by Hunting, Cosmetic and Toiletries, Vol. 96, pages 65-78 (July 1981), and a representative listing of opacifiers is found in the CTFA Cosmetic Ingredient Handbook, J. Nikitakis, ed., The Cosmetic, Toiletry and Fragrance Association, Inc., Washington, D.C., 1988, at page 75

The opacifying or pearlescent material includes ethylene glycol monostearate, ethylene glycol distearate, polyethylene glycol distearate, stearic alcohol, bismuth oxychloride coated mica, mica coated metal oxides (e.g., titanium dioxide, chromium oxide, iron oxides), myristyl myristate, guanine, glitter (polyester or metallic), and mixtures thereof. Other pearlescent materials can be found in U.S. Patent No. 4,654,207, U.S. Patent No. 5,019,376, and U.S. Patent No. 5,384,114

In one aspect, the amount of the pearlescent material can be used in amounts ranging from 0.05% to 10% by weight, and from 0.1% to 3% by weight in another aspect, based upon the total weight of the stabilized composition.

Particulates

Numerous other substantially insoluble compounds and components which require stabilization and/or suspension can be utilized in the compositions of the present technology. Examples of such other insoluble compounds include pigments and exfoliants.

Exemplary pigments are metal compounds or semi-metallic compounds and may be used in ionic, nonionic or oxidized form. The pigments can be in this form either individually or in admixture or as individual mixed oxides or mixtures thereof, including mixtures of mixed oxides and pure oxides. Examples are the titanium oxides (e.g., $TiO_2$), zinc oxides (e.g., ZnO), aluminum oxides (for example, $Al_2O_3$), iron oxides (for example, $Fe_2O_3$), manganese oxides (e.g., MnO), silicon oxides (e.g., $SiO_2$), silicates, cerium oxide, zirconium oxides (e.g., $ZrO_2$), barium sulfate ($BaSO_4$), and mixtures thereof.

Other examples of pigments include D&C Red No. 30, D&C Red No. 36, D&C Orange No. 17, Green 3 Lake, Ext. Yellow 7 Lake, Orange 4 Lake, Red 28 Lake, the calcium lakes of D&C Red Nos. 7, 11, 31 and 34, the barium lake of D&C Red No. 12, the strontium lake D&C Red No. 13, the aluminum lakes of FD&C Yellow No. 5 and No. 6, the aluminum lakes of FD&C No. 40, the aluminum lakes of D&C Red Nos. 21, 22, 27, and 28, the aluminum lakes of FD&C Blue No. 1, the aluminum lakes of D&C Orange No. 5, the aluminum lakes of D&C Yellow No. 10; the zirconium lake of D&C Red No. 33, iron oxides, thermochromic dyes that change color with temperature, calcium carbonate, aluminum hydroxide, calcium sulfate, kaolin, ferric ammonium ferrocyanide, magnesium carbonate, carmine, barium sulfate, mica, bismuth oxychloride, zinc stearate, manganese violet, chromium oxide, titanium dioxide nanoparticles, barium oxide, ultramarine blue, bismuth citrate, hydroxyapatite, zirconium silicate, carbon black particles and the like. Other suitable particulates include various optical modifiers as described in US 7,202,199.

Numerous cosmetically useful particulate exfoliating agents are known in the art, and the selection and amount are determined by the exfoliating effect desired from the use of the composition, as recognized by those skilled in the cosmetic arts. Useful exfoliating agents include, but are not limited to, natural abrasives, inorganic abrasives, synthetic polymers, and the like, and mixtures thereof. Representative exfoliants include, but are not limited to, ground or powdered pumice, stone, zeolites, nut shells (e.g., almond, pecan, walnut, coconut, and the like), nut meals (e.g., almond, and the like), fruit pits (e.g., apricot, avocado, olive, peach, and the like), hulls, seed and kernel (e.g., oat bran, corn meal, rice bran, grape seed, kiwi seed, wheat, jojoba seed, loofah seed, rose hip seed, and the like), plant matter (e.g., tea tree leaves, corn cob, fruit fibers, seaweed, loofah sponge, microcrystalline cellulose, and the like), bivalve shells (oyster shell, and the like), calcium carbonate, dicalcium pyrophosphate, chalk, silica, kaolin clay, silicic acid, aluminum oxide, stannic oxide, sea salt (e.g., Dead Sea salt), talc, sugars (e.g., table, brown, and the like), polyethylene, polystyrene, microcrystalline polyamides (nylons), microcrystalline polyesters, polycarbonates, and stainless steel fibers. The foregoing exfoliants can be used in the form of granules, powders, flours, and fibers.

Other generally insoluble components suitable for use in the present compositions include clay, swellable clay, laponite, gas bubbles, liposomes, microsponges, cosmetic beads and flakes. Cosmetic beads, flakes and capsules can be included in a composition for aesthetic appearance or can function as micro- and macro-encapsulants for the delivery of benefit agents to the skin and hair. Exemplary bead components include, but are not limited to, agar beads, alginate beads,

jojoba beads, gelatin beads, Styrofoam ™ beads, polyacrylate, polymethylmethacrylate (PMMA), polyethylene beads, Unispheres™ and Unipearls™ cosmetic beads (Induchem USA, Inc., New York, NY), Lipocapsule™ Liposphere™, and Lipopearl™ microcapsules (Lipo Technologies Inc., Vandalia, OH), and Confetti II™ dermal delivery flakes (United-Guardian, Inc., Hauppauge, NY).

In one aspect of the present technology, the amount of particulate or insoluble component can range from 0.1% to 10% by weight, based on the total weight of the composition.

Propellants

Where desired, any known aerosol propellant can be utilized to deliver the personal care, home care, health care, and institutional care compositions containing the ASE and HASE polymers of the present technology in combination with one or more of the foregoing active ingredients and/or with the one or more additives and/or adjuvants, conventionally or popularly included in such products. Exemplary propellants include, but are not limited to, lower boiling hydrocarbons such as $C_3$-$C_6$ straight and branched chain hydrocarbons. Exemplary hydrocarbon propellants include propane, butane, isobutene, and mixtures thereof. Other suitable propellants include ethers, such as, dimethyl ether, hydrofluorocarbons, such as, 1,1-difluoroethane, and compressed gasses, such as air and carbon dioxide.

In one aspect, these compositions can contain from 0.1% to about 60% by weight of a propellant, and from 0.5 to 35% by weight in another aspect, based on the total weight of the composition.

Preservatives

In one aspect, any preservative suitable for use in personal care can be used in the composition for straightening hair. Suitable preservatives include polymethoxy bicyclic oxazolidine, methyl paraben, propyl paraben, ethyl paraben, butyl paraben, benzyltriazole, DMDM hydantoin (also known as 1,3-dimethyl-5,5-dimethyl hydantoin), imidazolidinyl urea, phenoxyethanol, phenoxyethylparaben, methylisothiazolinone, methylchloroisothiazolinone, benzoisothiazolinone, triclosan, and suitable polyquaternium compounds as disclosed above (e.g., Polyquaternium-1).

In another aspect, acid-based preservatives are useful in the exemplary compositions. The use of acid-based preservatives facilitates the formulation of products in the low pH range. Lowering the pH of a formulation inherently provides an inhospitable environment for microbial growth in addition to being suited to the straightening process. Moreover, formulating at low pH enhances the efficacy of acid-based preservatives, and affords a personal care product which maintains an acidic pH balance on the skin. Any acid-based preservative that is useful in personal care products can be used in the exemplary compositions. In one aspect the acid preservative is a carboxylic acid compound represented by the formula: $R^{80}C(O)OH$, wherein $R^{80}$ represents hydrogen, a saturated and unsaturated hydrocarbyl group containing 1 to 8 carbon atoms or $C_6$ to $C_{10}$ aryl. In another aspect, $R^{80}$ is selected from a hydrogen, a $C_1$ to $C_8$ alkyl group, a $C_2$ to $C_8$ alkenyl group, or phenyl. Exemplary acids are, but are not limited to, formic acid, acetic acid, propionic acid, sorbic acid, caprylic acid, and benzoic acid, and mixtures thereof.

In another aspect, suitable acids include but are not limited to, oxalic acid, succinic acid, glutaric acid, adipic acid, azelaic acid, maleic acid, fumaric acid, lactic acid, glyceric acid, tartronic acid malic acid, tartaric acid, gluconic acid, citric acid, ascorbic acid, salicylic acid, phthalic acid, mandelic acid, benzilic acid, and mixtures thereof.

Salts of the foregoing acids are also useful as long as they retain efficacy at low pH values. Suitable salts include the alkali metal (e.g., sodium, potassium, calcium) and ammonium salts of the acids enumerated above.

The acid-based preservatives and/or their salts can be used alone or in combination with non-acidic preservatives typically employed in personal care, home care, health care, and institutional and industrial care products.

The preservatives may comprise from 0.01 to 3.0 wt.%, or from 0.1 to 1 wt.%, or from 0.3 to 1 wt.%, based on the weight of the total composition.

Auxiliary Solvents and Diluents

The personal care, home care, health care, and institutional care compositions containing the thickened surfactant compositions of the present technology in combination with one or more of the foregoing active ingredients and/or with the one or more additives and/or adjuvants, conventionally or popularly included in personal care, health care, home care, and institutional care products discussed above can be prepared as water-free or water-based formulations, and formulations containing water-miscible auxiliary solvents and/or diluents, but are not limited thereto. The ASE and HASE polymers of the present technology are particularly useful for water-based, solvent based, hydroalcoholic based, and mixed solvent formulations, and for formulations containing water-miscible auxiliary solvents. Useful solvents commonly employed are typically liquids, such as water (deionized, distilled or purified), alcohols, fatty alcohols, polyols, and the like, and mixtures thereof. Non-aqueous or hydrophobic auxiliary solvents are commonly employed in substantially water-free products, such as nail lacquers, aerosol propellant sprays, or for specific functions, such as removal of oily

soils, sebum, make-up, or for dissolving dyes, fragrances, and the like, or are incorporated in the oily phase of an emulsion. Non-limiting examples of auxiliary solvents, other than water, include linear and branched alcohols, such as ethanol, propanol, isopropanol, hexanol, and the like; aromatic alcohols, such as benzyl alcohol, cyclohexanol, and the like; saturated $C_{12}$ to $C_{30}$ fatty alcohol, such as lauryl alcohol, myristyl alcohol, cetyl alcohol, stearyl alcohol, behenyl alcohol, and the like. Non-limiting examples of polyols include polyhydroxy alcohols, such as glycerin, propylene glycol, butylene glycol, hexylene glycol, $C_2$ to $C_4$ alkoxylated alcohols and $C_2$ to $C_4$ alkoxylated polyols, such as ethoxylated, propoxylated, and butoxylated ethers of alcohols, diols, and polyols having about 2 to about 30 carbon atoms and 1 to about 40 alkoxy units, polypropylene glycol, polybutylene glycol, and the like. Non-limiting examples of non-aqueous auxiliary solvents or diluents include silicones, and silicone derivatives, such as cyclomethicone, and the like, ketones such as acetone and methylethyl ketone; natural and synthetic oils and waxes, such as vegetable oils, plant oils, animal oils, essential oils, mineral oils, $C_7$ to $C_{40}$ isoparaffins, alkyl carboxylic esters, such as ethyl acetate, amyl acetate, ethyl lactate, and the like, jojoba oil, shark liver oil, and the like. Some of the foregoing non-aqueous auxiliary solvents or diluents may also be conditioners and emulsifiers.

## Exemplary Compositions

### Body Wash

In one aspect, a personal care composition in which the polymer of the present technology is useful is a body wash. Typical components of a body wash, in addition to the polymer thickener and water are: at least one surfactant; a sufficient pH adjusting agent (base and/or acid) to attain a pH of from 4 to 7.5, or from 4.5 to 6.5, or from 5.0 to 6.0; and optional ingredients selected from the adjuvants, additives and benefit agents discussed above, and mixtures thereof, including benefit agents selected from silicones, pearlizing agents, vitamins, oils, fragrances, dyes, preservatives, botanicals, exfoliating agents, insoluble gas bubbles, liposomes, microsponges, cosmetic beads and flakes. In one aspect, the surfactant is an anionic surfactant. In another aspect, the surfactant is a mixture of an anionic surfactant and an amphoteric surfactant, in optional combination with a non-ionic surfactant. The anionic surfactant can be sulfated or sulfate-free. In another aspect, the surfactant is a mixture of an anionic surfactant and an amphoteric surfactant, in optional combination with a cationic and/or a non-ionic surfactant. In one aspect, the anionic sulfated or sulfate-free surfactant can be present in an amount ranging from 5 to 40 wt.%, or from 6 to 30 wt.%, or from 8 to 25 wt.%, based on the total weight of the body wash composition. When mixtures of sulfated or sulfate-free anionic and amphoteric surfactants are used, the weight ratio of sulfated or sulfate-free anionic surfactant:amphoteric surfactant can range from 1:1 to 15:1, or from 1.5:1 to 10:1, or from 2.25:1 to 9:1, or from 4.5:1 to 7:1. The amount of the polymer of the present technology can range from 0.5 to 5 wt.%, or from 1 to 4.5 wt.%, or from 1.5 to 3.5 wt.%, based on the total weight of the body wash composition.

Body wash embodiments of the technology can be formulated as moisturizing body washes, antibacterial body washes, dual purpose body wash and shampoo, bath gels, shower gels, liquid hand soaps, body scrubs, bubble baths, facial scrubs, foot scrubs, and the like.

### Shampoos

In one aspect, a personal care composition in which the polymer of the present technology is useful is a shampoo. Typical components of a shampoo, in addition to the polymer thickener and water are: at least one surfactant; a sufficient pH adjusting agent (base and/or acid) to attain a pH of from 4 to 7.5, or from 3.5 to 6.0, or from 4.0 to 5.5; and optional ingredients selected from the adjuvants, additives and benefit agents discussed above, and mixtures thereof, including benefit agents selected from conditioning agents (e.g., silicones, oils, esters and/or cationic conditioning agents), pearlizing agents, vitamins, fragrances, dyes, preservatives including acids, botanicals, and insoluble gas bubbles, liposomes, and cosmetic beads and flakes, and anti-dandruff agents, and mixtures thereof. In one aspect, the surfactant is a sulfated or sulfate-free anionic surfactant. In another aspect, the surfactant is a mixture of a sulfated or sulfate-free anionic surfactant and an amphoteric surfactant, in optional combination with a cationic and/or a non-ionic surfactant. In one aspect, the sulfated or sulfate-free anionic surfactant can be present in an amount ranging from 5 to 40 wt.%, or from 6 to 30 wt.%, or from 8 to 25 wt.%, based on the total weight of the shampoo composition. When mixtures of sulfated or sulfate-free anionic and amphoteric surfactants are used, the weight ratio of sulfated or sulfate-free anionic surfactant to amphoteric surfactant can range from 1:1 to 10:1, or from 2.25:1 to 9:1, or from 4.5:1 to 7:1. The amount of the polymer of the present technology can range from 0.5 to 5 wt.%, or from 1 to 3 wt.%, or from 1.5 to 2.5 wt.%, based on the total weight of the shampoo composition.

The shampoo embodiments of the disclosed technology can be formulated as 2-in-1 shampoos, baby shampoos, conditioning shampoos, bodifying shampoos, moisturizing shampoos, temporary hair color shampoos, 3-in-1 shampoos, anti-dandruff shampoos, hair color maintenance shampoos, acid (neutralizing) shampoos, medicated shampoos, and salicylic acid shampoos, and the like.

Fatty Acid Liquid Soap Based Cleansers

In one aspect, a personal care composition in which the polymer of the present technology is useful is a fatty acid soap-based cleanser. Typical components of a fatty acid based soap cleanser, in addition to the polymer thickener are: at least one fatty acid salt; an optional surfactant (other than a fatty acid soap) or mixture of surfactants; a sufficient pH adjusting agent (base and/or acid) to attain a pH of above 7, or from 7.5 to 14, or from 8 to 12, or from 8.5 to 10; and optional ingredients selected from the adjuvants, additives and benefit agents discussed above, and mixtures thereof, including benefit agents selected from silicones, humectants, pearlizing agents, vitamins, oils, fragrances, dyes, preservatives, botanicals, anti-microbial agents, anti-dandruff agents, exfoliating agents, insoluble gas bubbles, liposomes, microsponges, cosmetic beads and flakes.

In one aspect, the fatty acid soaps are selected from at least one the fatty acid salt (e.g., sodium, potassium, ammonium) containing from 8 to 22 carbon atoms. In another aspect of the technology the liquid soap composition contains at least one fatty acid salt containing from 12 to 18 carbon atoms. The fatty acids utilized in the soaps can be saturated and unsaturated and can be derived from synthetic sources, as well as from the saponification of fats and natural oils by a suitable base (e.g., sodium, potassium, and ammonium hydroxides). Exemplary saturated fatty acids include but are not limited to octanoic, decanoic, lauric, myristic, pentadecanoic, palmitic, margaric, steric, isostearic, nonadecanoic, arachidic, behenic, and the like, and mixtures thereof. Exemplary unsaturated fatty acids include but are not limited to the salts (e.g., sodium, potassium, ammonium) of myristoleic, palmitoleic, oleic, linoleic, linolenic, and the like, and mixtures thereof. The fatty acids can be derived from animal fat such as tallow or from vegetable oil such as coconut oil, red oil, palm kernel oil, palm oil, cottonseed oil, olive oil, soybean oil, peanut oil, corn oil, and mixtures thereof. The amount of fatty acid soap that can be employed in the liquid cleansing compositions of this embodiment ranges from 1 to 50 wt.%, or from 10 to 35 wt.%, or from 12 to 25 wt.%, based on the weight of the total composition.

An optional sulfated or sulfate-free anionic surfactant can be present in the soap composition in an amount ranging from 1 to 25 wt.%, or from 5 to 20 wt.%, or from 8 to 15 wt.%, based on the weight of the total weight of the soap composition. Mixtures of sulfated or sulfate-free anionic and amphoteric surfactants can be used. The weight ratio of anionic surfactant to amphoteric surfactant can range from 1:1 to 10:1, or from 2.25:1 to 9:1, or from 4.5:1 to 7:1.

In the foregoing soap embodiments of the present technology, the amount of polymer can range from 0.5 to 5 wt.%, or from 1 to 3 wt.%, or from 1.5 to 2.5 wt.%, based on the total weight of the soap composition.

The liquid fatty acid soap based cleanser embodiments of the technology can be formulated as body washes, bath gels, shower gels, liquid hand soaps, body scrubs; bubble baths, facial scrubs, and foot scrubs, 2-in-1 shampoos, baby shampoos, conditioning shampoos, bodifying shampoos, moisturizing shampoos, temporary hair color shampoos, 3-in-1 shampoos, anti-dandruff shampoos, hair color maintenance shampoos, acid (neutralizing) shampoos, anti-dandruff shampoos, medicated shampoos, and salicylic acid shampoos, and the like.

Fixatives

In one aspect, a personal care composition in which the polymer of the present technology is useful is a hair fixative. The term "fixative" as applied to polymers encompasses the properties of film-formation, adhesion, or coating deposited on a surface on which the polymer is applied. The terms "hair styling, hair setting, and hair fixative" as commonly understood in the hair care arts, and as used herein, refer collectively to hair setting agents that are hair fixatives and film formers and which are topically applied to the hair to actively contribute to the ease of styling and/or holding of a hair set, and to maintain the re-stylability of the hair set. Hence, hair setting compositions include hair styling, hair fixative, and hair grooming products that conventionally are applied to the hair (wet or dry) in the form of gels, rinses, emulsions (oil-in-water, water-in-oil or multiphase), such as lotions and creams, pomades, sprays (pressurized or non-pressurized), spritzes, foams, such as mousses, shampoos, solids, such as sticks, semisolids and the like, or are applied from a hair setting aid having the hair setting composition impregnated therein or coated thereon, to leave the hair setting agent in contact on the hair for some period until removed, as by washing.

In one aspect, hair setting compositions encompasses products comprising at least one ASE and/or HASE polymer of the present technology alone or in combination with an auxiliary fixative polymer as a hair setting adjuvant. The product can be applied to the hair (wet or dry) before, during or after configuring the hair into the shape (curly or straight) desired, without limitation as to product form. The ASE and HASE polymers of the present technology are useful in combination with commercially available auxiliary hair fixative polymers, such as nonionic, cationic, and amphoteric hair setting polymers, cationic conditioning polymers, and combinations thereof. Conventional hair fixative and hair styling polymers are described above under section header "Auxiliary Fixatives".

Typical components of a hair fixative, in addition to the polymer thickeners of the present technology and water are: a sufficient pH adjusting agent (base and/or acid) to attain a pH of from 4 to 7.5, or from 3.5 to 6.0, or from 4.0 to 5.5; and optional ingredients selected from the adjuvants, additives and benefit agents discussed above, and mixtures thereof, including benefit agents selected from conditioning agents (e.g., silicones, oils, esters, waxes and/or cationic conditioning

agents), pearlizing agents, fatty acids, fatty alcohols, vitamins, fragrances, dyes, pigments preservatives including acids, botanicals, and insoluble gas bubbles, liposomes, and mixtures thereof.

The fixative polymer typically comprises 0.01 to 25 wt.%, or from 0.1 to 10 wt.%, or from 0.2 to 5 wt.%, based on the total weight of the fixative composition.

The present technology is illustrated by the following examples . Unless specifically indicated otherwise, parts and percentages are given by weight. All weights and percentages are expressed as 100 percent active material unless specified otherwise.

Methods

Molecular Weight Determination

The number average molecular weights for the polymers referenced herein are measured by GPC using a PL-GPC 220 high temperature GPC instrument manufactured by Polymer Laboratories (Varian, Inc.). Approximately 0.02 g polymer sample is dissolved in 5 ml of dimethyl acetamide (DMAc), containing 250 ppm of butylated hydroxytoluene (BHT) and 0.05 molar $NaNO_3$. The test sample solution is gently shaken for about two hours and filtered by passing the sample solution through a 0.45 μm PTFE disposable disc filter. The chromatographic conditions are: Mobile phase: DMAc, with 250 ppm BHT and 0.05m $NaNO_3$, 70° C, 1.0 ml/min. Sample size: 100μl Column set: PLgel (Guard + 2 x Mixed-A), all 10μm, in series. Waters Empower Pro LC/GPC software is used to analyze the results and to calculate $M_n$ of the polymer components of the technology.

The number average molecular weights referenced herein for the starch component are measured by GPC using a PL GPC-220 high temperature GPC instrument manufactured by Polymer Laboratories (Varian, Inc.). Approximately 0.20 g of sample solution was dissolved in 5 mL of DMSO containing 250 ppm of butylated hydroxytoluene (BHT) and 0.02 M $NaNO_3$. The solution was placed on a shaker at a speed of 30 overnight. Then, it was taken into an oven at 80 °C for two hours. The resulting sample solution was filtered through a 0.45 μM PTFE disposable syringe filter. The chromatographic conditions are: mobile phase: DMSO with 50 mM LiBr and 250 ppm BHT, 80 °C, 1.0 mL/min. Sample size: 100 μL Column set: PLgel Mixed-C 5 μm 300 x 7.5 mm with PLgel Guard 50 x 7.5 mm. Waters Empowers Pro is used to analyze results and calculate molecular weight of the polymer components against polysaccharide standards.

Viscosity

Brookfield rotating spindle method (all viscosity measurements reported herein are conducted by the Brookfield method whether mentioned or not): The viscosity measurements are reported in mPa·s, employing a Brookfield rotating spindle viscometer, Model DV2TRV (Brookfield Engineering Laboratories, Inc.), at about 20 revolutions per minute (rpm), at ambient room temperature of about 20 to 25 °C (hereafter referred to as viscosity). Spindle sizes are selected in accordance with the standard operating recommendations from the manufacturer. Generally, spindle sizes are selected as follows:

| Spindle Size No. | Viscosity Range (mPa·s) |
|---|---|
| 1 | 1 - 50 |
| 2 | 500 - 1,000 |
| 3 | 1,000 - 5,000 |
| 4 | 5,000 - 10,000 |
| 5 | 10,000 - 20,000 |
| 6 | 20,000 - 50,000 |
| 7 | >50,000 |

The spindle size recommendations are for illustrative purposes only. The artisan of ordinary skill in the art will select a spindle size appropriate for the system to be measured.

Yield Value

Yield Value, also referred to as Yield Stress, is defined as the initial resistance to flow under stress. It is measured by the Brookfield Yield Value (BYV) Extrapolation Method using a Brookfield viscometer (Model RV) at ambient room

temperature of about 20 to 25 °C. The Brookfield viscometer is used to measure the torque necessary to rotate a spindle through a liquid sample at speeds of 0.5 to 100 rpm. Multiplying the torque reading by the appropriate constant for the spindle and speed gives the apparent viscosity. Yield Value is an extrapolation of measured values to a shear rate of zero. The BYV is calculated by the following equation:

$$\text{BYV, dyn/cm}^2 = (\eta_{\alpha 1} - \eta_{\alpha 2})/100$$

where $\eta_{\alpha 1}$ and $\eta_{\alpha 2}$ = apparent viscosities obtained at two different spindle speeds (0.5 rpm and 1.0 rpm, respectively). These techniques and the usefulness of the Yield Value measurement are explained in Technical Data Sheet Number 244 (Revision: 5/98) from Noveon Consumer Specialties of Lubrizol Advanced Materials, Inc., herein incorporated by reference.

Clarity

The clarity (turbidity) of a composition is determined in Nephelometric Turbidity Units (NTU) employing a nephelometric turbidity meter (Mircro 100 Turbidimeter, HF Scientific, Inc.) at ambient room temperature of about 20 to 25 °C. Distilled water (NTU = 0) is utilized as a standard. Six-dram screw cap vials (70 mm x 25 mm) are filled almost to the top with test sample and centrifuged at 3200 rpm until all bubbles are removed. Upon centrifugation, each sample vial is wiped with tissue paper to remove any smudges before placement in the turbidity meter. The sample is placed in the turbidity meter and a reading is taken. Once the reading stabilizes the NTU value is recorded. The vial is given one-quarter turn and another reading is taken and recorded. This is repeated until four readings are taken. The lowest of the four readings is reported as the turbidity value. Compositions having an NTU value of about >100 were judged to be hazy or turbid.

Suspension Stability Test

Suspension Testing Procedure: The ability of a polymer system to suspend active and/or aesthetically pleasing insoluble oily or particulate materials is important from the standpoint of product efficacy and appeal. A six-dram vial (approximately 70 mm high x 25 mm in diameter) is filled to the approximately 65 mm point with a bath gel test formulation. Each sample vial is centrifuged to remove any trapped air bubbles contained in the formulation. Cosmetic beads (e.g., Lipopearl™ gelatin capsules; average diameter 500-3000 microns) are weighed into the centrifuged sample (1.0 wt.% based on the weight of the total composition) and stirred gently with a wooden stick until they are uniformly dispersed throughout the bath gel sample. The position of approximately 10 of the beads within each sample vial is noted by drawing a circle around the bead with black marker pen on the outer glass surface of the vial to establish the initial position of the beads within the gel. The vials are placed in a 45 °C oven to age for a 12-week period. The bead positioning within the outlined drawn circles indicates the suspension properties of each sample and are monitored on a weekly basis. The suspension results are visually graded as simply PASS/FAIL based on the number of beads remaining (*from the original 10 circled beads)* within their respective targeted hand drawn circles. Thus, a passing sample grade is indicated by having at least (7) circled beads still visibly located within their drawn circled targets on the outside of the vial glass. A failing grade is given to any samples which have more than (3) or all of the targeted and circles beads drifting outside of the drawn target circles after 12 weeks.

Particle Size Measurement

The particle size of the latexes was determined by Dynamic Light Scattering (DLS). Samples were prepared by diluting two-drops of the polymer latex (dispensed from a 5.8 ml VWR 16001-180 disposable polyethylene graduated transfer pipet) in 5 ml of deionized (D.I.) water. Intensity-weighted average particle size (diameter (d. nm)) was measured using a Particle Size Systems (PSS) Nicomp 380 ZLS particle size analyzer. Measurements were made using a laser wavelength of 632.5-635 nm with a scattering angle set to 90 degrees and the intensity setpoint (photopulse rate) of 300 kHz.

Abbreviation and Trade Name Ingredient List

The following ingredients are utilized in the examples of the present invention:

| Monomers | |
|---|---|
| EA | Ethyl Acrylate |

(continued)

| Monomers | |
|---|---|
| MAA | Methacrylic Acid |
| TMPTA | Trimethylolpropane Triacrylate (crosslinker) |
| Components | |
| Acrylates/C10-30 Alkyl Acrylate Crosspolymer | Carbopol™Ultrez 20 Polymer, Lubrizol Advanced Materials, Inc. |
| Aminomethyl Propanol | AMP-Utra™ PC 2000 (neutralizer), Angus Chemical Company |
| Acrylates Copolymer | Carbopol™ Aqua SF-1 Polymer (A crosslinked emulsion copolymer of two or more monomers consisting of acrylic acid, methacrylic acid or one of their simple esters), Lubrizol Advanced Materials, Inc. |
| Cocamidopropyl Betaine | Chembetaine™ CAD (amphoteric surfactant - 35% active), Lubrizol Advanced Materials, Inc. |
| Coco Glucoside | Plantaren ™ 818 UP (nonionic surfactant - 52% active), BASF |
| $C_{36}$ Dimer Acid | Pripol™ 1006 Difunctional $C_{36}$ Dimer Acid, Croda International, Plc. |
| Monomers | |
| Disodium Cocoyl Glutamate | Amisoft™ ECS-22W (anionic surfactant - 30% active), Ajinomoto Health & Nutrition North America, Inc. |
| MEG ESTER | Glucamate™ LT Thickener (PEG-120 Methyl Glucose Trioleate (and) Propylene Glycol - 43% active), Lubrizol Advanced Materials, Inc. |
| Oxidized Potato Starch | (Perfectamyl™ A 4692 Starch, Royal Avebe |
| PVAc | Polyvinyl Acetate |
| PVOH | Selvol™ 203 PVOH; Hydrolysis 87-89 (mole percent); Sekisui Specialty Chemicals America, LLC |
| Sodium Lauryl Ether Sulfate | Sulfochem™ ES-2BZ - 2 moles of ethoxylation (sulfated anionic surfactant - 28% active), Lubrizol Advanced Materials, Inc. |

EXAMPLE 1 (Polymer Synthesis)

An emulsion polymer was synthesized in a staged, semi-batch, emulsion polymerization process to produce a stable latex polymer particle. To an agitator equipped first reactor, 62.95 phm of EA and 34.5 of MAA were charged under a nitrogen atmosphere and mixed at 700 rpm to form a stable monomer mixture. To an agitator equipped, temperature controlled second reactor, 217.39 of D.I. water was added and mixed under a nitrogen atmosphere at 210 rpm and heated to 60 °C. As the reactor was heated, 29.38 phm (85 % wt./wt.) of an oxidatively degraded potato starch (Perfectamyl™ A 4692), 0.07 phm of lauric acid, and 0.17 phm of Selvol™ 203 PVOH were charged to the reactor, followed by 86.96 phm of D.I. water used to quantitatively transfer and rinse any solid powder adhered to the reactor walls into the reactor. When a homogenous mixture of reaction components was obtained, the pH of the mixture was adjusted to 5 using 10 percent acetic acid. After a 120-minute hold time at 60 °C, 0.1 phm of pullulanase enzyme dissolved in 5.65 phm of D.I. water was added to the reaction mixture. The enzyme-containing mixture was held for 30-minutes at 60 °C, after which the reactor contents were heated to 85 °C.

When the contents of the reactor reached 85 °C, 0.004 phm/g (wt./wt.) of aqueous ammonium persulfate initiator solution was fed into the reactor at a feed rate of 0.27 phm/min. into the second reactor. Simultaneously with the initiator feed, the monomer mixture from the first reactor was metered into the second reactor at a feed rate of 0.67phm/min over a period of 150-minutes.

After reacting for 30-miuntes, first stage polymer latex particles were formed. At the conclusion of the 30-minute first stage reaction, second stage monomers comprising 0.378 phm TMPTA crosslinker mixed in 2.17 phm of EA were added to the first reactor and fed into the second reactor with the remaining first reactor monomers over the remainder of the 150-minute monomer feed cycle. As the second stage monomer mixture was introduced to the reactor, the reaction temperature was increased to 88 °C.

Following the completion of the 150-minute monomer feed cycle, the reaction temperature was held at 88 °C for

60-minutes. At the conclusion of 60-minute hold, the reactor contents were cooled to 49 °C. To reduce residual monomer, a redox oxidant comprising a solution of 0.15 phm of t-butyl hydroperoxide diluted in 4 phm of D.I. water was added to the reactor contents, followed by the addition of a redox reductant comprising 0.16 phm of erythorbic acid diluted in 4 phm of D.I. water. The redox reaction was allowed to proceed for 30-minutes. At the conclusion of 30-minutes, the redox reaction was repeated. At the conclusion of the second 30-minute redox reaction, the resulting polymer product was cooled to room temperature, discharged from the reactor, and recovered as a polymer latex.

EXAMPLES 2 to 11 (Polymer Synthesis)

The polymers of Examples 2 to 11 were synthesized utilizing the same components, conditions and procedures as described in Example 1, except that the amount of the respective monomers, initiators, and stability package components (starch, PVOH, acid) were changed as set forth in Table 1.

TABLE 1

| Synthesis Ex. No.* | EA (phm)[1] | MAA (phm)[1] | TMPTA (phm)[1] | Meg Ester[2] | Acrylates/C10-30 Alkyl Acrylate Crosspolymer | Starch[2] | PVOH[2] | PVAc | Acid[2] |
|---|---|---|---|---|---|---|---|---|---|
| 2 | 65.12 | 34.50 | 0.38 | -- | -- | 24.98 | 0.07 | -- | 0.17[3] |
| 3 | 65.12 | 34.50 | 0.38 | -- | -- | 24.98 | 0.17 | -- | 0.07[3] |
| 4 | 65.12 | 34.50 | 0.38 | -- | -- | 24.98 | 0.17 | -- | 0.07[3] |
| 5 | 65.12 | 34.50 | 0.38 | | | 24.98 | 0.17 | -- | 0.07[4] |
| 6 | 65.12 | 34.50 | 0.38 | | | 24.98 | -- | 0.17 | |
| 7 | 65.12 | 34.50 | 0.38 | | | 24.98 | 0.17 | | 0.08[3] |
| 8[5] | 65.12 | 34.50 | 0.38 | -- | -- | 24.98 | 0.50 | -- | -- |
| 9[5] | 65.12 | 34.50 | 0.38 | | | 24.98 | 0.25 | -- | -- |
| 10[5] | 65.12 | 34.50 | 0.38 | 3.01 | -- | 24.98 | -- | -- | -- |
| 11[5] | 65.12 | 34.50 | 0.38 | -- | 0.25 | 24.98 | -- | -- | -- |

*The latex of synthesis examples 1, 2, 3, and 5-11 contained about 25 wt.% polymer solids and the latex of synthesis example 4 contained about 32 wt.% polymer solids.
[1]Parts per hundred total monomer (by wt.)
[2]Parts/100 parts total monomer (wt./wt.)
[3]Lauric acid
[4]$C_{36}$ dimer acid
[5]Comparative

Examples 12 to 18

Separate sulfate containing cleansing compositions were formulated utilizing the polymers (1.7 wt.% active polymer) set forth in Table 3 and the components set forth in Table 2. Additionally, two polymers prepared in accordance with Examples 47 and 57 of WO2014/139904 were identically formulated and tested. In separate mixing vessels the polymer of each example was added to an aqueous solution of a sulfate containing anionic surfactant, followed by adjusting the pH to 6.5 with a 50 % sodium hydroxide solution. An amphoteric surfactant, tetrasodium EDTA, and sodium benzoate were then added to the vessel and the pH was back-acid adjusted with citric acid to a pH of 5.0-5.2. Each sulfate containing cleansing formulation was evaluated for rheology properties and clarity attributes which are reported in Table 3.

Table 2

| (Sulfate Containing Formulations) | | | |
|---|---|---|---|
| | Component | Amount (wt. %) | Function |
| 1 | D.I. Water | q.s. to 100 | Diluent |
| 2 | Polymer (30% active polymer solids) | 1.7 | Rheology Modifier |

(continued)

| (Sulfate Containing Formulations) | | | |
|---|---|---|---|
| | Component | Amount (wt. %) | Function |
| 3 | Sulfochem™ ES-2BZ Surfactant | 12.4 | Sulfate Containing Anionic Surfactant |
| 4 | Chembetaine™ CAD Surfactant (35% active) | 2.6 | Amphoteric Surfactant |
| 5 | Tetrasodium EDTA | 0.05 | Chelating Agent |
| | Sodium Benzoate | 0.50 | Preservative |
| 7 | NaOH (50%) | q.s. to pH 6.5 | pH Adjusting Agent |
| 8 | Citric Acid **(50%)** | q.s. to pH 5-5.2 | pH Adjusting Agent |

Table 3

| Ex. No. | Synthesis Ex. No. | Viscosity (cP) | Yield Value ($dynes/cm^2$) | Clarity (NTU) | pH |
|---|---|---|---|---|---|
| 12 | 1 | 14060 | 252 | 40.9 | 5.15 |
| 13 | 2 | 12780 | 124 | 47.7 | 5.06 |
| 14 | 3 | 8400 | 76 | 85.1 | 5.04 |
| 15 | 4 | 12200 | 196 | 54.9 | 5.12 |
| 16[1] | 8 | 203000 | 450 | 95.3 | 5.0 |
| 17[1] | 47[3] | 21800 | 260 | OR[5] | 5.08 |
| 18[1] | 57[4] | 21750 | 610 | 313 | 5.18 |

[1]Comparative
[2]Formulated with1.6 wt.% of active polymer
[3]Prepared in accordance with Example 47 of WO2014/13990
[4]Prepared in accordance with Example 57 of WO2014/13990
[5]Out of range of the instrument's measurement scale

The polymers of the present technology when formulated into sulfate containing surfactant systems exhibit better clarity values (NTU) than polymers prepared without the stabilizer system of the present technology formulated into identical sulfate containing systems.

<u>EXAMPLES 19 to 26</u>

Separate sulfate-free containing cleansing compositions were formulated with the polymers and components set forth in Table 5 and Table 4, respectively. In a vessel, each polymer latex was combined with deionized (D.I.), disodium cocoyl glutamate and cocoglucoside and mixed until homogeneous. The pH was adjusted to 6.5 with 20% sodium hydroxide. Amphoteric surfactant was then added to the vessel and mixed with the contents until homogenous. The final pH was then adjusted to pH 7.0 - 7.2 with 20% sodium hydroxide.

Sample viscosities were measured using a Brookfield RV viscometer using spindle number 4 (or an appropriate spindle for the resulting viscosity). The viscosity of the sample was measured at 20 rpm for 60 seconds at ambient room temperature (20-24 °C). The yield value and clarity (NTU) for each sample formulation were also measured.

Table 4

| (Sulfate-Free Formulations) | | | |
|---|---|---|---|
| | Component | Amount (active wt. %) | Function |
| 1 | D.I. Water | q.s. to 100 | Diluent |
| 2 | Polymer (active polymer solids) | 3.3 | Rheology Modifier |
| 3 | Disodium Cocoyl Glutamate | 4 | Sulfate-Free Anionic Surfactant |
| 4 | Coco Glucoside | 3 | Nonionic Surfactant |

(continued)

| (Sulfate-Free Formulations) | | | |
|---|---|---|---|
| | Component | Amount (active wt. %) | Function |
| 5 | Cocamidopropyl Betaine | 5 | Amphoteric Surfactant |
| 6 | NaOH (20%) | q.s. to desired pH | 7-7.15 |
| 7 | D.I. Water | q.s. to 100 | Diluent |

Table 5

| Ex. No. | Synthesis Ex. No. | Suspension | Viscosity (cP) | Yield Value (dynes/cm$^2$) | NTU | pH |
|---|---|---|---|---|---|---|
| 19 | 1 | Pass | 2840 | 152 | 16.4 | 7.01 |
| 20 | 2 | Pass | 2830 | 110 | 15.7 | 7.05 |
| 21 | 3 | Pass | 2930 | 168 | 15.4 | 7.09 |
| 22 | 4 | Pass | 3670 | 226 | 16.4 | 7.03 |
| 23[1] | 8[1] | Pass | 3300 | 124 | 29.1 | 7.15 |
| 24[1] | 9[1] | Pass | 3430 | 168 | 25.2 | 7.0 |
| 25[1] | 10[1] | Pass | 2300 | 84 | 33.9 | 7.15 |
| 26[1] | 11[1] | Fail | 3120 | 114 | 39 | 7.08 |
| Comparative | | | | | | |

The polymers of the present technology when formulated into sulfate-free surfactant systems exhibit significantly better clarity values (NTU) than polymers prepared without the stabilizer system of the present technology formulated into identical sulfate-free systems. This offers a significant advantage over other liquid rheology modifiers as sulfate-free systems tend to be extremely challenging chassis to formulate. Obtaining a high viscosity and yield value in a clear sulfate-free system is attainable by formulating with the polymers of the present technology.

EXAMPLE 27 (Hydroalcoholic Gel Formulation)

A hydroalcoholic gel hand sanitizer was formulated utilizing the polymer of Synthesis Example 7 and the components set forth in Table 6. To a 250 ml beaker, the neat polymer latex was combined with D.I. water and mixed util homogeneous. The polymer was neutralized with aminomethyl propanol to the desired pH (7.3-7.7). To the neutralized latex dispersion was then added denatured ethanol. The desired final pH of the formulations should be in the ranges 7.3-7.7 and can be adjusted with additional aminomethyl propanol. The rheological properties of the formulations were compared to identically formulated commercially available ASE benchmark polymer, which are set forth in Table 7.

Sample viscosities were measured on a Brookfield RV viscometer using spindle number 3 (or appropriate spindle for the resulting viscosity). Sample yields were calculated by measuring the viscosity at 0.5 rpm for 60 seconds, and then at 1.0 rpm for 60 seconds. Those viscosities are then input in equation 1 to calculate the sample yield. Viscosity and yield data are collected in Table 5.

Table 6

| Component | (wt.%) | Function |
|---|---|---|
| Active Polymer (Synthesis Ex. No.7) | 5 | Rheology Modifier |
| Aminomethyl Propanol | pH 7.35 - 7.64 | Neutralizer |
| Denatured Ethanol | 70 | Antimicrobial |
| D.I. Water | q.s. to 100 | Diuent |

Table 7

| | Commercial Benchmark Polymer[1] | | | Polymer of Example 7 | |
|---|---|---|---|---|---|
| Active Polymer (wt.%) | 5 | 5 | 5 | 5 | 5 |
| Appearance[2] | Clear | Clear | Clear | N/A | Hazy |
| pH | 7.4 | 7.49 | 7.35 | 7.35 | 7.64 |
| Brookfield Viscosity (cP) | 4880 | 6540 | 6210 | 26800 | 7600 |
| Yield Value (dyn/cm$^2$) | 298 | 446 | 390 | 1990 | 504 |
| Clarity (NTU) | 6.65 | 9.08 | 5.52 | N/A | 448 |

[1]Carbopol Aqua SF-1 polymer - a crosslinked ASE polymer prepared from (meth)acrylic acid and a $C_1$-$C_5$ alkyl ester of (meth)acrylic acid
[2]Based on a visual observation

While hydroalcoholic compositions formulated with the polymer of Example 7 are hazy compared to identical compositions prepared with a commercial ASE benchmark polymer, the rheological properties (Brookfield viscosity and yield value) of compositions prepared with the polymers of the present technology are significantly better than compositions prepared with the benchmark ASE polymer.

Example 28 (Particle Size Stability Improvement at 4° C After Addition of Citric Acid)

An emulsion polymer latex was synthetized as described in Example 1. To enhance the shelf-life of the exemplary latex, different quantities of a 50% solution (wt./wt.) of citric acid/D.I. water were dosed to the polymer latex. The polymer was dosed with either a 2.5% (1.25% active citric acid), 5.0% (2.5% active citric acid), 7.5% (3.75% active citric acid) or 10% (5% active citric acid) solution of 50% (wt./wt.) citric acid/D.I. water with respect to the weight of the latex. A control sample diluted with 10 % D.I. water (wt./wt.) was also included. Stability was compared to samples without any dosing ('neat latex'). To understand the impact of citric acid on the long-term stability of the latex product all samples were held at 4° C and particle size was measured periodically to monitor flocculation of the latex particles. The results are set forth in Table 8.

TABLE 8

| | Samples | | | | | |
|---|---|---|---|---|---|---|
| 50% Citric Acid Solution (%)[1] (wt./wt.) | 0 | 2.5 | 5 | 7.5 | 10 | 0 |
| Active Citric Acid (%)[1] (wt./wt.) | 0 | 1.25 | 2.5 | 3.75 | 5 | 0 |
| D.I. Water (%)[1] (wt./wt.) | 0 | 1.25 | 2.5 | 3.75 | 5 | 10 |
| Particle size (d. nm) Day 0 | 258.0 | 256.0 | 249.0 | 256.0 | 260.0 | 328.7 |
| Particle size (d. nm) Day 3 | 334.0 | 324.0 | 281.0 | 274.0 | 260.0 | -- |
| Particle size (d. nm) Day 9 | 633.6 | 608.4 | 412.3 | 344.2 | 301.8 | -- |
| Particle size (d. nm) Day 15 | 885.0 | 872.0 | 521.0 | 421.0 | 343.0 | -- |
| Particle size (d. nm) Day 24 | -- | -- | -- | -- | -- | 1056 |
| Particle size (d. nm) Day 29 | 1832.0 | 1182.0 | 1037.0 | 683.0 | 456.0 | -- |
| Particle size (d. nm) Day 52 | -- | -- | -- | -- | -- | 2190 |
| Particle size (d. nm) Day 58 | 1761.4 | 1444.1 | 1379.4 | 1249.2 | 640.9 | -- |

[1]All post-added materials (citric acid solution, active citric acid, and water) are calculated with respect to the weight of the wet latex.

Increasing the amount of citric acid in the latex progressively decreases the slope of particle size growth and extends the life of the latex at low temperatures. The particle size of the water treated latex increased similarly to the way the neat, untreated, latex increased. The water control demonstrates that the slow particle size growth is not a function of simply diluting the latex. The addition of citric acid to the latex slows particle size growth. If there is excessive growth of the

polymer particle, the latex will become viscous, and the material cannot be handled.

Example 29 (Particle Size Stability Improvement at 4° C After Addition of Erythorbic Acid)

An emulsion polymer latex was synthetized as described in Example 1. The effect of different amounts of erythorbic acid (added as a 25% solution in D.I. water to the latex) on particle size is shown in Table 9. The amount of erythorbic acid post-added was based on the weight of wet latex used.

TABLE 9

| | Samples | | |
|---|---|---|---|
| 25% Ervthorbic Acid Solution (%) (wt./wt) | 0 | 10 | 20 |
| Active Erythorbic Acid (%) (wt./wt.) | 0 | 2.5 | 5 |
| D.I. Water (%) (wt./wt.) | 0 | 7.5 | 15 |
| Particle size (d. nm) Day 0 | 291.0 | 286.0 | 283.0 |
| Particle size (d. nm) Day 6 | 733.0 | 385.0 | 309.0 |
| Particle size (d. nm) Day 9 | 990.0 | -- | -- |
| Particle size (d. nm) Day 13 | -- | 458.0 | 327.0 |
| Particle size (d. nm) Day 20 | -- | 672.0 | 367.0 |
| Particle size (d. nm) Day 27 | -- | 712.0 | 392.0 |
| Particle size (d. nm) Day 34 | -- | 809.0 | 419.0 |
| Particle size (d. nm) Day 42 | -- | -- | 462.0 |

The particle size growth (coalescence) was mitigated over time by dosing the emulsion polymer latex with erythorbic acid at 2.5 % and 5 % (based on the total weight of the latex) compared with the un-dosed latex.

Example 30 (Particle Size Stability Improvement at 4° C After Addition of a Citric Acid and Erythorbic Acid Blend)

An emulsion polymer latex was synthetized as described in Example 1. The effect of different amounts of a blend of citric acid (added as 50% solution in D.I. water (w/w)) and erythorbic acid (added as a 25% solution in D.I. water (w/w)) to the latex at 4° C on particle size is shown in Table 10. The amount of citric acid/erythorbic acid that was post-added was based on the weight of wet latex used.

TABLE 10

| | Samples | | |
|---|---|---|---|
| 25% Erythorbic Acid Solution (%) (wt./wt) | 2.5 | 5 | 10 |
| Active Erythorbic Acid (%) (wt./wt.) | 0.625 | 1.25 | 2.5 |
| 50% Citric Acid Solution (%) (wt./wt) | 1.25 | 2.5 | 5 |
| Active Citric Acid (%) (wt./wt.) | 0.625 | 1.25 | 2.5 |
| D.I. Water (%) (wt./wt) | 2.5 | 5 | 10 |
| Particle size (d. nm) Day 0 | 238.0 | 239.0 | 236.0 |
| Particle size (d. nm) Day 7 | 700.0 | 386.0 | 339.0 |
| Particle size (d. nm) Day 14 | 1067.0 | 611.0 | 469.0 |
| Particle size (d. nm) Day 21 | -- | 820.0 | 661.0 |
| Particle size (d. nm) Day 27 | -- | -- | 739.0 |

As dosing levels of the citric acid/erythorbic blend increases, the particle size growth (coalescence) over time of the latex particles was mitigated.

Example 31 (Particle Size Stability Improvement at 4° C After Addition of Sodium Bisulfite)

An emulsion polymer latex was synthetized as described in Example 1. The effect of different amounts of sodium bisulfite (added as 25% solution in D.I. water (w/w)) to the latex on particle size is shown in Table 11. The amount of sodium bisulfite that was post-added was based on the weight of wet latex used.

TABLE 11

| | Samples | | |
|---|---|---|---|
| 25% Sodium Bisulfite Solution (%) (wt./wt.) | 0 | 10 | 20 |
| Active Sodium Bisulfite (%) (wt./wt.) | 0 | 2.5 | 5 |
| D.I. Water (%) (wt./wt.) | 0 | 7.5 | 15 |
| Particle size (d. nm) Day 0 | 291.0 | 286.0 | 284.0 |
| Particle size (d. nm) Day 6 | 733.0 | 476.0 | 359.0 |
| Particle size (d. nm) Day 9 | 990.0 | -- | -- |
| Particle size (d. nm) Day 13 | -- | 688.0 | 422.0 |
| Particle size (d. nm) Day 20 | -- | 1547.0 | 576.0 |
| Particle size (d. nm) Day 27 | -- | -- | 647.0 |
| Particle size (d. nm) Day 34 | -- | -- | 749.0 |
| Particle size (d. nm) Day 42 | -- | -- | 954.0 |

The results indicate that the dosing levels of sodium bisulfite mitigate the coalescence of the latex particles compared to the un-dosed latex.

Example 32 (Particle Size Stability Improvement at Ambient Room Temperature After Addition of Citric Acid)

To confirm that coalescence mitigation extends to ambient conditions, citric acid treated latex samples were monitored at room temperature (20-25° C) and compared to the average shelf-life of untreated latex. A polymer latex was synthesized as described in Example 1. The latex was treated with 10% (wt./wt.) of a 50 % citric acid solution (wt./wt.) and compared to the same polymer latex that was not treated with citric acid. The results are presented in Table 12.

TABLE 12

| Latex Sample | Age (months) | Particle Size (d. nm) |
|---|---|---|
| Polymer of Ex. 1 | 11 | 319.2 |
| Polymer of Ex. 1 | 3 | 1492 |

The average particle size for a latex not treated with citric acid and held at room temperature for 3 months coalesced rapidly. The particle size of the citric acid treated samples was significantly lower than the untreated samples held at room temperature. This demonstrates that the stability observed at low temperature extends to room temperature stability as well.

## Claims

**1.** A polymer latex composition prepared by polymerizing a monomer composition comprising:

a) from 20 to 60 parts by wt., or from 30 to 50 parts by wt., or from 35 to 40 parts by wt. of at least one ethylenically unsaturated $C_3$-$C_6$ acid group containing monomer;
b) from 40 to 75 parts by wt., or from 50 to 70 parts by wt., or from 60 to 68 parts by wt. of at least one $C_1$ to $C_{30}$ alkyl ester of (meth)acrylic acid;
c) from 0 to 15 parts by wt., or from 0.1 to 10 parts by wt., or from 1 to 5 parts by wt. of at least one monomer selected from an associative monomer, a semi-hydrophobic monomer, and mixtures thereof;

d) from 0 to 10 parts by wt., or from 0.05 to 8 parts by wt., or from 0.1 to 5 parts by wt., or from 0.5 to 3 parts by wt., or from 0.75 to 2 parts by wt. of at least one polyunsaturated crosslinking monomer; wherein the sum of a), b), c) and d) in said monomer mixture is 100 parts by wt., and wherein said monomer mixture is polymerized in the presence of a stabilizer composition comprising:

i) from 0.1 to 75 parts by wt., or from 0.5 to 60 parts by wt., or from 1 to 55 parts by wt., or from 5 to 35 parts by wt., or from 10 to 30 parts by wt. of an enzymatically digested starch per 100 parts by wt. of said monomer composition;
ii) from 0.001 to 2 parts by wt., or from 0.005 to 1.5 parts by wt., or from 0.05 to 1 parts by wt., or from 0.1 to 0.75 parts by wt., or from 0.15 to 0.5 parts by wt. of PVOH per 100 parts by wt. of said polymerizable monomer composition; and
iii) from 0.001 to 0.5 parts by wt., or from 0.005 to 0.4 parts by wt., or from 0.01 to 0.3 parts by wt., or from 0.03 to 0.2 parts by wt., or from 0.05 to 0.1 parts by wt. of at least one acid selected from a saturated $C_2$ to $C_{50}$ mono- or diacid, an unsaturated $C_{10}$ to $C_{50}$ mono- or diacid, and mixtures thereof per 100 parts by wt. of said polymerizable monomer composition;

wherein said at least one associative monomer (c) in said monomer composition is represented by formulas I and/or II:

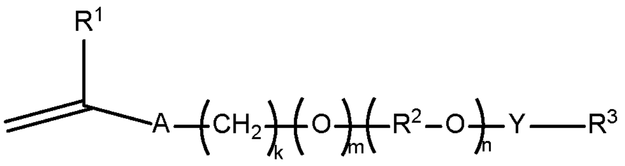
I

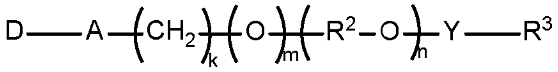
II

wherein $R^1$ is hydrogen or methyl; A is $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; $(R^2-O)_n$ is a polyoxyalkylene moiety, which can be a homopolymer, a random copolymer, or a block copolymer of $C_2$-$C_4$ oxyalkylene units, $R^2$ is a divalent alkylene moiety selected from $C_2H_4$, $C_3H_6$, or $C_4H_8$, and combinations thereof; and n is an integer in the range of 2 to 150, or from 10 to 120, or from 15 to 60; Y is $-R^2O-$, $-R^2NH-$, $-C(O)-$, $-C(O)NH-$, $-R^2NHC(O)NH-$, or $-C(O)NHC(O)-$; $R^3$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an aralkyl substituted phenyl, and an aryl-substituted $C_2$-$C_{30}$ alkyl; wherein the $R^3$ alkyl group, aryl group, phenyl group optionally comprises one or more substituents selected from the group consisting of a hydroxyl group, an alkoxyl group, benzyl group styryl group, and a halogen group; and
wherein said at least one semi-hydrophobic monomer (c) in said monomer composition is represented by formulas IV and/or V:

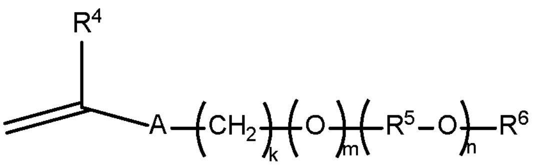
IV

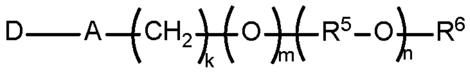
V

wherein $R^4$ is hydrogen or methyl; A is $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from about 0 to about 30, and m is 0 or 1, with the proviso that when k is 0, m is 0, and when k is in the range of 1 to about 30, m is 1; $(R^5-O)_n$ is a polyoxyalkylene moiety, which

can be a homopolymer, a random copolymer, or a block copolymer of $C_2$-$C_4$ oxyalkylene units, $R^5$ is a divalent alkylene moiety selected from $C_2H_4$, $C_3H_6$, or $C_4H_8$, and combinations thereof; and n is an integer in the ranging from about 2 to about 150, or from about 5 to about 120, or from about 10 to about 60; $R^6$ is selected from hydrogen and a linear or branched $C_1$-$C_4$ alkyl group; and D represents a vinyl or an allyl moiety.

**2.** A polymer composition of claim 1, wherein said at least one ethylenically unsaturated $C_3$-$C_6$ carboxylic acid monomer (a) in said monomer composition is selected from acrylic acid, methacrylic acid, itaconic acid, citraconic acid, maleic acid, maleic anhydride, fumaric acid, crotonic acid, aconitic acid, 2-acrylamido-2-methyl-1-propanesulfonic acid, and mixtures thereof, and/or,
wherein said at least one $C_1$ to $C_{30}$ linear/branched alkyl ester of (meth)acrylic acid (b) in said monomer composition is selected from methyl acrylate, methyl methacrylate, ethyl acrylate, ethyl methacrylate, hydroxyethyl acrylate, hydroxyethyl methacrylate, n-propyl acrylate, n-propyl methacrylate, isopropyl acrylate, isopropyl methacrylate, hydroxypropyl acrylate, hydroxypropyl methacrylate, n-butyl acrylate, n-butyl methacrylate, isobutyl acrylate, isobutyl methacrylate, tert-butyl acrylate, tert-butyl methacrylate, sec-butyl acrylate, sec-butyl methacrylate, hydroxybutyl acrylate, hydroxybutyl methacrylate, n-pentyl acrylate, n-pentyl methacrylate, neopentyl acrylate, neopentyl methacrylate, cyclohexyl acrylate, cyclohexyl methacrylate, 2-hexyl acrylate, 2-hexyl methacrylate, 2-ethylhexyl acrylate, 2-ethylhexyl methacrylate, n-octyl acrylate, n-octyl methacrylate, isooctyl acrylate, isooctyl methacrylate, decyl acrylate, decyl methacrylate, isodecyl acrylate, isodecyl methacrylate, dodecyl acrylate, dodecyl methacrylate, 2-propylheptyl acrylate and 2-propylheptyl methacrylate, lauryl acrylate, lauryl methacrylate, cetyl acrylate, cetyl methacrylate, stearyl acrylate, stearyl methacrylate, behenyl acrylate, behenyl methacrylate, melissyl acrylate, melissyl methacrylate, and mixtures thereof.

**3.** A polymer composition of claim 1 or claim 2, wherein said at least one associative monomer (c) in said monomer composition is represented by formula III:

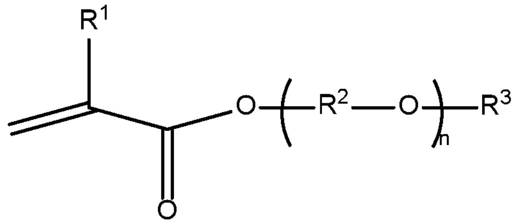
III

wherein $R^1$ is hydrogen or methyl; $R^2$ is a divalent alkylene moiety independently selected from $C_2H_4$, $C_3H_6$, and $C_4H_8$, and n represents an integer ranging from 5 to 60, ($R^2$-O) can be arranged in a random or a block configuration; $R^3$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an aralkyl substituted phenyl, and an aryl-substituted $C_2$-$C_{30}$ alkyl, wherein the $R^3$ alkyl group, aryl group, phenyl group optionally comprises one or more substituents selected from the group consisting of a hydroxyl group, an alkoxyl group, benzyl group styryl group, and a halogen group, or wherein said at least one associative monomer (c) in said monomer composition is selected from lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth) acrylate, phenyl polyethoxylated (meth)acrylate, nonylphenyl polyethoxylated (meth)acrylate, w-tristyrylphenyl polyoxyethylene methacrylate, wherein the polyethoxylated moiety of the monomer contains from 2 to 150, or from 5 to 120, or from 10 to 60, or from 15 to 30 ethylene oxide units.

**4.** A polymer composition of any of the previous claims, wherein said at least one semi-hydrophobic monomer (c) in said monomer composition is represented by formulas VI and/or VII:

$$CH_2{=}C(R^4)C(O)O\text{-}(C_2H_4O)_a(C_3H_6O)_b\text{-}H \qquad VI$$

$$CH_2{=}C(R^4)C(O)O\text{-}(C_2H_4O)_a(C_3H_6O)_b\text{-}CH_3 \qquad VII$$

wherein $R^4$ is hydrogen or methyl, and "a" is an integer ranging from 0 or 2 to 120, or from 5 to 45, or from 10 to 25; and "b" is an integer ranging from 0 to 120, or from 2 to 45, or from 10 to 25, subject to the proviso that "a" and "b" cannot be 0 at the same time.

5. A polymer composition of any of the previous claims, wherein said at least one polyunsaturated crosslinking monomer (d) is selected from a polyfunctional (meth)acrylate, a polyallyl ether, amphiphilic polyunsaturated macromonomer, and mixtures thereof, preferably, wherein said at least one polyunsaturated crosslinking monomer (d) is selected from ethylene glycol di(meth)acrylate, triethylene glycol di(meth)acrylate, 1,3-butylene glycol di(meth)acrylate, 1,4-butylene glycol di(meth)acrylate, 1,6-hexanediol di(meth)acrylate, neopentyl glycol di(meth)acrylate, 1,9-nonanediol di(meth)acrylate, trimethylolpropane tri(meth)acrylate, trimethylolethane tri(meth)acrylate, tetramethylolmethane tri(meth)acrylate, ditrimethylolpropane tetra(meth)acrylate, tetramethylolmethane tetra(meth)acrylate, pentaerythritol tetra(meth)acrylate; dipentaerythritol hexa(meth)acrylate, 1,4-cyclohexanediol dimethacrylate, allyl (meth)acrylate, diallylphthalate, diallyl itaconate, diallyl fumarate, and diallyl maleate; polyallyl ethers of sucrose having from 2 to 8 allyl groups per molecule, polyallyl ethers of pentaerythritol, polyallyl ethers of trimethylolpropane, and mixtures thereof, and/or wherein said at least one polyunsaturated crosslinking monomer (d) is selected from an amphiphilic polyunsaturated macromonomer represented by formula (XI):

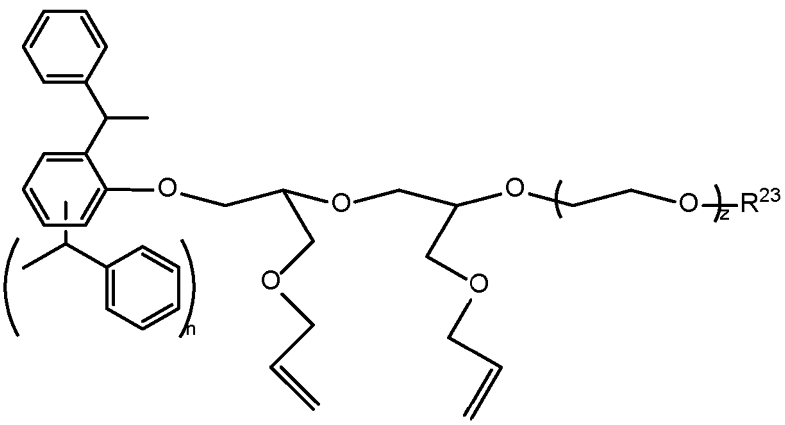

(XI)

wherein n is 1 or 2; z is 4-40, or 5-38, or 10-20; and $R^{23}$ is H, $SO_3^-M^+$ or $PO_3^{2-}$ $M^+$, and M is selected from $Na^+$, $K^+$, and $NH_4^+$.

6. A polymer composition of any of the previous claims, wherein said enzymatically digested starch is selected from potato starch, corn starch, tapioca starch, wheat starch, rice starch, sorghum starch, and mixtures thereof, preferably wherein said enzymatically digested starch is oxidized potato starch.

7. A polymer composition of any of the previous claims, wherein said enzymatically digested starch has a $M_w$ ranging from 10 to 100 kDa, or from 20 to 80 kDa, or from 30 to 70 kDa, or from 55 to 60 kDa.

8. A polymer composition of any of the previous claims, wherein said polyvinyl alcohol has a degree of hydrolysis ranging from 80 to 95%, or from 85 to 90%, or from 87 to 89%.

9. A polymer composition of any of the previous claims, wherein said saturated $C_2$ to $C_{50}$ mono- or di-acid and/or said unsaturated $C_{10}$ to $C_{50}$ mono- or diacid, and mixtures thereof is selected from acetic acid, caproic acid, caprylic acid, pelargonic acid, capric acid, undecanoic acid, lauric acid, myristic acid, palmitic acid, palmitoleic acid, stearic acid, isostearic acid, hydroxy stearic acid, oleic acid, ricinoleic acid, vaccenic acid, linolenic acid, $\alpha$-linolenic acid, $\gamma$-linolenic acid, arachidic acid, gadoleic acid, arachidonic acid, eicosapentaenoic acid, behenic acid, docosahexaenoic acid, lignoceric acid, malonic acid, succinic acid, glutaric acid, adipic acid, pimelic acid, suberic acid, azelaic acid, undecanedioic acid, dodecandioic acid, brassylic acid, thapsic acid japanic acid, phellogenic acid, equisetolic acid, citric acid, isocitric acid, aconitic acid, propane-1,2,3-tricarboxylic acid, agaric acid, trmesic acid, tartronic acid, mesoxalic acid, malic acid, tartaric acid, oxaloacetic acid aspartic acid, dioxosuccinic acid, dimer acids containing 24 to 44 carbon atoms, and mixtures thereof.

10. A polymer composition of any of the previous claims, wherein said monomer composition comprises:

a) from 30 to 40 parts by wt. of an ethylenically unsaturated carboxylic acid monomer selected from acrylic acid, methacrylic acid, and mixtures thereof;
b) from 60 to 70 parts by wt. an alkyl ester of acrylic acid selected from ethyl acrylate, butyl acrylate, and mixtures thereof;

c) from 0 to 5 parts by wt. of an optional associative monomer selected from lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), wherein the polyethoxylated moiety contains from 10 to 60 ethylene oxide units; and
d) from 0 or from 0.1 to 2 parts by wt. of at least one polyunsaturated crosslinking monomer; wherein the sum of a), b), c) and d) in said monomer composition is 100 parts by wt.; and wherein said monomer composition is polymerized by free radical polymerization in the presence of a stabilizer composition comprising:

i) from 10 to 30 parts by wt. of an enzymatically digested potato starch per 100 parts by wt. of said monomer composition;
ii) from 0.15 to 0.5 parts by wt. of PVOH per 100 parts by wt. of said polymerizable monomer composition; and
iii) from 0.05 to 0.1 parts by wt. of at least one saturated $C_2$ to $C_{50}$ mono- or di-acid, and/or unsaturated $C_{10}$ to $C_{50}$ mono- and diacid, and mixtures thereof is selected from acetic acid, caproic acid, caprylic acid, pelargonic acid, capric acid, undecanoic acid, lauric acid, myristic acid, palmitic acid, palmitoleic acid, stearic acid, isostearic acid, oleic acid, $C_{36}$ dimer acid, and mixtures thereof, preferably wherein said saturated $C_2$ to $C_{50}$ mono- or di-acid and/or $C_{10}$ to $C_{50}$ unsaturated mono- or diacid is selected from capric acid, lauric acid, myristic acid, steric acid, oleic acid, and mixtures thereof.

**11.** A cleansing composition comprising a polymer composition of any of the previous claims and at least one surfactant selected from an anionic surfactant, an amphoteric surfactant, a nonionic surfactant, a cationic surfactant, and mixtures thereof.

**12.** A cleansing composition of claim 11, wherein said at least one surfactant is sulfate-free.

**13.** A cleansing composition of claim 11 or 12, wherein said composition is neutralized with an alkaline material to a pH ranging from 4 to 12.

**14.** A cleansing composition of claim 13, wherein said composition is back-acid treated with an acidic material to is at least 0.75 to 7 pH units below the initial base-neutralized pH range of from 4 to 12.

**15.** A method for making a polymer latex composition comprising polymerizing a monomer composition by free radical emulsion polymerization, said monomer composition comprising:

a) from 20 to 60 parts by wt., or from 30 to 50 parts by wt., or from 35 to 40 parts by wt. of at least one ethylenically unsaturated $C_3$-$C_6$ acid group containing monomer;
b) from 40 to 75 parts by wt., or from 50 to 70 parts by wt., or from 60 to 68 parts by wt. of at least one $C_1$ to $C_{30}$ alkyl ester of (meth)acrylic acid;
c) from 0 to 15 parts by wt., or from 0.1 to 10 parts by wt., or from 1 to 5 parts by wt. of at least one monomer selected from an associative monomer, a semi-hydrophobic monomer, and mixtures thereof;
d) from 0 to 10 parts by wt., or from 0.05 to 8 parts by wt., or from 0.1 to 5 parts by wt., or from 0.5 to 3 parts by wt., or from 0.75 to 2 parts by wt. of at least one polyunsaturated crosslinking monomer; wherein the sum of a), b), c) and d) in said monomer mixture is 100 parts by wt., in the presence of a stabilizer composition comprising:

i) from 0.1 to 75 parts by wt., or from 0.5 to 60 parts by wt., or from 1 to 55 parts by wt., or from 5 to 35 parts by wt., or from 10 to 30 parts by wt. of an enzymatically digested starch per 100 parts by wt. of said monomer composition;
ii) from 0.001 to 2 parts by wt., or from 0.005 to 1.5 parts by wt., or from 0.05 to 1 parts by wt., or from 0.1 to 0.75 parts by wt., or from 0.15 to 0.5 parts by wt. of PVOH per 100 parts by wt. of said polymerizable monomer composition; and
iii) from 0.001 to 0.5 parts by wt., or from 0.005 to 0.4 parts by wt., or from 0.01 to 0.3 parts by wt., or from 0.03 to 0.2 parts by wt., or from 0.05 to 0.1 parts by wt. of at least one acid selected from a saturated $C_2$ to $C_{50}$ mono- or di-acid, an unsaturated $C_{10}$ to $C_{50}$ mono- or diacid, and mixtures thereof per 100 parts by wt. of said polymerizable monomer composition;

wherein said at least one associative monomer (c) in said monomer composition is represented by formulas I and/or II:

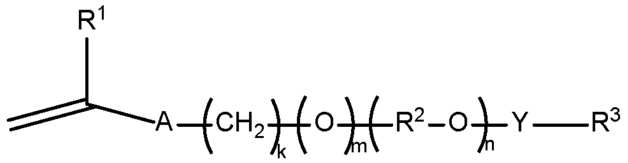

I

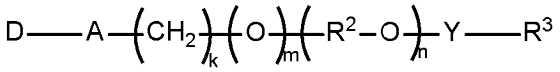

II

wherein $R^1$ is hydrogen or methyl; A is $-CH_2C(O)O-$, $-C(O)O-$, -O-, $-CH_2O-$, -NHC(O)NH-, -C(O)NH-, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to about 30, m is 1; D represents a vinyl or an allyl moiety; $(R^2-O)_n$ is a polyoxyalkylene moiety, which can be a homopolymer, a random copolymer, or a block copolymer of $C_2$-$C_4$ oxyalkylene units, $R^2$ is a divalent alkylene moiety selected from $C_2H_4$, $C_3H_6$, or $C_4H_8$, and combinations thereof; and n is an integer in the range of 2 to 150, or from 10 to 120, or from 15 to 60; Y is $-R^2O-$, $-R^2NH-$, -C(O)-, -C(O)NH-, $-R^2NHC(O)NH-$, or -C(O)NHC(O)-; $R^3$ is a substituted or unsubstituted alkyl selected from a $C_8$-$C_{30}$ linear alkyl, a $C_8$-$C_{30}$ branched alkyl, a $C_8$-$C_{30}$ carbocyclic alkyl, a $C_2$-$C_{30}$ alkyl-substituted phenyl, an aralkyl substituted phenyl, and an aryl-substituted $C_2$-$C_{30}$ alkyl; wherein the $R^3$ alkyl group, aryl group, phenyl group optionally comprises one or more substituents selected from the group consisting of a hydroxyl group, an alkoxyl group, benzyl group styryl group, and a halogen group; and

wherein said at least one semi-hydrophobic monomer (c) in said monomer composition is represented by formulas IV and/or V:

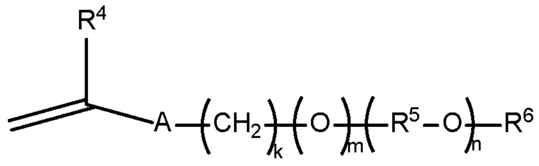

IV

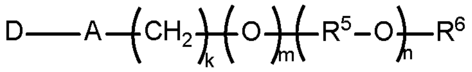

V

wherein $R^4$ is hydrogen or methyl; A is $-CH_2C(O)O-$, $-C(O)O-$, -O-, $-CH_2O-$, -NHC(O)NH-, -C(O)NH-, $-Ar-(CE_2)_z-NHC(O)O-$, $-Ar-(CE_2)_z-NHC(O)NH-$, or $-CH_2CH_2NHC(O)-$; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to about 30, m is 1; $(R^5-O)_n$ is a polyoxyalkylene moiety, which can be a homopolymer, a random copolymer, or a block copolymer of $C_2$-$C_4$ oxyalkylene units, $R^5$ is a divalent alkylene moiety selected from $C_2H_4$, $C_3H_6$, or $C_4H_8$, and combinations thereof; and n is an integer in the ranging from 2 to 150, or from 5 to 120, or from 10 to 60; $R^6$ is selected from hydrogen and a linear or branched $C_1$-$C_4$ alkyl group; and D represents a vinyl or an allyl moiety.

**16.** A method of mitigating the coalescence and/or flocculation of particles in a polymer latex obtained in claim 15 by dosing said latex with an organic acid, an inorganic acid, a reducing agent, and mixtures thereof in an amount ranging from 1.25 to 10 wt.%, or from 2.5 to 7.5 wt.%, or from 3.5 to 5 wt.%, based on the total weight of the emulsion polymer latex.

**17.** A method of claim 16, wherein said organic acid is selected from acetic acid, citric acid, glycolic acid, lactic acid, mandelic acid, hydroxycaproic acid, hydroxycaprylic acid, malic acid, tartaric acid, α-hydroxypropionic acid), salicylic acid, tropic acid, trethocanic acid, β-hydroxypropionic acid, β-hydroxybutyric acid, β-hydroxy β-methylbutyric acid, carnitine, natural fruit acids, caproic acid, caprylic acid, pelargonic acid, capric acid, undecanoic acid, lauric acid, myristic acid, palmitic acid, palmitoleic acid, stearic acid, isostearic acid, hydroxy stearic acid, oleic acid, ricinoleic acid, vaccenic acid, linolenic acid, α-linolenic acid, γ-linolenic acid, arachidic acid, gadoleic acid, arachidonic acid, eicosapentaenoic acid, behenic acid, docosahexaenoic acid, lignoceric acid, malonic acid, succinic acid, glutaric acid, adipic acid, pimelic acid, suberic acid, azelaic acid, undecanedioic acid, dodecandioic acid, brassylic acid, thapsic acid japanic acid, phellogenic acid, equisetolic acid, isocitric acid, aconitic acid, propane-1,2,3-tricarboxylic

acid, agaric acid, trimesic acid, tartronic acid, mesoxalic acid, oxaloacetic acid aspartic acid, dioxosuccinic acid, formamidine sulfinic acid, sodium salt of organic sulfinic acids, hydroxymethanesulfonic acid, erythorbic acid; ascorbic acid; isoascorbic acid, glyceric acid, 2-hydroxy-2-sulfinatoacetic acid, salts thereof, and combinations thereof, and/or wherein said inorganic acid is selected from hydrochloric acid, nitric acid, sulfuric acid, sulfamic acid, phosphoric acid, and mixtures thereof, and/or
wherein said reducing agent is selected from sodium formaldehyde sulfate, transition metals, hydrazine, sodium sulfite, sodium bisulfite, sodium thiosulfate, sodium hydrosulfite, sodium sulfide, sodium hydrosulfide, sodium dithionite, acetone bisulfite; ethanolamine; monosaccharides, disaccharides, oligosaccharides, and oxidized derivatives thereof; and mixtures thereof.

**18.** A method of claim 16 or claim 17, wherein said organic acid, inorganic acid, reducing agent, and mixtures thereof is added to the polymer latex subsequent to the polymerization reaction.

**Patentansprüche**

**1.** Polymerlatexzusammensetzung, die durch Polymerisieren einer Monomerzusammensetzung hergestellt wird, umfassend:

a) von zu 20 bis 60 Gewichtsteile, oder von zu 30 bis 50 Gewichtsteile, oder von zu 35 bis 40 Gewichtsteile mindestens eine ethylenisch ungesättigte $C_3$-$C_6$-Säuregruppe, die Monomer enthält;
b) von zu 40 bis 75 Gewichtsteile, oder von zu 50 bis 70 Gewichtsteile, oder von zu 60 bis 68 Gewichtsteile mindestens ein $C_1$- bis $C_{30}$-Alkylester einer (Meth)acrylsäure;
c) von zu 0 bis 15 Gewichtsteile oder von zu 0,1 bis 10 Gewichtsteile oder von zu 1 bis 5 Gewichtsteile mindestens ein Monomer, das aus einem assoziativen Monomer, einem semihydrophoben Monomer und Mischungen davon ausgewählt ist;
d) von zu 0 bis 10 Gewichtsteile oder von zu 0,05 bis 8 Gewichtsteile oder von zu 0,1 bis 5 Gewichtsteile oder von zu 0,5 bis 3 Gewichtsteile oder von zu 0,75 bis 2 Gewichtsteile mindestens ein mehrfach ungesättigtes Vernetzungsmonomer; wobei die Summe aus a), b), c) und d) in der Monomermischung 100 Gewichtsteile beträgt und wobei die Monomermischung in der Gegenwart einer Stabilisatorzusammensetzung polymerisiert wird, umfassend:

i) von zu 0,1 bis 75 Gewichtsteile oder von zu 0,5 bis 60 Gewichtsteile oder von zu 1 bis 55 Gewichtsteile oder von zu 5 bis 35 Gewichtsteile oder von zu 10 bis 30 Gewichtsteile eine enzymatisch verdaute Stärke pro 100 Gewichtsteile der Monomerzusammensetzung;
ii) von zu 0,001 bis 2 Gewichtsteile oder von zu 0,005 bis 1,5 Gewichtsteile oder von zu 0,05 bis 1 Gewichtsteile oder von zu 0,1 bis 0,75 Gewichtsteile oder von zu 0,15 bis 0,5 Gewichtsteile PVOH pro 100 Gewichtsteile der polymerisierbaren Monomerzusammensetzung; und
iii) von zu 0,001 bis 0,5 Gewichtsteile oder von zu 0,005 bis 0,4 Gewichtsteile oder von zu 0,01 bis 0,3 Gewichtsteile oder von zu 0,03 bis 0,2 Gewichtsteile oder von zu 0,05 bis 0,1 Gewichtsteile mindestens eine Säure, die aus gesättigter $C_2$-bis $C_{50}$-Mono- oder Disäure, einer ungesättigten $C_{10}$- bis $C_{50}$-Mono- oder Disäure und Mischungen davon pro 100 Gewichtsteile der polymerisierbaren Monomerzusammensetzung ausgewählt ist;

wobei das mindestens eine assoziative Monomer (c) in der Monomerzusammensetzung durch Formeln I und/oder II dargestellt wird:

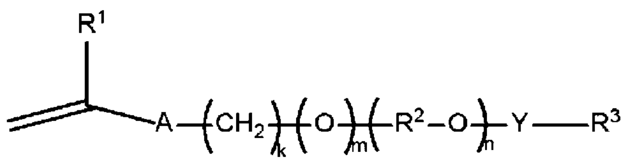

I

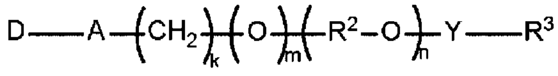

II

wobei $R^1$ Wasserstoff oder Methyl ist;

A $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist; Ar ein zweiwertiges Arylen (z. B. Phenylen) ist; E H oder Methyl ist; z 0 oder 1 ist; k eine ganze Zahl in einem Bereich von 0 bis 30 ist und m 0 oder 1 ist, mit der Maßgabe, dass, wenn k 0 ist, m 0 ist, und wenn k in dem Bereich von 1 bis 30 liegt, m 1 ist; D einen Vinyl- oder Allylrest darstellt; $(R^2-O)_n$ ein Polyoxyalkylenrest ist, der ein Homopolymer, ein zufälliges Copolymer oder ein Blockcopolymer von $C_2$-$C_4$-Oxyalkyleneinheiten sein kann, $R^2$ ein zweiwertiger Alkylenrest ist, der aus $C_2H_4$, $C_3H_6$ oder $C_4H_8$ und Kombinationen davon ausgewählt ist; und n eine ganze Zahl in dem Bereich von 2 bis 150 oder von 10 bis 120 oder von 15 bis 60 ist;

Y $-R^2O-$, $-R^2NH-$, $-C(O)-$, $-C(O)NH-$, $-R^2NHC(O)NH-$ oder $-C(O)NHC(O)-$ ist; $R^3$ ein substituiertes oder unsubstituiertes Alkyl ist, das aus einem linearen $C_8$-$C_{30}$-Alkyl, einem verzweigten $C_8$-$C_{30}$-Alkyl, einem carbocyclischen $C_8$-$C_{30}$-Alkyl, einem substituierten $C_2$-$C_{30}$-Alkyl-Phenyl, einem substituierten Aralkyl-Phenyl und einem arylsubstituierten $C_2$-$C_{30}$-Alkyl ausgewählt ist; wobei $R^3$ Alkylgruppe, Arylgruppe oder Phenylgruppe optional einen oder mehrere Substituenten umfasst, die aus der Gruppe ausgewählt sind, bestehend aus einer Hydroxylgruppe, einer Alkoxylgruppe, Benzylgruppe, Styrylgruppe und einer Halogengruppe; und

wobei das mindestens eine semi-hydrophobe Monomer (c) in der Monomerzusammensetzung durch Formeln IV und/oder V dargestellt wird:

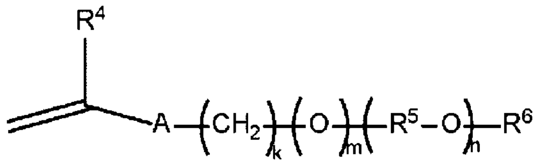
IV

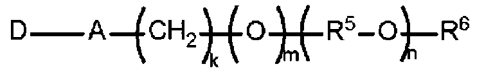
V

wobei $R^4$ Wasserstoff oder Methyl ist; A $CH_2C(O)O-$ $-C(O)O-$, $-O-$, $-CH_2O-$, $-NHC(O)NH-$, $-C(O)NH-$ $-Ar-(CE_2)_z-NHC(O)O-$ , $-Ar-(CE_2)_z-NHC(O)NH-$ oder $-CH_2CH_2NHC(O)-$ ist; Ar ein zweiwertiges Arylen (z. B. Phenylen) ist; E H oder Methyl ist; z 0 oder 1 ist; k eine ganze Zahl in dem Bereich von etwa 0 bis etwa 30 ist und m 0 oder 1 ist, mit der Maßgabe, dass, wenn k 0 ist, m 0 ist, und wenn k in dem Bereich von 1 bis etwa 30 liegt, m 1 ist; $(R^5-O)_n$ ein Polyoxyalkylenrest ist, der ein Homopolymer, ein zufälliges Copolymer oder ein Blockcopolymer von $C_2$-$C_4$-Oxyalkyleneinheiten sein kann, $R^5$ ein zweiwertiger Alkylenrest ist, der aus $C_2H_4$, $C_3H_6$ oder $C_4H_8$ und Kombinationen davon ausgewählt ist; und n eine ganze Zahl in dem Bereich von etwa 2 bis etwa 150 oder von etwa 5 bis etwa 120 oder von etwa 10 bis etwa 60; $R^6$ aus Wasserstoff und einer linearen oder verzweigten $C_1$-$C_4$-Alkylgruppe ausgewählt ist; und D einen Vinyl- oder Allylrest darstellt.

2. Polymerzusammensetzung nach Anspruch 1, wobei das mindestens eine ethylenisch ungesättigte $C_3$-$C_6$-Carbonsäuremonomer (a) in der Monomerzusammensetzung aus Acrylsäure, Methacrylsäure, Itaconsäure, Citraconsäure, Maleinsäure, Maleinsäureanhydrid, Fumarsäure, Crotonsäure, Aconitsäure, 2-Acrylamido-2-methyl-1-propansulfonsäure und Mischungen davon ausgewählt ist, und/oder,
wobei der mindestens eine lineare/verzweigte $C_1$- bis $C_{30}$-Alkylester der (Meth)acrylsäure (b) in der Monomerzusammensetzung aus Methylacrylat, Methylmethacrylat, Ethylacrylat, Ethylmethacrylat, Hydroxyethylacrylat, Hydroxyethylmethacrylat, n-Propylacrylat, n-Propylmethacrylat, Isopropylacrylat, Isopropylmethacrylat, Hydroxypropylacrylat, Hydroxypropylmethacrylat, n-Butylacrylat, n-Butylmethacrylat, Isobutylacrylat, Isobutylmethacrylat, tert-Butylacrylat, tert-Butylmethacrylat, sec-Butylacrylat, sec-Butylmethacrylat, Hydroxybutylacrylat, Hydroxybutylmethacrylat, n-Pentylacrylat, n-Pentylmethacrylat, Neopentylacrylat, Neopentylmethacrylat, Cyclohexylacrylat, Cyclohexylmethacrylat, 2-Hexylacrylat, 2-Hexylmethacrylat, 2-Ethylhexylacrylat, 2-Ethylhexylmethacrylat, n-Octylacrylat, n-Octylmethacrylat, Isooctylacrylat, Isooctylmethacrylat, Decylacrylat, Decylmethacrylat, Isodecylacrylat, Isodecylmethacrylat, Dodecylacrylat, Dodecylmethacrylat, 2-Propylheptylacrylat und 2-Propylheptylmethacrylat, Laurylacrylat, Laurylmethacrylat, Cetylacrylat, Cetylmethacrylat, Stearylacrylat, Stearylmethacrylat, Behenylacrylat, Behenylmethacrylat, Melissylacrylat, Melissylmethacrylat und Mischungen davon ausgewählt ist.

3. Polymerzusammensetzung nach Anspruch 1 oder 2, wobei das mindestens eine assoziative Monomer (c) in der

Monomerzusammensetzung durch Formel III dargestellt wird:

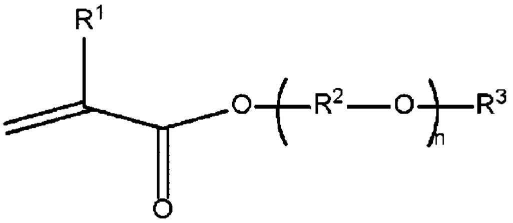

III

wobei $R^1$ Wasserstoff oder Methyl ist; $R^2$ ein zweiwertiger Alkylenrest ist, der unabhängig aus $C_2H_4$, $C_3H_6$ und $C_4H_8$ ausgewählt ist, und n eine ganze Zahl in dem Bereich von 5 bis 60 darstellt, ($R^2$-O) in einer zufälligen oder einer Blockkonfiguration angeordnet werden kann; $R^3$ ein substituiertes oder unsubstituiertes Alkyl ist, das aus einem linearen $C_8$-$C_{30}$-Alkyl, einem verzweigten $C_8$-$C_{30}$-Alkyl, einem carbocyclischen $C_8$-$C_{30}$-Alkyl, einem alkylsubstituierten $C_2$-$C_{30}$-Phenyl, einem aralkylsubstituierten Phenyl und einem arylsubstituierten $C_2$-$C_{30}$-Alkyl ausgewählt ist, wobei die $R^3$-Alkylgruppe, -Arylgruppe, -Phenylgruppe optional einen oder mehrere Substituenten umfasst, die aus der Gruppe ausgewählt sind, bestehend aus einer Hydroxylgruppe, einer Alkoxylgruppe, Benzylgruppe, Styrylgruppe und einer Halogengruppe, oder wobei das mindestens eine assoziative Monomer (c) in der Monomerzusammensetzung aus laurylpolyethoxyliertem Methacrylat (LEM), cetylpolyethoxyliertem Methacrylat (CEM), cetearylpolyethoxyliertem Methacrylat (CSEM), stearylpolyethoxyliertem (Meth)acrylat, arachidylpolyethoxyliertem (Meth)acrylat, behenylpolyethoxyliertem Methacrylat (BEM), cerotylpolyethoxyliertem (Meth)acrylat, montanylpolyethoxyliertem (Meth)acrylat, melissylpolyethoxyliertem (Meth)acrylat, phenylpolyethoxyliertem (Meth)acrylat, nonylphenylpolyethoxyliertem (Meth)acrylat, ω-Tristyrylphenylpolyoxyethylenmethacrylat ausgewählt ist, wobei der polyethoxylierte Rest des Monomers von 2 bis 150, oder von 5 bis 120 oder von 10 bis 60 oder von 15 bis 30 Ethylenoxideinheiten enthält.

4. Polymerzusammensetzung nach einem der vorstehenden Ansprüche, wobei das mindestens eine semi-hydrophobe Monomer (c) in der Monomerzusammensetzung durch Formeln VI und/oder VII dargestellt wird:

$$CH_2=C(R^4)C(O)O-(C_2H_4O)_a(C_3H_6O)_b-H \quad VI$$

$$CH_2=C(R^4)C(O)O-(C_2H_4O)_a(C_3H_6O)_b-CH_3 \quad VII$$

wobei $R^4$ Wasserstoff oder Methyl ist und "a" eine ganze Zahl in dem Bereich von 0 oder 2 bis 120 oder von 5 bis 45 oder von 10 bis 25 ist; und "b" eine ganze Zahl in dem Bereich von 0 bis 120 oder von 2 bis 45 oder von 10 bis 25 ist, mit der Maßgabe, dass "a" und "b" nicht gleichzeitig 0 sein können.

5. Polymerzusammensetzung nach einem der vorstehenden Ansprüche, wobei das mindestens eine mehrfach ungesättigte Vernetzungsmonomer (d) aus einem mehrfach funktionellen (Meth)acrylat, einem Polyallylether, einem amphiphilen mehrfach ungesättigten Makromonomer und Mischungen davon ausgewählt ist, vorzugsweise wobei das mindestens eine mehrfach ungesättigte Vernetzungsmonomer (d) aus Ethylenglycoldi(meth)acrylat, Triethylenglycoldi(meth)acrylat, 1,3-Butylenglycoldi(meth)acrylat, 1,4-Butylenglycoldi(meth)acrylat, 1,6-Hexandioldi(meth)acrylat, Neopentylglycoldi(meth)acrylat, 1,9-Nonandioldi(meth)acrylat, Trimethylolpropantri(meth)acrylat, Trimethylolethantri(meth)acrylat, Tetramethylolmethantri(meth)acrylat, Ditrimethylolpropantetra(meth)acrylat, Tetramethylolmethantetra(meth)acrylat, Pentaerythrittetra(meth)acrylat; Dipentaerythrithexa(meth)acrylat, 1,4-Cyclohexandioldimethacrylat, Allyl(meth)acrylat, Diallylphthalat, Diallylitaconat, Diallylfumarat und Diallylmaleat; Polyallylether von Saccharose, die 2 bis 8 Allylgruppen pro Molekül aufweisen, Polyallylether von Pentaerythrit, Polyallylether von Trimethylolpropan und Mischungen davon ausgewählt ist, und/oder wobei das mindestens eine mehrfach ungesättigte Vernetzungsmonomer (d) aus einem amphiphilen mehrfach ungesättigten Makromonomer ausgewählt ist, das durch Formel (XI) dargestellt wird:

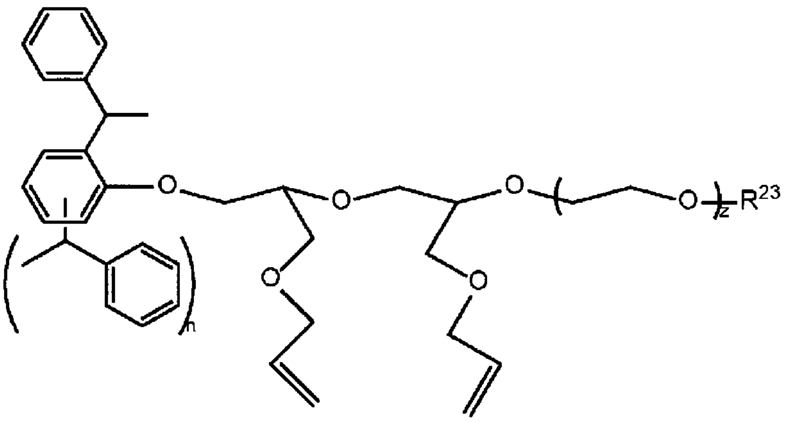

(XI)

wobei n 1 oder 2 ist; z 4-40 oder 5-38 oder 10-20 ist; und $R^{23}$ H, $SO_3^{-}M^{+}$ oder $PO_3^{2-}M^{+}$ ist und M aus $Na^{+}$, $K^{+}$ und $NH4^{+}$ ausgewählt ist.

**6.** Polymerzusammensetzung nach einem der vorstehenden Ansprüche, wobei die enzymatisch verdaute Stärke aus Kartoffelstärke, Maisstärke, Tapiokastärke, Weizenstärke, Reisstärke, Sorghumstärke und Mischungen davon ausgewählt ist, wobei die enzymatisch verdaute Stärke vorzugsweise oxidierte Kartoffelstärke ist.

**7.** Polymerzusammensetzung nach einem der vorstehenden Ansprüche, wobei die enzymatisch verdaute Stärke ein $M_w$ in dem Bereich von 10 bis 100 kDa oder von 20 bis 80 kDa oder von 30 bis 70 kDa oder von 55 bis 60 kDa aufweist.

**8.** Polymerzusammensetzung nach einem der vorstehenden Ansprüche, wobei der Polyvinylalkohol einen Hydrolysegrad in dem Bereich von 80 bis 95 % oder von 85 bis 90 % oder von 87 bis 89 % aufweist.

**9.** Polymerzusammensetzung nach einem der vorstehenden Ansprüche, wobei die gesättigte $C_2$- bis $C_{50}$-Mono- oder Disäure und/oder die ungesättigte $C_{10}$- bis $C_{50}$-Mono- oder Disäure und Mischungen davon aus Essigsäure, Capronsäure, Caprylsäure, Pelargonsäure, Caprinsäure, Undecansäure, Laurinsäure, Myristinsäure, Palmitinsäure, Palmitoleinsäure, Stearinsäure, Isostearinsäure, Hydroxystearinsäure, Ölsäure, Ricinolsäure, Vaccensäure, Linolensäure, $\alpha$-Linolensäure, $\gamma$-Linolensäure, Arachinsäure, Gadoleinsäure, Arachidonsäure, Eicosapentaensäure, Behensäure, Docosahexaensäure, Lignocerinsäure, Malonsäure, Bernsteinsäure, Glutarsäure, Adipinsäure, Pimelinsäure, Korksäure, Azelainsäure, Undecandisäure, Dodecandisäure, Brassylsäure, Thapsinsäure, Japansäure, Phellogensäure, Equisetolsäure, Zitronensäure, Isozitronensäure, Aconitsäure, Propan-1,2,3-tricarbonsäure, Agarinsäure, Trimesinsäure, Tartronsäure, Mesoxalsäure, Äpfelsäure, Weinsäure, Oxalessigsäure, Asparaginsäure, Dioxobernsteinsäure, Dimersäuren, die 24 bis 44 Kohlenstoffatome enthalten, und Mischungen davon ausgewählt ist.

**10.** Polymerzusammensetzung nach einem der vorstehenden Ansprüche, wobei die Monomerzusammensetzung umfasst:

a) von zu 30 bis 40 Gewichtsteile ein ethylenisch ungesättigtes Carbonsäuremonomer, das aus Acrylsäure, Methacrylsäure und Mischungen davon ausgewählt ist;
b) von zu 60 bis 70 Gewichtsteile ein Alkylester der Acrylsäure, der aus Ethylacrylat, Butylacrylat und Mischungen davon ausgewählt ist;
c) von zu 0 bis 5 Gewichtsteile ein optional assoziatives Monomer, das aus laurylpolyethoxyliertem Methacrylat (LEM), cetylpolyethoxyliertem Methacrylat (CEM), cetearylpolyethoxyliertem Methacrylat (CSEM), stearylpolyethoxyliertem (Meth)acrylat, arachidylpolyethoxyliertem (Meth)acrylat, behenylpolyethoxyliertem Methacrylat (BEM) ausgewählt ist, wobei der polyethoxylierte Rest von 10 bis 60 Ethylenoxideinheiten enthält; und
d) von zu 0 oder 0,1 bis 2 Gewichtsteile mindestens ein mehrfach ungesättigtes Vernetzungsmonomer; wobei die Summe aus a), b), c) und d) in der Monomerzusammensetzung 100 Gewichtsteile beträgt; und wobei die Monomerzusammensetzung durch radikalische Polymerisation in der Gegenwart einer Stabilisatorzusammensetzung polymerisiert wird, umfassend:

i) von zu 10 bis 30 Gewichtsteile eine enzymatisch verdaute Kartoffelstärke pro 100 Gewichtsteile der Monomerzusammensetzung;

ii) von zu 0,15 bis 0,5 Gewichtsteile PVOH pro 100 Gewichtsteile der polymerisierbaren Monomerzusammensetzung; und

iii) von zu 0,05 bis 0,1 Gewichtsteile mindestens eine gesättigte $C_2$- bis $C_{50}$-Mono- oder Disäure und/oder ungesättigte $C_{10}$- bis $C_{50}$-Mono- und Disäure und Mischungen davon aus Essigsäure, Capronsäure, Caprylsäure, Pelargonsäure, Caprinsäure, Undecansäure, Laurinsäure, Myristinsäure, Palmitinsäure, Palmitoleinsäure, Stearinsäure, Isostearinsäure, Ölsäure, $C_{36}$-Dimersäure und Mischungen davon ausgewählt ist, vorzugsweise wobei die gesättigte $C_2$- bis $C_{50}$-Mono- oder Disäure und/oder ungesättigte $C_{10}$- bis $C_{50}$-Mono- oder Disäure aus Caprinsäure, Laurinsäure, Myristinsäure, Stearinsäure, Ölsäure und Mischungen davon ausgewählt ist.

**11.** Reinigungszusammensetzung, umfassend eine Polymerzusammensetzung nach einem der vorstehenden Ansprüche und mindestens ein Tensid, das aus einem anionischen Tensid, einem amphoteren Tensid, einem nichtionischen Tensid, einem kationischen Tensid und Mischungen davon ausgewählt ist.

**12.** Reinigungszusammensetzung nach Anspruch 11, wobei das mindestens eine Tensid sulfatfrei ist.

**13.** Reinigungszusammensetzung nach Anspruch 11 oder 12, wobei die Zusammensetzung mit einem alkalischen Material auf einen pH-Wert in dem Bereich von 4 bis 12 neutralisiert ist.

**14.** Reinigungszusammensetzung nach Anspruch 13, wobei die Zusammensetzung mit einem sauren Material rücksäuremodifiziert wird, um mindestens 0,75 bis 7 pH-Wert-Einheiten unter dem anfänglichen basenneutralisierten pH-Wert-Bereich von 4 bis 12 zu liegen.

**15.** Verfahren zum Herstellen einer Polymerlatexzusammensetzung, umfassend das Polymerisieren einer Monomerzusammensetzung durch radikalische Emulsionspolymerisation, die Monomerzusammensetzung umfassend:

a) von zu 20 bis 60 Gewichtsteile, oder von zu 30 bis 50 Gewichtsteile, oder von zu 35 bis 40 Gewichtsteile mindestens eine ethylenisch ungesättigte $C_3$-$C_6$-Säuregruppe, die Monomer enthält;

b) von zu 40 bis 75 Gewichtsteile, oder von zu 50 bis 70 Gewichtsteile, oder von zu 60 bis 68 Gewichtsteile mindestens ein $C_1$- bis $C_{30}$-Alkylester einer (Meth)acrylsäure;

c) von zu 0 bis 15 Gewichtsteile oder von zu 0,1 bis 10 Gewichtsteile oder von zu 1 bis 5 Gewichtsteile mindestens ein Monomer, das aus einem assoziativen Monomer, einem semihydrophoben Monomer und Mischungen davon ausgewählt ist;

d) von zu 0 bis 10 Gewichtsteile oder von zu 0,05 bis 8 Gewichtsteile oder von zu 0,1 bis 5 Gewichtsteile oder von zu 0,5 bis 3 Gewichtsteile oder von zu 0,75 bis 2 Gewichtsteile mindestens ein mehrfach ungesättigtes Vernetzungsmonomer; wobei die Summe aus a), b), c) und d) in der Monomermischung 100 Gewichtsteile beträgt, in der Gegenwart einer Stabilisatorzusammensetzung, umfassend:

i) von zu 0,1 bis 75 Gewichtsteile oder von zu 0,5 bis 60 Gewichtsteile oder von zu 1 bis 55 Gewichtsteile oder von zu 5 bis 35 Gewichtsteile oder von zu 10 bis 30 Gewichtsteile eine enzymatisch verdaute Stärke pro 100 Gewichtsteile der Monomerzusammensetzung;

ii) von zu 0,001 bis 2 Gewichtsteile oder von zu 0,005 bis 1,5 Gewichtsteile oder von zu 0,05 bis 1 Gewichtsteile oder von zu 0,1 bis 0,75 Gewichtsteile oder von zu 0,15 bis 0,5 Gewichtsteile PVOH pro 100 Gewichtsteile der polymerisierbaren Monomerzusammensetzung; und

iii) von zu 0,001 bis 0,5 Gewichtsteile oder von zu 0,005 bis 0,4 Gewichtsteile oder von zu 0,01 bis 0,3 Gewichtsteile oder von zu 0,03 bis 0,2 Gewichtsteile oder von zu 0,05 bis 0,1 Gewichtsteile mindestens eine Säure, die aus gesättigter $C_2$- bis $C_{50}$-Mono- oder Disäure, einer ungesättigten $C_{10}$- bis $C_{50}$-Mono- oder Disäure und Mischungen davon pro 100 Gewichtsteile der polymerisierbaren Monomerzusammensetzung ausgewählt sind;

wobei das mindestens eine assoziative Monomer (c) in der Monomerzusammensetzung durch Formeln I und/oder II dargestellt wird:

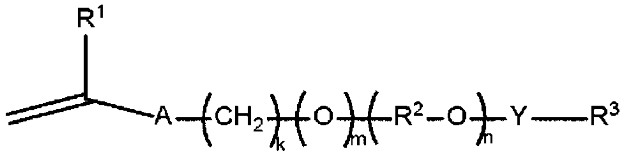

I

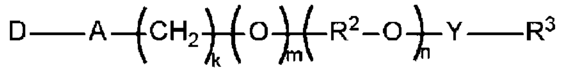 II

wobei $R^1$ Wasserstoff oder Methyl ist;

A $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist; Ar ein zweiwertiges Arylen (z. B. Phenylen) ist; E H oder Methyl ist; z 0 oder 1 ist; k eine ganze Zahl in dem Bereich von 0 bis 30 ist und m 0 oder 1 ist, mit der Maßgabe, dass, wenn k 0 ist, m 0 ist, und wenn k in dem Bereich von 1 bis etwa 30 liegt, m 1 ist; D einen Vinyl- oder Allylrest darstellt; $(R^2-O)_n$ ein Polyoxyalkylenrest ist, der ein Homopolymer, ein zufälliges Copolymer oder ein Blockcopolymer von $C_2$-$C_4$-Oxyalkyleneinheiten sein kann, $R^2$ ein zweiwertiger Alkylenrest ist, der aus $C_2H_4$, $C_3H_6$ oder $C_4H_8$ und Kombinationen davon ausgewählt ist; und n eine ganze Zahl in dem Bereich von 2 bis 150 oder von 10 bis 120 oder von 15 bis 60 ist;

Y $-R^2O-$ $-R^2NH-$, $-C(O)-$, $-C(O)NH-$, $-R^2NHC(O)NH-$ oder $-C(O)NHC(O)-$ ist; $R^3$ ein substituiertes oder unsubstituiertes Alkyl ist, das aus einem linearen $C_8$-$C_{30}$-Alkyl, einem verzweigten $C_3$-$C_{30}$-Alkyl, einem carbocyclischen $C_8$-$C_{30}$-Alkyl, einem substituierten $C_2$-$C_{30}$-Alkyl-Phenyl, einem substituierten Aralkyl-Phenyl und einem arylsubstituierten $C_2$-$C_{30}$-Alkyl ausgewählt ist; wobei $R^3$ Alkylgruppe, Arylgruppe oder Phenylgruppe optional einen oder mehrere Substituenten umfasst, die aus der Gruppe ausgewählt sind, bestehend aus einer Hydroxylgruppe, einer Alkoxylgruppe, Benzylgruppe, Styrylgruppe und einer Halogengruppe; und

wobei das mindestens eine semi-hydrophobe Monomer (c) in der Monomerzusammensetzung durch Formeln IV und/oder V dargestellt wird:

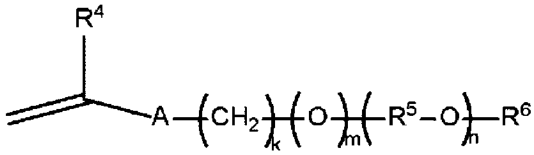 IV

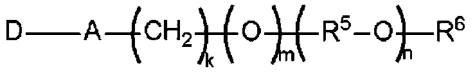 V

wobei $R^4$ Wasserstoff oder Methyl ist;
A $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$ oder $-CH_2CH_2NHC(O)-$ ist; Ar ein zweiwertiges Arylen (z. B. Phenylen) ist; E H oder Methyl ist; z 0 oder 1 ist; k eine ganze Zahl in dem Bereich von 0 bis 30 ist und m 0 oder 1 ist, mit der Maßgabe, dass, wenn k 0 ist, m 0 ist, und wenn k in dem Bereich von 1 bis etwa 30 liegt, m 1 ist; $(R^5-O)_n$ ein Polyoxyalkylenrest ist, der ein Homopolymer, ein zufälliges Copolymer oder ein Blockcopolymer von $C_2$-$C_4$-Oxyalkyleneinheiten sein kann, $R^5$ ein zweiwertiger Alkylenrest ist, der aus $C_2H_4$, $C_3H_6$ oder $C_4H_8$ und Kombinationen davon ausgewählt ist; und n eine ganze Zahl in dem Bereich von 2 bis 150 oder von 5 bis 120 oder von 10 bis 60 ist; $R^6$ aus Wasserstoff und einer linearen oder verzweigten $C_1$-$C_4$-Alkylgruppe ausgewählt ist; und D einen Vinyl- oder Allylrest darstellt.

**16.** Verfahren zum Vermindern der Koaleszenz und/oder der Ausflockung von Partikeln in einem Polymerlatex, der nach Anspruch 15 erhalten wird, durch Versetzen des Latex mit einer organischen Säure, einer anorganischen Säure, einem Reduktionsmittel und Mischungen davon in einer Menge in dem Bereich von 1,25 bis 10 Gew.-% oder von 2,5 bis 7,5 Gew.-% oder von 3,5 bis 5 Gew.-%, basierend auf dem Gesamtgewicht des Emulsionspolymerlatex.

**17.** Verfahren nach Anspruch 16, wobei die organische Säure aus Essigsäure, Zitronensäure, Glycolsäure, Milchsäure, Mandelsäure, Hydroxycapronsäure, Hydroxycaprylsäure, Äpfelsäure, Weinsäure, $\alpha$-Hydroxypropionsäure), Salicylsäure, Tropicinsäure, Trethocansäure, $\beta$-Hydroxypropionsäure, $\beta$-Hydroxybuttersäure, $\beta$-Hydroxy-$\beta$-methylbuttersäure, Carnitin, natürlichen Fruchtsäuren, Capronsäure, Caprylsäure, Pelargonsäure, Caprinsäure, Undecansäure, Laurinsäure, Myristinsäure, Palmitinsäure, Palmitoleinsäure, Stearinsäure, Isostearinsäure, Hydroxystearinsäure, Ölsäure, Ricinolsäure, Vaccensäure, Linolensäure, $\alpha$-Linolensäure, $\gamma$-Linolensäure, Arachinsäure, Gadoleinsäure, Arachidonsäure, Eicosapentaensäure, Behensäure, Docosahexaensäure, Lignocerinsäure, Malonsäure, Bernsteinsäure, Glutarsäure, Adipinsäure, Pimelinsäure, Korksäure, Azelainsäure, Undecandisäure, Dodecandisäure, Brassylsäure, Thapsinsäure, Japansäure, Phellogensäure, Equisetolsäure, Isocitronensäure, Aconitsäure, Propan-1,2,3-tricarbonsäure, Agarinsäure, Trimesinsäure, Tartronsäure, Mesoxalsäure, Oxalessigsäure, Asparaginsäure, Dioxobernsteinsäure, Formamidinsulfinsäure, Natriumsalz organischen Sulfinsäuren, Hydroxymethansulfonsäure, Erythorbinsäure; Ascorbinsäure; Isoascorbinsäure, Glycerinsäure, 2-Hydroxy-2-sulfinatoessigsäure, Salze davon und Kombinationen davon ausgewählt ist, und/oder wobei die anorganische Säure aus Salzsäure, Salpetersäure, Schwefelsäure, Sulfaminsäure, Phosphorsäure und Mischungen davon ausgewählt ist, und/oder
wobei das Reduktionsmittel aus Natriumformaldehydsulfat, Übergangsmetallen, Hydrazin, Natriumsulfit, Natriumbisulfit, Natriumthiosulfat, Natriumhydrogensulfit, Natriumsulfid, Natriumhydrogensulfid, Natriumdithionit und Acetonbisulfit; Ethanolamin; Monosacchariden, Disacchariden, Oligosacchariden und oxidierten Derivaten davon; und Mischungen davon ausgewählt ist.

**18.** Verfahren nach Anspruch 16 oder 17, wobei die organische Säure, die anorganische Säure, das Reduktionsmittel und Mischungen davon dem Polymerlatex im Anschluss an die Polymerisationsreaktion zugesetzt werden.

## Revendications

**1.** Composition de latex polymère préparée par polymérisation d'une composition de monomères comprenant :

a) de 20 à 60 parties en poids, ou de 30 à 50 parties en poids, ou de 35 à 40 parties en poids d'au moins un monomère à teneur en groupe acide en $C_3$-$C_6$ à insaturation éthylénique ;
b) de 40 à 75 parties en poids, ou de 50 à 70 parties en poids, ou de 60 à 68 parties en poids d'au moins un ester d'alkyle en $C_1$ à $C_{30}$ d'acide (méth)acrylique ;
c) de 0 à 15 parties en poids, ou de 0,1 à 10 parties en poids, ou de 1 à 5 parties en poids d'au moins un monomère choisi parmi un monomère associatif, un monomère semi-hydrophobe, et leurs mélanges ;
d) de 0 à 10 parties en poids, ou de 0,05 à 8 parties en poids, ou de 0,1 à 5 parties en poids, ou de 0,5 à 3 parties en poids, ou de 0,75 à 2 parties en poids d'au moins un monomère de réticulation polyinsaturé ; dans laquelle la somme de a), b), c) et d) dans ledit mélange de monomères est de 100 parties en poids, et dans laquelle ledit mélange de monomères est polymérisé en présence d'une composition stabilisante comprenant :

i) de 0,1 à 75 parties en poids, ou de 0,5 à 60 parties en poids, ou de 1 à 55 parties en poids, ou de 5 à 35 parties en poids, ou de 10 à 30 parties en poids d'un amidon digéré par voie enzymatique pour 100 parties en poids de ladite composition de monomères ;
ii) de 0,001 à 2 parties en poids, ou de 0,005 à 1,5 partie en poids, ou de 0,05 à 1 partie en poids, ou de 0,1 à 0,75 partie en poids, ou de 0,15 à 0,5 partie en poids de PVOH pour 100 parties en poids de ladite composition de monomères polymérisable ; et
iii) de 0,001 à 0,5 partie en poids, ou de 0,005 à 0,4 partie en poids, ou de 0,01 à 0,3 partie en poids, ou de 0,03 à 0,2 partie en poids, ou de 0,05 à 0,1 partie en poids d'au moins un acide choisi parmi un mono- ou diacide saturé en $C_2$ à $C_{50}$, un mono- ou diacide insaturé en $C_{10}$ à $C_{50}$, et leurs mélanges, pour 100 parties en poids de ladite composition de monomères polymérisable ;

dans laquelle ledit au moins un monomère associatif (c) dans ladite composition de monomères est représenté par les formules I et/ou II :

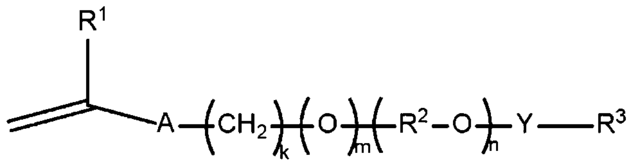
I

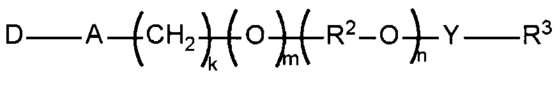
II

où $R^1$ est hydrogène ou méthyle ; A est $-CH_2C(O)O-$, $-C(O)O-$, $-O-$, $-CH_2O-$, $-NHC(O)NH-$, $-C(O)NH-$, $-Ar-(CE_2)_z-NHC(O)O-$, $-Ar-(CE_2)_z-NHC(O)NH-$, ou $-CH_2CH_2NHC(O)-$ ; Ar est un arylène divalent (par exemple phénylène) ; E est H ou méthyle ; z vaut 0 ou 1 ; k est un nombre entier compris entre 0 et 30, et m vaut 0 ou 1, à condition que lorsque k vaut 0, m vaille 0, et lorsque k est compris entre 1 et 30, m vaille 1 ; D représente un groupement vinyle ou allyle ; $(R^2-O)_n$ est un groupement polyoxyalkylène, qui peut être un

homopolymère, un copolymère aléatoire, ou un copolymère à blocs de motifs oxyalkylène en $C_2$-$C_4$, $R^2$ est un groupement alkylène divalent choisi parmi $C_2H_4$, $C_3H_6$, ou $C_4H_8$, et leurs combinaisons ; et n est un nombre entier compris dans la plage de 2 à 150, ou de 10 à 120, ou de 15 à 60 ; Y est -$R^2$O-, -$R^2$NH-, -C(O)-, -C(O)NH-, -$R^2$NHC(O)NH-, ou -C(O)NHC(O)- ; $R^3$ est un alkyle substitué ou non substitué choisi parmi un alkyle linéaire en $C_8$-$C_{30}$, un alkyle ramifié en $C_8$-$C_{30}$, un alkyle carbocyclique en $C_8$-$C_{30}$, un phényle substitué par alkyle en $C_2$-$C_{30}$, un phényle substitué par aralkyle, et un alkyle en $C_2$-$C_{30}$ substitué par aryle ; où le groupe alkyle, groupe aryle, groupe phényle $R^3$ comprend facultativement un ou plusieurs substituants choisis dans le groupe constitué d'un groupe hydroxyle, d'un groupe alcoxyle, d'un groupe benzyle, d'un groupe styryle, et d'un groupe halogène ; et

dans laquelle ledit au moins un monomère semi-hydrophobe (c) dans ladite composition de monomères est représenté par les formules IV et/ou V :

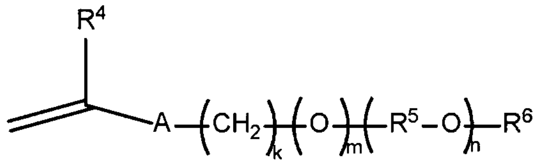

IV

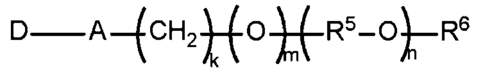

V

où $R^4$ est hydrogène ou méthyle ; A est -$CH_2$C(O)O-, -C(O)O-, -O-, - $CH_2$O-, -NHC(O)NH-, -C(O)NH-, -Ar-$(CE_2)_z$-NHC(O)O-, -Ar-$(CE_2)_z$-NHC(O)NH-, ou -$CH_2CH_2$NHC(O)- ; Ar est un arylène divalent (par exemple phénylène) ; E est H ou méthyle ; z vaut 0 ou 1 ; k est un nombre entier compris dans la plage d'environ 0 à environ 30, et m vaut 0 ou 1, à condition que lorsque k vaut 0, m vaille 0, et lorsque k est compris dans la plage de 1 à environ 30, m vaille 1 ; $(R^5\text{-O})_n$ est un groupement polyoxyalkylène, qui peut être un homopolymère, un copolymère aléatoire, ou un copolymère à blocs de motifs oxyalkylène en $C_2$-$C_4$, $R^5$ est un groupement alkylène divalent choisi parmi $C_2H_4$, $C_3H_6$, ou $C_4H_8$, et leurs combinaisons ; et n est un nombre entier compris dans la plage allant d'environ 2 à environ 150, ou d'environ 5 à environ 120, ou d'environ 10 à environ 60 ; $R^6$ est choisi parmi hydrogène et un groupe alkyle en $C_1$-$C_4$ linéaire ou ramifié ; et D représente un groupement vinyle ou allyle.

**2.** Composition de polymères selon la revendication 1, dans laquelle ledit au moins un monomère d'acide carboxylique en $C_3$-$C_6$ à insaturation éthylénique (a) dans ladite composition de monomères est choisi parmi acide acrylique, acide méthacrylique, acide itaconique, acide citraconique, acide maléique, anhydride maléique, acide fumarique, acide crotonique, acide aconitique, acide 2-acrylamido-2-méthyl-1-propanesulfonique, et leurs mélanges, et/ou,

dans laquelle ledit au moins un ester d'alkyle linéaire/ramifié en $C_1$ à $C_{30}$ d'acide (méth)acrylique (b) dans ladite composition de monomères est choisi parmi acrylate de méthyle, méthacrylate de méthyle, acrylate d'éthyle, méthacrylate d'éthyle, acrylate d'hydroxyéthyle, méthacrylate d'hydroxyéthyle, acrylate de n-propyle, méthacrylate de n-propyle, acrylate d'isopropyle, méthacrylate d'isopropyle, acrylate d'hydroxypropyle, méthacrylate d'hydroxypropyle, acrylate de n-butyle, méthacrylate de n-butyle, acrylate d'isobutyle, méthacrylate d'isobutyle, acrylate de tert-butyle, méthacrylate de tert-butyle, acrylate de sec-butyle, méthacrylate de sec-butyle, acrylate d'hydroxybutyle, méthacrylate d'hydroxybutyle, acrylate de n-pentyle, méthacrylate de n-pentyle, acrylate de néopentyle, méthacrylate de néopentyle, acrylate de cyclohexyle, méthacrylate de cyclohexyle, acrylate de 2-hexyle, méthacrylate de 2-hexyle, acrylate de 2-éthylhexyle, méthacrylate de 2-éthylhexyle, acrylate de n-octyle, méthacrylate de n-octyle, acrylate d'isooctyle, méthacrylate d'isooctyle, acrylate de décyle, méthacrylate de décyle, acrylate d'isodécyle, méthacrylate d'isodécyle, acrylate de dodécyle, méthacrylate de dodécyle, acrylate de 2-propylheptyle et méthacrylate de 2-propylheptyle, acrylate de lauryle, méthacrylate de lauryle, acrylate de cétyle, méthacrylate de cétyle, acrylate de stéaryle, méthacrylate de stéaryle, acrylate de béhényle, méthacrylate de béhényle, acrylate de mélissyle, méthacrylate de mélissyle, et leurs mélanges.

**3.** Composition de polymère selon la revendication 1 ou la revendication 2, dans laquelle ledit au moins un monomère associatif (c) dans ladite composition de monomères est représenté par la formule III :

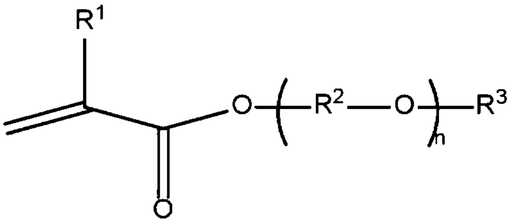

III

où $R^1$ est hydrogène ou méthyle ; $R^2$ est un groupement alkylène divalent choisi indépendamment parmi $C_2H_4$, $C_3H_6$, et $C_4H_8$, et n représente un entier compris dans une plage allant de 5 à 60, ($R^2$-O) peut être agencé selon une configuration aléatoire ou à blocs ; $R^3$ est un alkyle substitué ou non substitué choisi parmi un alkyle linéaire en $C_8$-$C_{30}$, un alkyle ramifié en $C_8$-$C_{30}$, un alkyle carbocyclique en $C_8$-$C_{30}$, un phényle substitué par alkyle en $C_2$-$C_{30}$, un phényle substitué par aralkyle, et un alkyle en $C_2$-$C_{30}$ substitué par aryle, où le groupe alkyle, groupe aryle, groupe phényle $R^3$ comprend facultativement un ou plusieurs substituants choisis dans le groupe constitué d'un groupe hydroxyle, d'un groupe alcoxyle, d'un groupe benzyle, d'un groupe styryle, et d'un groupe halogène, ou dans laquelle ledit au moins un monomère associatif (c) dans ladite composition de monomères est choisi parmi méthacrylate de lauryle polyéthoxylé (LEM), méthacrylate de cétyle polyéthoxylé (CEM), méthacrylate de cétéaryle polyéthoxylé (CSEM), (méth)acrylate de stéaryle polyéthoxylé, (méth)acrylate d'arachidyle polyéthoxylé, méthacrylate de béhényle polyéthoxylé (BEM), (méth)acrylate de cérotyle polyéthoxylé, (méth)acrylate de montanyle polyéthoxylé, (méth)acrylate de mélissyle polyéthoxylé, (méth)acrylate de phényle polyéthoxylé, (méth)acrylate de nonylphényle polyéthoxylé, méthacrylate de ω-tristyrylphényle polyéthoxylé, où le groupement polyéthoxylé du monomère contient de 2 à 150, ou de 5 à 120, ou de 10 à 60, ou de 15 à 30 motifs oxyde d'éthylène.

**4.** Composition de polymère selon l'une quelconque des revendications précédentes, dans laquelle ledit au moins un monomère semi-hydrophobe (c) dans ladite composition de monomères est représenté par les formules VI et/ou VII :

$$CH_2=C(R^4)C(O)O-(C_2H_4O)_a(C_3H_6O)_b-H \qquad VI$$

$$CH_2=C(R^4)C(O)O-(C_2H_4O)_a(C_3H_6O)_b-CH_3 \qquad VII$$

où $R^4$ est hydrogène ou méthyle, et « a » est un nombre entier compris dans la plage allant de 0 ou 2 à 120, ou de 5 à 45, ou de 10 à 25 ; et « b » est un nombre entier compris dans la plage allant de 0 à 120, ou de 2 à 45, ou de 10 à 25, à condition que « a » et « b » ne puissent pas être 0 en même temps.

**5.** Composition de polymère selon l'une quelconque des revendications précédentes, dans laquelle ledit au moins un monomère de réticulation polyinsaturé (d) est choisi parmi un (méth)acrylate polyfonctionnel, un éther polyallylique, un macromonomère polyinsaturé amphiphile, et leurs mélanges, de préférence, dans laquelle ledit au moins un monomère de réticulation polyinsaturé (d) est choisi parmi di(méth)acrylate d'éthylène glycol, di(méth)acrylate de triéthylène glycol, di(méth)acrylate de 1,3-butylène glycol, di(méth)acrylate de 1,4-butylène glycol, di(méth)acrylate de 1,6-hexanediol, di(méth)acrylate de néopentylglycol, di(méth)acrylate de 1,9-nonanediol, tri(méth)acrylate de triméthylolpropane, tri(méth)acrylate de triméthyloléthane, tri(méth)acrylate de tétraméthylolméthane, tétra(méth) acrylate de ditriméthylolpropane, tétra(méth)acrylate de tétraméthylolméthane, tétra(méth)acrylate de pentaérythritol ; hexa(méth)acrylate de dipentaérythritol, diméthacrylate de 1,4-cyclohexanediol, (méth)acrylate d'allyle, diallylphthalate, itaconate de diallyle, fumarate de diallyle, et maléate de diallyle ; éthers polyallyliques de saccharose ayant de 2 à 8 groupes allyle par molécule, éthers polyallyliques de pentaérythritol, éthers polyallyliques de triméthylolpropane, et leurs mélanges, et/ou dans laquelle ledit au moins un monomère de réticulation polyinsaturé (d) est choisi parmi un macromonomère polyinsaturé amphiphile représenté par la formule (XI) :

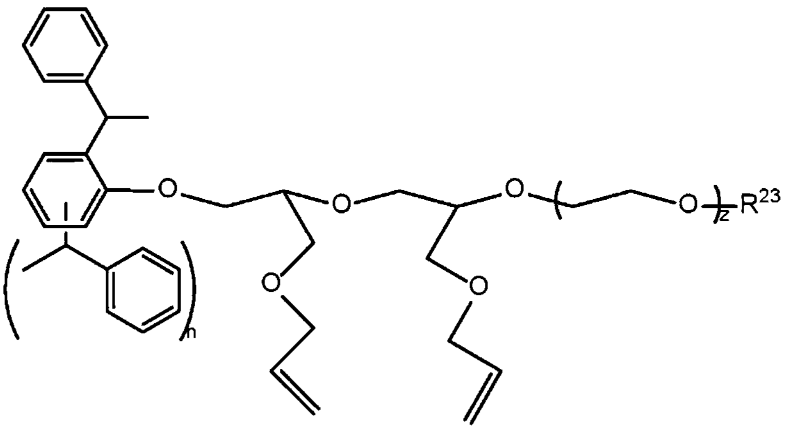

(XI)

où n vaut 1 ou 2 ; z vaut de 4 à 40, ou de 5 à 38, ou de 10 à 20 ; et $R^{23}$ est H, $SO_3^-M^+$ ou $PO_3^{2-}M^+$, et M est choisi parmi $Na^+$, $K^+$, et $NH4^+$.

6. Composition de polymère selon l'une quelconque des revendications précédentes, dans laquelle ledit amidon digéré par voie enzymatique est choisi parmi amidon de pomme de terre, amidon de maïs, amidon de tapioca, amidon de blé, amidon de riz, amidon de sorgho, et leurs mélanges, de préférence dans laquelle ledit amidon digéré par voie enzymatique est l'amidon de pomme de terre oxydé.

7. Composition de polymère selon l'une quelconque des revendications précédentes, dans laquelle ledit amidon digéré par voie enzymatique a une $M_w$ comprise dans la plage allant de 10 à 100 kDa, ou de 20 à 80 kDa, ou de 30 à 70 kDa, ou de 55 à 60 kDa.

8. Composition de polymère selon l'une quelconque des revendications précédentes, dans laquelle ledit alcool polyvinylique a un degré d'hydrolyse compris dans la plage allant de 80 à 95 %, ou de 85 à 90 %, ou de 87 à 89 %.

9. Composition de polymère selon l'une quelconque des revendications précédentes, dans laquelle ledit mono- ou di-acide saturé en $C_2$ à $C_{50}$ et/ou ledit mono- ou di-acide insaturé en $C_{10}$ à $C_{50}$, et leurs mélanges, est choisi parmi acide acétique, acide caproïque, acide caprylique, acide pélargonique, acide caprique, acide undécanoïque, acide laurique, acide myristique, acide palmitique, acide palmitoléique, acide stéarique, acide isostéarique, acide hydroxystéarique, acide oléique, acide ricinoléique, acide vaccénique, acide linolénique, acide $\alpha$-linolénique, acide $\gamma$-linolénique, acide arachidique, acide gadoléique, acide arachidonique, acide eicosapentaénoïque, acide béhénique, acide docosahexaénoïque, acide lignocérique, acide malonique, acide succinique, acide glutarique, acide adipique, acide pimélique, acide subérique, acide azélaïque, acide undécanedioïque, acide dodécandioïque, acide brassylique, acide thapsique, acide japanique, acide phellogénique, acide équisétolique, acide citrique, isocitrique, acide aconitique, acide propane-1,2,3-tricarboxylique, acide agarique, acide trimésique, acide tartronique, acide mésoxalique, acide malique, acide tartrique, acide oxaloacétique, acide aspartique, acide dioxosuccinique, acides dimères contenant de 24 à 44 atomes de carbone, et leurs mélanges.

10. Composition de polymère selon l'une quelconque des revendications précédentes, dans laquelle ladite composition de monomères comprend :

a) de 30 à 40 parties en poids d'un monomère acide carboxylique à insaturation éthylénique choisi parmi acide acrylique, acide méthacrylique, et leurs mélanges ;
b) de 60 à 70 parties en poids d'un ester d'alkyle d'acide acrylique choisi parmi acrylate d'éthyle, acrylate de butyle, et leurs mélanges ;
c) de 0 à 5 parties en poids d'un monomère associatif facultatif choisi parmi méthacrylate de lauryle polyéthoxylé (LEM), méthacrylate de cétyle polyéthoxylé (CEM), méthacrylate de cétéaryle polyéthoxylé (CSEM), (méth)acrylate de stéaryle polyéthoxylé, (méth)acrylate d'arachidyle polyéthoxylé, méthacrylate de béhényle polyéthoxylé (BEM), dans laquelle le groupement polyéthoxylé contient de 10 à 60 motifs oxyde d'éthylène ; et
d) de 0 ou de 0,1 à 2 parties en poids d'au moins un monomère de réticulation polyinsaturé ; dans laquelle la somme de a), b), c) et d) dans ladite composition de monomères est de 100 parties en poids ; et dans laquelle ladite composition de monomères est polymérisée par polymérisation radicalaire en présence d'une composition stabilisante comprenant :

i) de 10 à 30 parties en poids d'un amidon de pomme de terre digéré par voie enzymatique pour 100 parties en poids de ladite composition de monomères ;
ii) de 0,15 à 0,5 partie en poids de PVOH pour 100 parties en poids de ladite composition de monomères polymérisable ; et
iii) de 0,05 à 0,1 partie en poids d'au moins un mono- ou di-acide saturé en C2 à C50 et/ou mono- ou di-acide insaturé en C10 à C50, et leurs mélanges, choisi parmi acide acétique, acide caproïque, acide caprylique, acide pélargonique, acide caprique, acide undécanoïque, acide laurique, acide myristique, acide palmitique, acide palmitoléique, acide stéarique, acide isostéarique, acide oléique, acide dimère en $C_{36}$, et leurs mélanges, de préférence dans laquelle ledit mono- ou di-acide saturé en $C_2$ à $C_{50}$ et/ou mono- ou di-acide insaturé en $C_{10}$ à $C_{50}$ est choisi parmi acide caprique, acide laurique, acide myristique, acide stéarique, acide oléique, et leurs mélanges.

**11.** Composition nettoyante comprenant une composition de polymère selon l'une quelconque des revendications précédentes et au moins un agent tensioactif choisi parmi un agent tensioactif anionique, un agent tensioactif amphotère, un agent tensioactif non ionique, un agent tensioactif cationique, et leurs mélanges.

**12.** Composition nettoyante selon la revendication 11, dans laquelle ledit au moins un agent tensioactif est exempt de sulfate.

**13.** Composition nettoyante selon la revendication 11 ou 12, dans laquelle ladite composition est neutralisée avec une matière alcaline à un pH compris dans la plage allant de 4 à 12.

**14.** Composition nettoyante selon la revendication 13, dans laquelle ladite composition est traitée par rétro-acidification avec une matière acide est inférieure d'au moins 0,75 à 7 unités de pH à la plage de pH initiale neutralisée par base allant de 4 à 12.

**15.** Procédé de préparation d'une composition de latex polymère comprenant la polymérisation d'une composition de monomères par polymérisation radicalaire en émulsion, ladite composition de monomères comprenant :

a) de 20 à 60 parties en poids, ou de 30 à 50 parties en poids, ou de 35 à 40 parties en poids d'au moins un monomère à teneur en groupe acide en $C_3$-$C_6$ à insaturation éthylénique ;
b) de 40 à 75 parties en poids, ou de 50 à 70 parties en poids, ou de 60 à 68 parties en poids d'au moins un ester d'alkyle en $C_1$ à $C_{30}$ d'acide (méth)acrylique ;
c) de 0 à 15 parties en poids, ou de 0,1 à 10 parties en poids, ou de 1 à 5 parties en poids d'au moins un monomère choisi parmi un monomère associatif, un monomère semi-hydrophobe, et leurs mélanges ;
d) de 0 à 10 parties en poids, ou de 0,05 à 8 parties en poids, ou de 0,1 à 5 parties en poids, ou de 0,5 à 3 parties en poids, ou de 0,75 à 2 parties en poids d'au moins un monomère de réticulation polyinsaturé ; dans lequel la somme de a), b), c) et d) dans ledit mélange de monomères est de 100 parties en poids, en présence d'une composition stabilisante comprenant :

i) de 0,1 à 75 parties en poids, ou de 0,5 à 60 parties en poids, ou de 1 à 55 parties en poids, ou de 5 à 35 parties en poids, ou de 10 à 30 parties en poids d'un amidon digéré par voie enzymatique pour 100 parties en poids de ladite composition de monomères ;
ii) de 0,001 à 2 parties en poids, ou de 0,005 à 1,5 partie en poids, ou de 0,05 à 1 partie en poids, ou de 0,1 à 0,75 partie en poids, ou de 0,15 à 0,5 partie en poids de PVOH pour 100 parties en poids de ladite composition de monomères polymérisable ; et
iii) de 0,001 à 0,5 partie en poids, ou de 0,005 à 0,4 partie en poids, ou de 0,01 à 0,3 partie en poids, ou de 0,03 à 0,2 partie en poids, ou de 0,05 à 0,1 partie en poids d'au moins un acide choisi parmi un mono- ou diacide saturé en $C_2$ à $C_{50}$, un mono- ou diacide insaturé en $C_{10}$ à $C_{50}$, et leurs mélanges, pour 100 parties en poids de ladite composition de monomères polymérisable ;

dans lequel ledit au moins un monomère associatif (c) dans ladite composition de monomères est représenté par les formules I et/ou II :

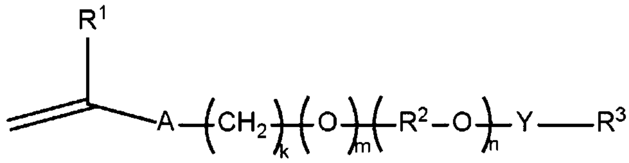

I

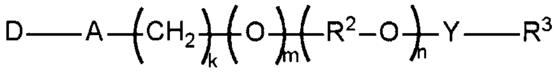

II

où $R^1$ est hydrogène ou méthyle ; A est $-CH_2C(O)O-$, $-C(O)O-$, $-O-$, $- CH_2O-$, $-NHC(O)NH-$, $-C(O)NH-$, $-Ar-(CE_2)_z-NHC(O)O-$, $-Ar-(CE_2)_z-NHC(O)NH-$, ou $-CH_2CH_2NHC(O)-$ ; Ar est un arylène divalent (par exemple phénylène) ; E est H ou méthyle ; z vaut 0 ou 1 ; k est un nombre entier compris dans la plage allant de 0 à 30, et m vaut 0 ou 1, à condition que lorsque k vaut 0, m vaille 0, et lorsque k est compris dans la plage allant de 1 à environ 30, m vaille 1 ; D représente un groupement vinyle ou allyle ; $(R^2-O)_n$ est un groupement polyoxyalkylène, qui peut être un homopolymère, un copolymère aléatoire, ou un copolymère à blocs de motifs oxyalkylène en $C_2$-$C_4$, $R^2$ est un groupement alkylène divalent choisi parmi $C_2H_4$, $C_3H_6$, ou $C_4H_8$, et leurs combinaisons ; et n est un nombre entier compris dans la plage de 2 à 150, ou de 10 à 120, ou de 15 à 60 ; Y est $-R^2O-$, $-R^2NH-$, $- C(O)-$, $-C(O)NH-$, $-R^2NHC(O)NH-$, ou $-C(O)NHC(O)-$ ; $R^3$ est un alkyle substitué ou non substitué choisi parmi un alkyle linéaire en $C_8$-$C_{30}$, un alkyle ramifié en $C_8$-$C_{30}$, un alkyle carbocyclique en $C_8$-$C_{30}$, un phényle substitué par alkyle en $C_2$-$C_{30}$, un phényle substitué par aralkyle, et un alkyle en $C_2$-$C_{30}$ substitué par aryle ; où le groupe alkyle, groupe aryle, groupe phényle $R^3$ comprend facultativement un ou plusieurs substituants choisis dans le groupe constitué d'un groupe hydroxyle, d'un groupe alcoxyle, d'un groupe benzyle, d'un groupe styryle, et d'un groupe halogène ; et

dans lequel ledit au moins un monomère semi-hydrophobe (c) dans ladite composition de monomères est représenté par les formules IV et/ou V :

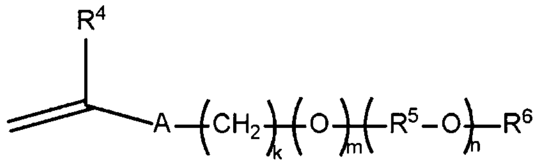

IV

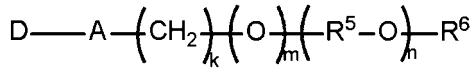

V

où $R^4$ est hydrogène ou méthyle ; A est $-CH_2C(O)O-$, $-C(O)O-$, $-O-$, $- CH_2O-$, $-NHC(O)NH-$, $-C(O)NH-$, $-Ar-(CE_2)_z-NHC(O)O-$, $-Ar-(CE_2)_z-NHC(O)NH-$, ou $-CH_2CH_2NHC(O)-$ ; Ar est un arylène divalent (par exemple phénylène) ; E est H ou méthyle ; z vaut 0 ou 1 ; k est un nombre entier compris dans la plage allant de 0 à 30, et m vaut 0 ou 1, à condition que lorsque k vaut 0, m vaille 0, et lorsque k est compris dans la plage allant de 1 à environ 30, m vaille 1 ; $(R^5-O)_n$ est un groupement polyoxyalkylène, qui peut être un homopolymère, un copolymère aléatoire, ou un copolymère à blocs de motifs oxyalkylène en $C_2$-$C_4$, $R^5$ est un groupement alkylène divalent choisi parmi $C_2H_4$, $C_3H_6$, ou $C_4H_8$, et leurs combinaisons ; et n est un nombre entier compris dans la plage allant de 2 à 150, ou de 5 à 120, ou de 10 à 60 ; $R^6$ est choisi parmi hydrogène et un groupe alkyle en $C_1$-$C_4$ linéaire ou ramifié ; et D représente un groupement vinyle ou allyle.

**16.** Procédé d'atténuation de la coalescence et/ou de la floculation de particules dans un latex polymère obtenu selon la revendication 15 par administration audit latex d'un acide organique, d'un acide inorganique, d'un agent réducteur, et leurs mélanges, en une quantité comprise dans une plage allant de 1,25 à 10 % en poids, ou de 2,5 à 7,5 % en poids, ou de 3,5 à 5 % en poids, sur la base du poids total du latex polymère en émulsion.

**17.** Procédé selon la revendication 16, dans lequel ledit acide organique est choisi parmi acide acétique, acide citrique, acide glycolique, acide lactique, acide mandélique, acide hydroxycaproïque, acide hydroxycaprylique, acide malique, acide tartrique, acide α-hydroxypropionique), acide salicylique, acide tropique, acide tréthocanique, acide β-hydroxypropionique, acide β-hydroxybutyrique, acide β-hydroxy β-méthylbutyrique, carnitine, acides de fruits naturels, acide caproïque, acide caprylique, acide pélargonique, acide caprique, acide undécanoïque, acide laurique, acide myristique, acide palmitique, acide palmitoléique, acide stéarique, acide isostéarique, acide hydroxy stéarique, acide oléique, acide ricinoléique, acide vaccénique, acide linolénique, acide α-linolénique, acide γ-linolénique, acide arachidique, acide gadoléique, acide arachidonique, acide eicosapentaénoïque, acide béhénique, acide docosahexaénoïque, acide lignocérique, acide malonique, acide succinique, acide glutarique, acide adipique, acide pimélique, acide subérique, acide azélaïque, acide undécanedioïque, acide dodécandioïque, acide brassylique, acide thapsique, acide japanique, acide phellogénique, acide équisétolique, acide isocitrique, acide aconitique, acide propane-1,2,3-tricarboxylique, acide agarique, acide trimésique, acide tartronique, acide mésoxalique, acide oxaloacétique, acide aspartique, acide dioxosuccinique, acide formamidine sulfinique, sel de sodium d'acides sulfiniques organiques, acide hydroxyméthanesulfonique, acide érythorbique ; acide ascorbique ; acide isoascorbique, acide glycérique, acide 2-hydroxy-2-sulfinatoacétique, leurs sels, et leurs combinaisons, et/ou dans lequel ledit acide inorganique est choisi parmi acide chlorhydrique, acide nitrique, acide sulfurique, acide sulfamique, acide phosphorique, et leurs mélanges, et/ou

dans lequel ledit agent réducteur est choisi parmi sulfate de sodium formaldéhyde, métaux de transition, hydrazine, sulfite de sodium, bisulfite de sodium, thiosulfate de sodium, hydrosulfite de sodium, sulfure de sodium, hydrosulfure de sodium, dithionite de sodium, bisulfite d'acétone ; éthanolamine ; monosaccharides, disaccharides, oligosaccharides, et leurs dérivés oxydés ; et leurs mélanges.

**18.** Procédé selon la revendication 16 ou la revendication 17, dans lequel ledit acide organique, acide inorganique, agent réducteur, et leurs mélanges, est ajouté au latex polymère après la réaction de polymérisation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014139904 A
- WO 2017112586 A1
- WO 2019126162 A1
- US 9051341 B
- US 4138380 A
- US 4110291 A
- US 6573375 B
- US 6727357 B
- WO 9921530 A
- US 3929678 A
- US 4565647 A
- US 5720964 A
- US 5858948 A
- US 5456849 A
- US 7115550 B
- US 3288770 A
- US 3412019 A
- US 4772462 A
- US 5275809 A
- US 34584 A
- US 5104646 A
- US 5106609 A
- US 4152416 A
- US 2826551 A
- US 3964500 A
- US 4364837 A
- GB 849433 A
- EP 0582152 A
- WO 9323009 A
- US 5136063 A
- US 5180843 A
- EP 0486135 A
- WO 9311103 A
- US 4230688 A
- US 4136163 A
- US 6183766 B
- US 7001594 B
- US 2809971 A
- US 3236733 A
- US 3753196 A
- US 3761418 A
- US 4345080 A
- US 4323683 A
- US 4379753 A
- US 4470982 A
- US 20040213751
- US 8491877 B
- WO 0100151 A
- FR 2679771
- EP 1184426 A
- US 7205271 B
- US 4654207 A
- US 5019376 A
- US 5384114 A
- US 7202199 B
- WO 201413990 A


### Non-patent literature cited in the description


- Dimer Acids. **T. E. BREUER**. Kirk-Othmer Encyclopedia of Chemical Technology. John Wiley & Sons, 1993, vol. 8, 223-237
- **SCHWARTZ** ; **PERRY** ; **BERCH**. McCutcheon's Emulsifiers and Detergents
- CTFA Cosmetic Ingredient Handbook. Cosmetic and Fragrance Assn., Inc., 2002
- Encyclopedia of Polymer Science and Engineering. John Wiley & Sons, Inc., 1989, vol. 15, 204-308
- **NOLL** ; **WALTER**. Chemistry and Technology of Silicones. Academic Press, 1968
- Silicon Compounds. Petrarch Systems, Inc., 1984
- **TODD** ; **BYERS**. Volatile Silicone Fluids for Cosmetics. *Cosmetics and Toiletries*, 1976, vol. 91 (1), 27-32
- **KASPRZAK**. Volatile Silicones. *Soap/Cosmetics/Chemical Specialities*, December 1986, 40-43
- **O'LENICK, A. J., JR.** Guerbet chemistry. *Journal of Surfactants and Detergents*, 2001, vol. 4, 311-315
- Harry's Cosmeticology. Chemical Publishing Company Inc., 1982, 266
- AHAs & Cellulite Products How They Work. *COSMETICS & TOILETRIES, C&T Ingredient Resource Series*, 1995
- *Cosmeceuticals*, 1998
- CTFA International Color Handbook. Micelle Press, 1992
- US Food and Drug Administration, FDA/IAS Booklet. Cosmetic Handbook. US Food and Drug Administration, FDA/IAS Booklet, 1992

- **HUNTING**. Opacifiers and pearling agents in shampoos. *Cosmetic and Toiletries*, July 1981, vol. 96, 65-78, Opacifiers and pearling agents in shampoos
- CTFA Cosmetic Ingredient Handbook. The Cosmetic, Toiletry and Fragrance Association, Inc., 1988, 75